# EUROPEAN PATENT APPLICATION

(11) **EP 4 342 891 A1**
(43) Date of publication of application: **27.03.2024**
(21) Application number: 22804086.1
(22) Date of filing: 20.05.2022
(51) Int. Cl.: C07D 401/14, C07D 471/10, C07D 498/10, C07D 221/20, C07D 241/18, C07D 241/20, A61K 31/497, A61K 31/675, A61P 35/00, A61P 35/02

(54) **SPIRO COMPOUND AND USE THEREOF**

(30) Priority: 21.05.2021 CN 202110557549; 27.08.2021 CN 202111000088
(71) Applicant: CSPC Zhongqi Pharmaceutical Technology (Shijiazhuang) Co., Ltd., Shijiazhuang, Hebei 050035 (CN)
(72) Inventor: MI, Guorui, Shijiazhuang, Hebei 050035 (CN); ZHAO, Chuanwu, Shijiazhuang, Hebei 050035 (CN); GUO, Qian, Shijiazhuang, Hebei 050035 (CN); ZHANG, Jianan, Shijiazhuang, Hebei 050035 (CN); LI, Chunna, Shijiazhuang, Hebei 050035 (CN); WEI, Wenli, Shijiazhuang, Hebei 050035 (CN); XU, Yanxia, Shijiazhuang, Hebei 050035 (CN)
(74) Representative: Barrow, Nicholas Martin
(86) International application number: PCT/CN2022/094255
(87) International publication number: WO 2022/242767

(57) **Abstract**

Provided in the present invention are a class of compounds as represented by formula (I), or a prodrug, a tautomer, a stereoisomer, a solvate, and an isotopic derivative thereof, or a pharmaceutically acceptable salt thereof. The compound has an SHP2 kinase inhibitory effect and can be used for treating and/or preventing diseases, disorders, and conditions mediated by SHP2 activity, such as tumors, cancers, cancer metastasis, cardiovascular diseases, immune disorders, visual disorders, etc.

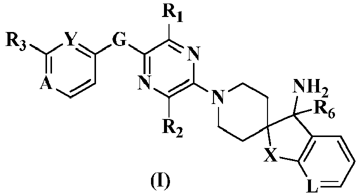

## Description

### Cross-references to related applications

This application claims the benefit of priority of the Chinese invention patent applications No. 202110557549.0 and No. 202111000088.3 filed respectively on May 21, 2021, and August 27, 2021, with China National Intellectual Property Administration, the contents of which are incorporated herein by reference in their entirety.

### Technical Field

This disclosure relates to the field of medical technology, specifically to a class of novel compounds with SHP2 inhibitor activity and their use in the treatment and prevention of diseases, disorders, and conditions mediated by SHP2 activity, such as hyperproliferative diseases.

### Background

SHP2 (Src homology domain, Src homology-2 domain) is encoded by Protein Tyrosine Phosphatase, Nonreceptor type 11 (PTP nonreceptor 11, PTPN11) and catalyzes the dephosphorylation of tyrosine in protein. It is generally believed that the N-terminal of SHP2 contains two SH2 domains including N-src homology 2 domain (SH2) and C-SH2, and the C-terminal contains a catalytically active PTP domain. In the inactive state, SHP2 is in a self-inhibition state, and N-SH2 and C-PTP combine eachp other to inhibit the activity of the phosphatase. In the presence of extracellular stimuli, they bind to relevant receptors and activate multiple downstream signal pathways, such as multiple signaling pathways including Ras/MAPK, and PI3K/AKT, and regulate cell proliferation, differentiation, apoptosis, and survival.

It is disclosed in the literature (Eur J Med Genet. 2015.58:509; WO2018013597) that germline mutations in PTPN11 and SHP2 have been confirmed to be associated with a variety of human diseases, such as Noonan syndrome (NS), Leopard Syndrome, and various malignant tumors (leukemia, etc.). Research from Revolution Medicines also shows that SHP2 plays an important role in oncogenic growth and survival signaling in some common cancers.

In addition, it is shown in the study (Sci adv. 2020, 6 (5): eaay4458) that programmed death 1 (PD-1) is also involved in mediating and activating SHP2. In cancer, PD-1 inhibits T cell stimulus and mediates immune escape. After stimulation, PD-1 is phosphorylated at its immunoreceptor tyrosine-based inhibitory motif (ITIM) and immunoreceptor tyrosine-based switching motif (ITSM). Then it binds with SHP2 to initiate T cell inactivation. Therefore, SHP2 inhibitors can stimulate adaptive and innate immune activities in the tumor microenvironment, with the potential to restore the anti-tumor immune response that has been silenced by cancer cells.

In view of the foregoing, there is a need to develop SHP2 inhibitors for the treatment and prevention of diseases, disorders, and conditions that are mediated by SHP2 activity.

### Summary of the Invention

The present disclosure provides a class of SHP2 inhibitor compounds with novel structures and also provides use of this class of compounds, or prodrugs, tautomers, stereoisomers, solvates, isotope derivatives, or pharmaceutically acceptable salts thereof in treating diseases, disorders, and conditions that are mediated by SHP2 activity.

On the one hand, the present disclosure provides a compound represented by formula (I) or prodrug, tautomer, stereoisomer, solvate, isotope derivative, or pharmaceutically acceptable salt thereof, which compound has the following structure: 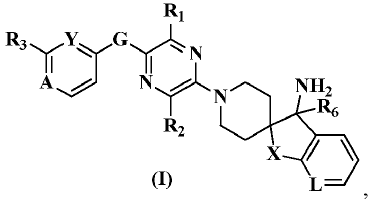 wherein,
X is selected from O, CH₂, NH, and S;
L is N or CH;
G is a bond or S;
A is CH or N;
R₃ is independently selected from optionally substituted 8-12 membered heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, - COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl;
R₁ is independently selected from hydrogen, deuterium, and amino;
R₂ is independently selected from hydrogen, halogen, cyano, -CONH₂, -CORs, -COORs, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two hydrogens on the same substitution site are replaced by the same oxygen to form a double bond;
the heteroatoms in the heterocycloalkyl, heteroaryl, and heterocyclyl are independently selected from O, N, and S, and the number of heteroatoms is 1, 2, 3, or 4.

In an embodiment of the present disclosure, there is provided a compound represented by formula (I), or a prodrug, tautomer, stereoisomer, solvate, isotope derivative, or pharmaceutically acceptable salt thereof, which compound has the following structure: 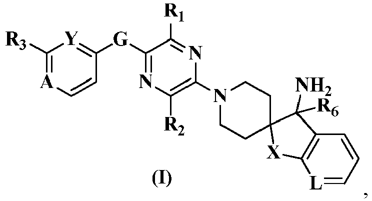 wherein,
X is selected from O, CH₂, NH, and S;
L is N or CH;
G is a bond or S;
A is CH or N;
R₃ is amino;
R₁ is independently selected from hydrogen, amino, and deuterium;
R₂ is independently selected from halogen, cyano, -CONH₂, -CORs, -COORs, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two hydrogens on the same substitution site are replaced by the same oxygen to form a double bond;
the heteroatoms in the heterocycloalkyl, heteroaryl, and heterocyclyl are independently selected from O, N, and S, and the number of heteroatoms is 1, 2, 3, or 4.

In another embodiment of the present disclosure, there is provided a compound represented by formula (I), or a prodrug, tautomer, stereoisomer, solvate, isotope derivative, or pharmaceutically acceptable salt thereof, which compound has the following structure: 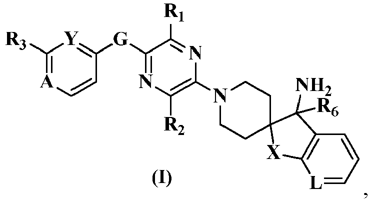 wherein,
X is selected from O, CH₂, NH, and S;
L is N or CH;
G is a bond or S;
A is CH or N;
R₃ is independently selected from amino, optionally substituted 8-12 membered heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, - COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl;
R₁ is independently selected from hydrogen, deuterium, and amino;
R₂ is independently selected from hydrogen, halogen, cyano, -CONH₂, -CORs, -COORs, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two H's on the same substitution site are replaced by the same O to form a double bond;
the heteroatoms in the heterocycloalkyl, heteroaryl, and heterocyclyl are independently selected from O, N, and S, and the number of heteroatoms is 1, 2, 3, or 4.

In another embodiment of the present disclosure, R₆ is deuterium.

In another embodiment of the present disclosure, there is provided a compound represented by formula (I-1), or a prodrug, tautomer, stereoisomer, solvate, isotope derivative, or pharmaceutically acceptable salt thereof, which compound has the following structure: 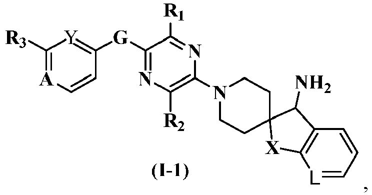 wherein,
X is selected from O, CH₂, NH, and S;
L is N or CH;
G is a bond or S;
A is CH or N;
R₃ is independently selected from optionally substituted 8-12 membered heterocyclyl, optionally substituted C₆₋₁₀aryl, and optionally substituted 5-14 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, -COOCH₃, - CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl;
R₁ is independently selected from hydrogen, deuterium, and amino;
R₂ is independently selected from hydrogen, halogen, cyano, -CONH₂, -CORs, -COORs, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
said "oxo" group means that two H's on the same substitution site are replaced by the same O to form a double bond;
the heteroatoms in the above-mentioned heterocycloalkyl, heteroaryl, and heterocyclyl are independently selected from O, N, and S, and the number of heteroatoms is 1, 2, 3, or 4;
wherein the compound is not: 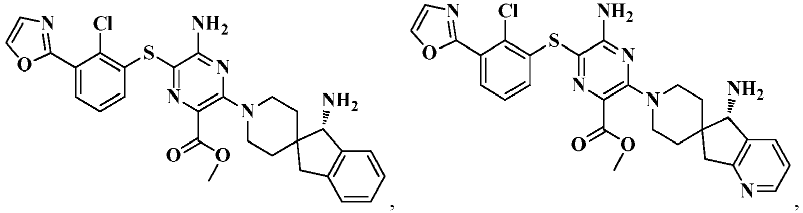 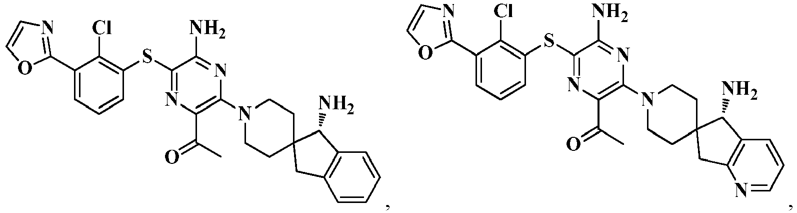 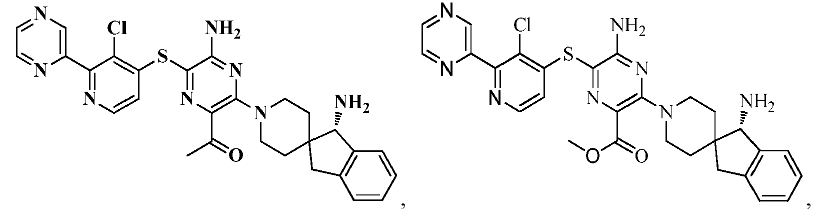 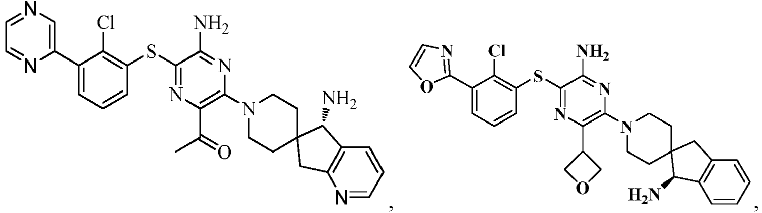 and

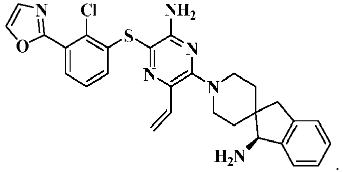

In another embodiment of the present disclosure, there is provided a compound having a structure as represented by formula (I-1-1), or a prodrug, tautomer, stereoisomer, solvate, isotope derivative, or pharmaceutically acceptable salt thereof, 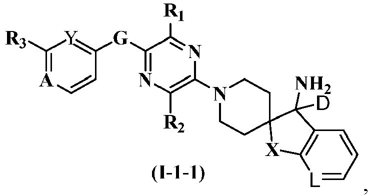 wherein the definitions of G, A, Y, R₁, R₂, R₃, X, and L are identical to those described for the compound represented by formula (I) or formula (I-1) in this disclosure.

In some embodiments of this disclosure, there are provided the compound of formula (I), the compound of formula (I-1), and the compound of formula (I-1-1) as described above, or a prodrug, tautomer, stereoisomer, solvate, isotope derivative, or pharmaceutically acceptable salt thereof, wherein said R₃ is amino.

In some embodiments of this disclosure, there are provided the compound of formula (I), the compound of formula (I-1), and the compound of formula (I-1-1) as described above, or a prodrug, tautomer, stereoisomer, solvate, isotope derivative, or pharmaceutically acceptable salt thereof, wherein said R₃ is independently selected from optionally substituted 8-12 membered heterocyclyl, optionally substituted C₆₋₁₀aryl, and optionally substituted 5-14 membered heteroaryl.

In some embodiments of this disclosure, said R₃ in the compound of formula (I), the compound of formula (I-1), and the compound of formula (I-1-1) is independently selected from optionally substituted 8-12membered bicyclic heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, -COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl.

In some embodiments of this disclosure, said R₃ in the compound of formula (I), the compound of formula (I-1), and the compound of formula (I-1-1) is independently selected from optionally substituted 8-10 membered bicyclic heterocyclyl, optionally substituted C₆₋₈aᵣyl, and optionally substituted 5-10 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, -COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl.

In some embodiments of this disclosure, said R₃ in the compound of formula (I), the compound of formula (I-1), and the compound of formula (I-1-1) is independently selected from optionally substituted 8-10 membered bicyclic heterocyclyl, optionally substituted C₆₋₈aᵣyl, and optionally substituted 5-10 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, oxo, cyano, -CONH₂, and C₁₋₆alkyl.

In some embodiments of this disclosure, said R₃ in the compound of formula (I), the compound of formula (I-1), and the compound of formula (I-1-1) is independently selected from optionally substituted 10 membered bicyclic heterocyclyl, optionally substituted C₆₋₈aᵣyl, and optionally substituted 5-10 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, - CONH₂, methyl, ethyl, n-propyl, isopropyl, and n-butyl, wherein the heteroatoms in the heterocyclyl and heteroaryl are independently selected from O, N, and S, and the number of heteroatoms is 1, 2 or 3.

In some embodiments of this disclosure, said R₃ in the compound of formula (I), the compound of formula (I-1), and the compound of formula (I-1-1) is independently selected from optionally substituted C₆₋₈aryl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, oxo, cyano, -CONH₂, and C₁₋₆alkyl, wherein the heteroatoms in the heteroaryl are independently selected from O, N, and S, and the number of heteroatoms is 1 or 2.

In some embodiments of this disclosure, said R₃ in the compound of formula (I), the compound of formula (I-1), and the compound of formula (I-1-1) is independently selected from optionally substituted phenyl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of cyano, -CONH₂, and methyl, wherein the heteroatoms in the heteroaryl are independently selected from O, N, and S, and the number of heteroatoms is 1 or 2.

In some embodiments of this disclosure, said R₃ in the compound of formula (I), the compound of formula (I-1), and the compound of formula (I-1-1) is independently selected from optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, -COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl.

In some embodiments of this disclosure, said R₃ in the compound of formula (I), the compound of formula (I-1), and the compound of formula (I-1-1) is independently selected from optionally substituted 5-10 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, oxo, cyano, -CONH₂, and C₁₋₆alkyl.

In some embodiments of this disclosure, said R₃ in the compound of formula (I), the compound of formula (I-1), and the compound of formula (I-1-1) is independently selected from optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, -CONH₂, and C₁₋₆alkyl, wherein the heteroatoms in the heteroaryl are independently selected from O, N, and S, and the number of heteroatoms is 1 or 2.

In some embodiments of this disclosure, said R₃ in the compound of formula (I), the compound of formula (I-1), and the compound of formula (I-1-1) is independently selected from optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, -CONH₂, and C₁₋₃alkyl, wherein the heteroatoms in the heteroaryl are independently selected from O, N, and S, and the number of heteroatoms is 1 or 2.

In some embodiments of this disclosure, said R₃ in the compound of formula (I), the compound of formula (I-1), and the compound of formula (I-1-1) is independently selected from optionally substituted 5 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, cyano, -CONH₂, and methyl, wherein the heteroatoms in the heteroaryl are independently selected from O, N, and S, and the number of heteroatoms is 1 or 2.

In some embodiments of this disclosure, said R₃ in the compound of formula (I), the compound of formula (I-1), and the compound of formula (I-1-1) is independently selected from the following structures, which are optionally substituted:

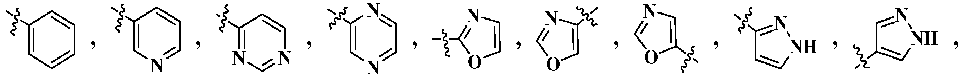

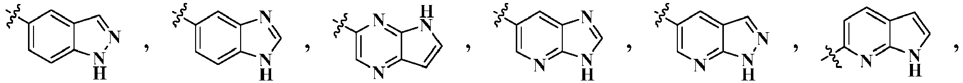

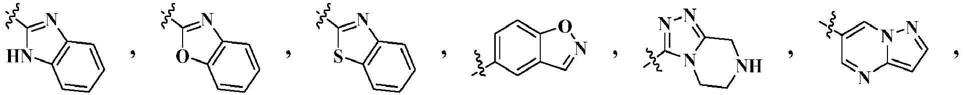

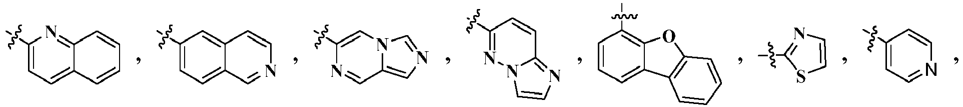

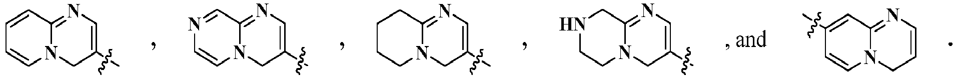

In some embodiments of this disclosure, said R₃ in the compound of formula (I), the compound of formula (I-1), and the compound of formula (I-1-1) is independently selected from the following structures, which are optionally substituted:

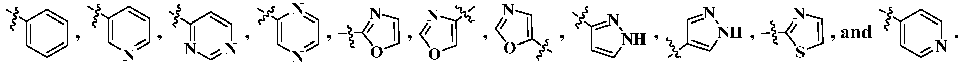

In some embodiments of this disclosure, said R₃ in the compound of formula (I), the compound of formula (I-1), and the compound of formula (I-1-1) is independently selected from the following structures, which are optionally substituted:

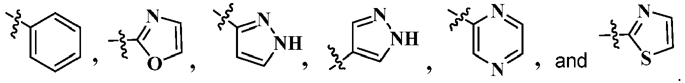

In some embodiments of this disclosure, said R₃ in the compound of formula (I), the compound of formula (I-1), and the compound of formula (I-1-1) is independently selected from amino, and optionally substituted

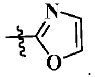

In some embodiments of this disclosure, said R₃ in the compound of formula (I), the compound of formula (I-1), and the compound of formula (I-1-1) is independently selected from optionally substituted

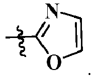

In some embodiments of this disclosure, said R₃ in the compound of formula (I), the compound of formula (I-1), and the compound of formula (I-1-1) is independently selected from the following structures:

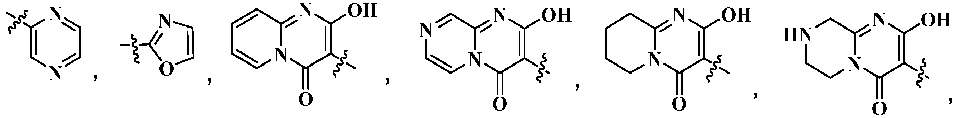

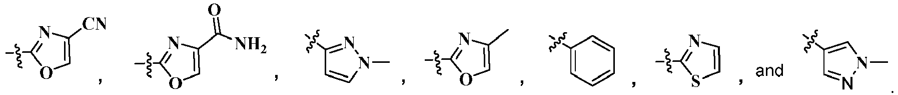

In some embodiments of this disclosure, said R₃ in the compound of formula (I), the compound of formula (I-1), and the compound of formula (I-1-1) is independently selected from the following structures:

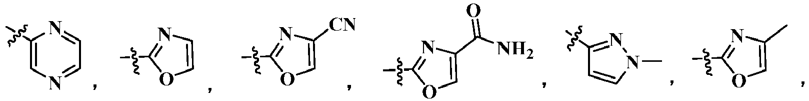

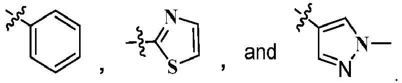

In some embodiments of this disclosure, there are provided the compound of formula (I), the compound of formula (I-1), and the compound of formula (I-1-1), as described above, or a prodrug, tautomer, stereoisomer, solvate, isotope derivative, or pharmaceutically acceptable salt thereof, wherein R₂ is independently selected from halogen, cyano, -CONH₂, -COR₅, -COOR₅, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In some embodiments of this disclosure, there are provided the compound of formula (I), the compound of formula (I-1), and the compound of formula (I-1-1), as described above, or a prodrug, tautomer, stereoisomer, solvate, isotope derivative, or pharmaceutically acceptable salt thereof, wherein R₂ is independently selected from hydrogen, halogen, cyano, -CONH₂, -COR₅, -COOR₅, optionally substituted C₁₋₆alkyl, optionally substituted C₂₋₆alkenyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In some embodiments of this disclosure, said R₂ in the compound of formula (I), the compound of formula (I-1), and the compound of formula (I-1-1) is independently selected from hydrogen, halogen, cyano, - CONH₂, -CORs, -COORs, optionally substituted C₁₋₄alkyl, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In some specific embodiments of this disclosure, said R₂ in the compound of formula (I), the compound of formula (I-1), and the compound of formula (I-1-1) is independently selected from halogen, cyano, -CONH₂, -COR₅, -COOR₅, C₁₋₄alkyl that is substituted by one or more substituents selected from hydroxy, C₁₋₆alkoxyl and halogen, C₂₋₆alkenyl, C₃₋₆cycloalkyl, and 3-6 membered heterocycloalkyl.

In some embodiments of this disclosure, said R₂ in the compound of formula (I), the compound of formula (I-1), and the compound of formula (I-1-1) is independently selected from halogen, cyano, -CONH₂, -CORs, -COORs, hydroxy-substituted C₁₋₄alkyl, C₂₋₆alkenyl, C₃₋₆cycloalkyl, and 3-6 membered heterocycloalkyl.

In some embodiments of this disclosure, said R₂ in the compound of formula (I), the compound of formula (I-1), and the compound of formula (I-1-1) is independently selected from halogen, -CORs, -COOR₅, hydroxy-substituted C₁₋₄alkyl, C₂₋₆alkenyl, and 3-6 membered heterocycloalkyl; or, R₂ is independently selected from halogen, -COR₅, -COOR₅, C₂₋₆alkenyl and 3-6 membered heterocycloalkyl; or, R₂ is independently selected from -CORs and -COORs; or, R₂ is -CORs or R₂ is -COORs, in which preferably, R₅ is C₃₋₆cycloalkyl or C₁₋₆alkyl, preferably C₃₋₄cycloalkyl or C₁₋₃alkyl, for example cyclopropyl, or methyl; or, R₂ is independently selected from -CO(C₁₋₆alkyl); or, R₂ is independently selected from -CO(C₁₋₃alkyl). In some embodiments of this disclosure, said R₅ in the compound of formula (I), the compound of formula (I-1), and the compound of formula (I -1-1) is independently selected from hydrogen, optionally substituted C₁₋₆alkyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In some embodiments of this disclosure, said R₅ in the compound of formula (I), the compound of formula (I-1), and the compound of formula (I -1-1) is independently selected from hydrogen, optionally substituted C₁₋₆alkyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, and C₁₋₆alkyl.

In some embodiments of this disclosure, said R₅ in the compound of formula (I), the compound of formula (I-1), and the compound of formula (I-1-1) is independently selected from hydrogen, C₁₋₆alkyl, C₃₋₆cycloalkyl, and 3-6 membered heterocycloalkyl; further, R₅ is independently selected from C₁₋₃alkyl, and C₃₋₆cycloalkyl; or R₅ is independently selected from C₁₋₃alkyl, e.g. methyl.

In some embodiments of this disclosure, there are provided the compound of formula (I), the compound of formula (I-1), and the compound of formula (I-1-1) or a prodrug, tautomer, stereoisomer, solvate, isotope derivative, or pharmaceutically acceptable salt thereof, wherein R₂ is independently selected from halogen, cyano, -CONH₂, -CO-(C₁₋₃alkyl), -CO-(C₃₋₆cycloalkyl), -COO-(C₁₋₃alkyl), -COO-(C₃₋₆cycloalkyl), - CH₂OH, -CH₂CH₂OH, -CH (OH)CH₃, C₂₋₆alkenyl, C₃₋₆cycloalkyl, and 3-6 membered heterocycloalkyl. In some embodiments of this disclosure, said R₂ in the compound of formula (I), the compound of formula (I-1), and the compound of formula (I-1-1) is independently selected from halogen, -CO-(C₁₋₃alkyl), -CO-(C₃₋₆cycloalkyl), -COO-(C₁₋₃alkyl), -COO-(C₃₋₆cycloalkyl), -CH₂OH, C₂₋₄alkenyl, and 3-6 membered heterocycloalkyl.

In some embodiments of this disclosure, said R₂ in the compound of formula (I), the compound of formula (I-1), and the compound of formula (I-1-1) is independently selected from halogen, -C(O)CH₃, -C(O)-(cyclopropane), -C(O)OCH₃, -C(O)O-(cyclopropane), -CH₂OH, ethenyl, and oxetanyl.

In some embodiments of this disclosure, said R₂ in the compound of formula (I), the compound of formula (I-1), and the compound of formula (I-1-1) is independently selected from hydrogen, F, -C(O)CH₃, - C(O)OCH₃, -CH₂OH, -CH=CH₂, 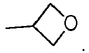 In some embodiments of this disclosure, said R₂ in the compound of formula (I), the compound of formula (I-1), and the compound of formula (I-1-1) is independently selected from F, -C(O)CH₃, -C(O)OCH₃, - CH=CH₂, and

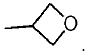

In some embodiments of this disclosure, said R₂ in the compound of formula (I), the compound of formula (I-1), and the compound of formula (I-1-1) is independently selected from -C(O)CH₃ and -C(O)OCH₃.

In some embodiments of this disclosure, said R₂ in the compound of formula (I), the compound of formula (I-1), and the compound of formula (I-1-1) is -C(O)CH₃.

In some preferable embodiments of the present disclosure, there are provided the compound of formula (I), the compound of formula (I-1), and the compound of formula (I-1-1) or a prodrug, tautomer, stereoisomer, solvate, isotope derivative, or pharmaceutically acceptable salt thereof, wherein R₁ is independently selected from hydrogen, and amino; preferably, R₁ is independently selected from amino.

In an embodiment of the present disclosure, there is provided a compound having a structure as represented by formula (I-2) or a prodrug, tautomer, stereoisomer, solvate, isotope derivative, or pharmaceutically acceptable salt thereof, 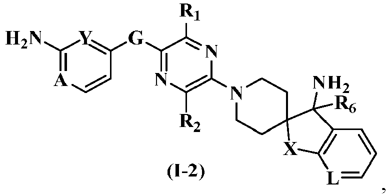
wherein R₁ is independently selected from deuterium and amino, preferably amino;
R₂ is independently selected from cyano, -CONH₂, -COR₅, -COOR₅, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
R₆ is hydrogen or deuterium, preferably deuterium;
the definitions of X, L, G, A, and Y are identical to those described for the compound represented by formula (I) or formula (I-1) in this disclosure.

In an embodiment of the present disclosure, there is provided a compound as represented by formula (I-2) or a prodrug, tautomer, stereoisomer, solvate, isotope derivative, or pharmaceutically acceptable salt thereof, wherein R₂ is independently selected from hydrogen, halogen, cyano, -CONH₂, -CORs, -COORs, optionally substituted C₁₋₆alkyl, optionally substituted C₂₋₆alkenyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In an embodiment of the present disclosure, R₂ in the compound represented by formula (I-2) is independently selected from cyano, -CONH₂, -COR₅, -COOR₅, optionally substituted C₁₋₄alkyl, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl.

In yet another embodiment of the present disclosure, R₂ in the compound represented by formula (I-2) is independently selected from cyano, -CONH₂, -CORs, -COORs, hydroxy-substituted C₁₋₄alkyl, C₂₋₆alkenyl, C₃₋₆cycloalkyl, and 3-6 membered heterocycloalkyl.

In another embodiment of the present disclosure, R₂ in the compound with a structure represented by formula (I-2) or a prodrug, tautomer, stereoisomer, solvate, isotope derivative, or pharmaceutically acceptable salt thereof is independently selected from cyano, -CONH₂, -CORs, -COORs, unsubstituted C₂₋₆alkyl, substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from cyano, -CONH₂, -COR₅, -COOR₅, substituted C₁₋₆alkyl, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from cyano, -CONH₂, -CORs, -COORs, C₁₋₆alkyl that is substituted by one or more substituents selected from hydroxy, halogen, and C₁₋₃alkoxyl, C₂₋₆alkenyl, C₃₋₆cycloalkyl, and 3-6 membered heterocycloalkyl; or
when R₁ is deuterium: R₂ is selected from -CO-(C₃₋₆cycloalkyl), -COORs, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
when R₁ is deuterium: R₂ is selected from -CO-(C₃₋₆cycloalkyl), -COORs, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
when R₁ is deuterium: R₂ is selected from -CO-(C₃₋₆cycloalkyl), -COORs, C₂₋₆alkenyl, C₃₋₆cycloalkyl, and 3-6 membered heterocycloalkyl.

In an embodiment of the compound with a structure represented by formula (I-2) of the present disclosure, when R₁ is deuterium: R₂ is halogen.

In some embodiments of this disclosure, in the compound with a structure represented by formula (I-2) or a prodrug, tautomer, stereoisomer, solvate, isotope derivative, or pharmaceutically acceptable salt thereof, R₁ is hydrogen; R₂ is independently selected from halogen, -CO-(C₃₋₆cycloalkyl), -COORs, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₁ is hydrogen; R₂ is independently selected from halogen, -CO-(C₃₋₆cycloalkyl), -COORs, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₁ is hydrogen; R₂ is independently selected from halogen, -CO-(C₃₋₆cycloalkyl), -COORs, C₂₋₆alkenyl, C₃₋₆cycloalkyl, and 3-6 membered heterocycloalkyl.

In an embodiment of the present disclosure, R₅ in the compound represented by the above formula (I-2) is independently selected from hydrogen, optionally substituted C₁₋₆alkyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl. In an embodiment of the present disclosure, R₅ in the compound represented by the above formula (I-2) is independently selected from hydrogen, optionally substituted C₁₋₆alkyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, and C₁₋₆alkyl.

In an embodiment of the present disclosure, R₅ in the compound represented by the above formula (I-2) is independently selected from hydrogen, C₁₋₆alkyl, C₃₋₆cycloalkyl, and 3-6 membered heterocycloalkyl.

In an embodiment of the present disclosure, R₅ in the compound represented by the above formula (I-2) is independently selected from C₁₋₃alkyl, and C₃₋₆cycloalkyl; or R₅ is C₁₋₃alkyl; or R₅ is C₃₋₆cycloalkyl.

In an embodiment of the present disclosure, there is provided a compound as represented by formula (I-2) or a prodrug, tautomer, stereoisomer, solvate, isotope derivative, or pharmaceutically acceptable salt thereof, wherein R₂ is independently selected from cyano, -CONH₂, -CO-(C₁₋₃alkyl), -CO-(C₃₋₆cycloalkyl), -COO-(C₁₋₃alkyl), -COO-(C₃₋₆cycloalkyl), -CH₂OH, -CH₂CH₂OH, -CH (OH)CH₃, C₂₋₆alkenyl, C₃₋₆cycloalkyl, and 3-6 membered heterocycloalkyl;
preferably, R₂ is independently selected from -CO-(C₁₋₃alkyl), -CO-(C₃₋₆cycloalkyl), -COO-(C₁₋₃alkyl), - COO-(C₃₋₆cycloalkyl), -CH₂OH, C₂₋₄alkenyl, and 3-6 membered heterocycloalkyl;
further preferably, R₂ is independently selected from -C(O)CH₃, -C(O)-(cyclopropane), -C(O)OCH₃, - C(O)O-(cyclopropane), -CH₂OH, ethenyl, and oxetanyl;
further preferably, R₂ is independently selected from -C(O)CH₃, -C(O)OCH₃, -CH₂OH, -CH=CH₂, and 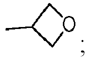 further preferably, R₂ is independently selected from -C(O)CH₃, -C(O)OCH₃, -CH=CH₂, and

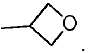

In an embodiment of the present disclosure, there is provided a compound as represented by formula (I-2) or a prodrug, tautomer, stereoisomer, solvate, isotope derivative, or pharmaceutically acceptable salt thereof,
wherein R₂ is independently selected from -CO-(C₁₋₆alkyl), and -COO-(C₁₋₆alkyl);
preferably, R₂ is independently selected from -CO-(C₁₋₃alkyl), and -COO-(C₁₋₃alkyl);
further preferably, R₂ is independently selected from -C(O)CH₃, and -C(O)OCH₃;
further preferably, R₂ is independently selected from -C(O)CH₃.

In an embodiment of the present disclosure, there is provided a compound as represented by formula (I-2) or a prodrug, tautomer, stereoisomer, solvate, isotope derivative, or pharmaceutically acceptable salt thereof, wherein L is CH, and X is O;
preferably, L is CH, X is O, and G is S.

In an embodiment of the present disclosure, there is provided a compound as represented by formula (I-2) or a prodrug, tautomer, stereoisomer, solvate, isotope derivative, or pharmaceutically acceptable salt thereof, wherein R₁ is independently selected from amino, R₂ is independently selected from -CO-(C₁₋₆alkyl), and -COO-(C₁₋₆alkyl), L is CH, and X is O.

In an embodiment of the present disclosure, there is provided a compound as represented by formula (I-2) or a prodrug, tautomer, stereoisomer, solvate, isotope derivative, or pharmaceutically acceptable salt thereof, wherein R₁ is independently selected from amino, R₂ is independently selected from -CO-(C₁₋₆alkyl), and -COO-(C₁₋₆alkyl), L is CH, R₆ is independently selected from deuterium;
preferably, R₁ is independently selected from amino, R₂ is independently selected from -CO-(C₁₋₃alkyl), or -COO-(C₁₋₃alkyl), R₆ is deuterium, L is CH, X is O, and G is S.

In some embodiments of this disclosure, there are provided the compound of formula (I), the compound of formula (I-1), the compound of formula (I-1-1), and the compound of formula (I-2), or a prodrug, tautomer, stereoisomer, solvate, isotope derivative, or pharmaceutically acceptable salt thereof, wherein X is O or CH₂; preferably, X is O.

In some embodiments of this disclosure, there are provided the compound of formula (I), the compound of formula (I-1), the compound of formula (I-1-1), and the compound of formula (I-2), or a prodrug, tautomer, stereoisomer, solvate, isotope derivative, or pharmaceutically acceptable salt thereof, wherein said compounds have the structures as represented by formulae (I-A), (I-B), (I-1-A), (I-1-B), (I-2-A) and (I-2-B): 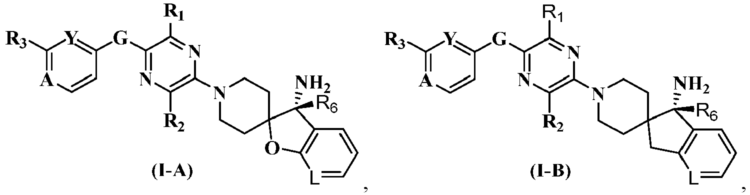 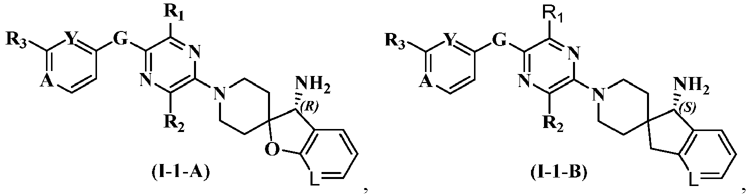 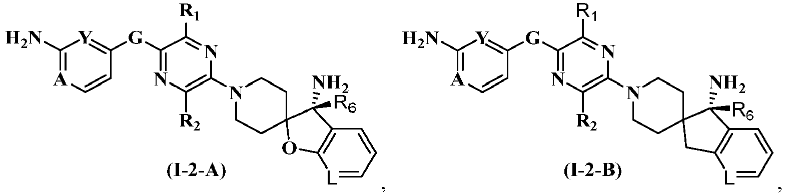 wherein the definitions of L, G, A, Y, R₁, R₂, R₃, and R₆ are identical to those described for the compounds represented by the above formula (I), formula (I-1), and formula (I-2) in this disclosure.

In some embodiments of this disclosure, there are provided the compound of formula (I), the compound of formula (I-1), the compound of formula (I-1-1), and the compound of formula (I-2), or a prodrug, tautomer, stereoisomer, solvate, isotope derivative, or pharmaceutically acceptable salt thereof, wherein L is CH.

In some embodiments of this disclosure, there are provided compounds (compound of formula (I), compound of formula (I-1), compound of formula (I-1-1), compound of formula (I-2)) or a prodrug, tautomer, stereoisomer, solvate, isotope derivative, or pharmaceutically acceptable salt thereof, wherein said compounds have the structures as represented by formulae (I-A1), (I-1-A1) and (I-2-A1): 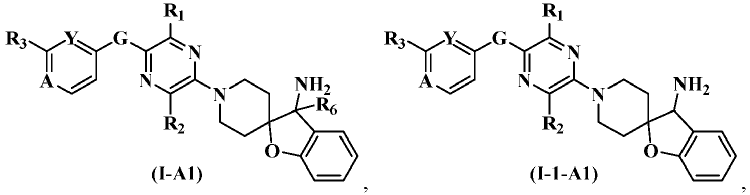 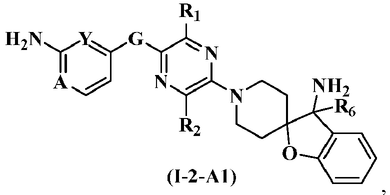 wherein the definitions of G, A, Y, R₁, R₂, R₃, and R₆ are identical to those described for the compounds represented by the above formula (I), formula (I-1), and formula (I-2) in this disclosure.

In some embodiments of the present disclosure, said compound has a structure as represented by formula (I-A2), (I-A3), (I-2-A2), or (I-2-A3): 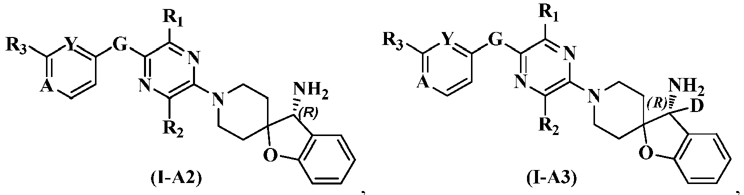 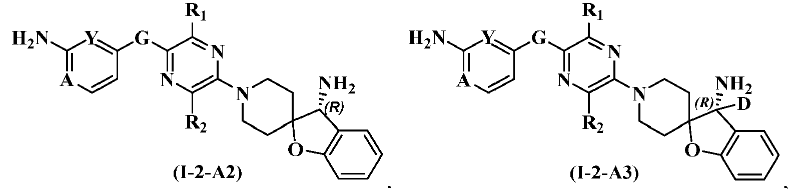 wherein the definitions of G, A, Y, R₁, R₂, and R₃ are identical to those described for the compound represented by the above formula (I), formula (I-1), or formula (I-2) in this disclosure.

In some embodiments of this disclosure, there are provided the compound of formula (I), the compound of formula (I-1), the compound of formula (I-1-1), and the compound of formula (I-2), or a prodrug, tautomer, stereoisomer, solvate, isotope derivative, or pharmaceutically acceptable salt thereof, wherein said compounds have a structure as represented by formula (I-Ba): 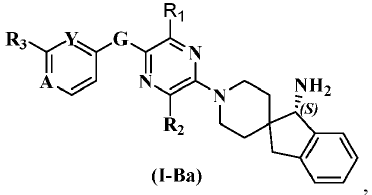 wherein the definitions of G, A, Y, R₁, R₂, and R₃ are identical to those described for the compound represented by the above formula (I), formula (I-1), or formula (I-2) in this disclosure.

In some embodiments of this disclosure, there are provided compounds (compound of formula (I), compound of formula (I-1), compound of formula (I-1-1), compound of formula (I-2)) or a prodrug, tautomer, stereoisomer, solvate, isotope derivative, or pharmaceutically acceptable salt thereof, wherein said compounds have the structures as represented by formula (I-B1), formula (I-1-B1) and formula (I-2-B1): 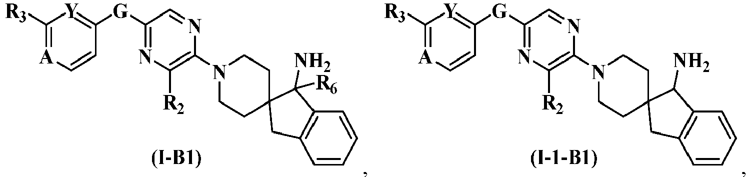 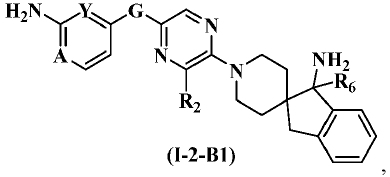 wherein the definitions of G, A, Y, R₂, R₃, and R₆ are identical to those described for the compounds represented by the above formula (I), formula (I-1), and formula (I-2) in this disclosure.

In some embodiments of this disclosure, said compound has a structure as represented by formula (I-B2), formula (I-B3), formula (I-2-B2), or formula (I-2-B3): 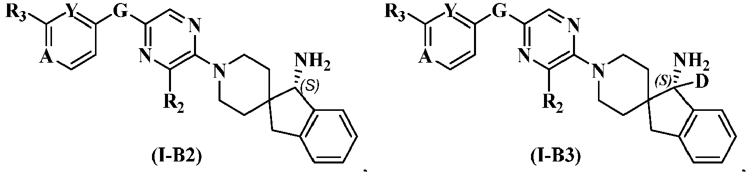 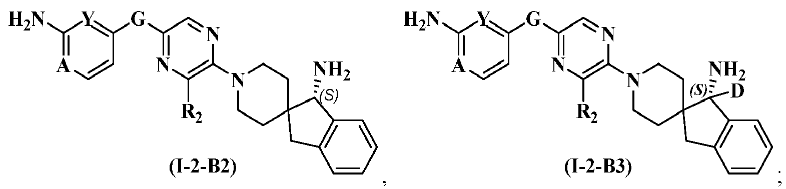 wherein the definitions of G, A, Y, R₂, and R₃ are identical to those described for the compound represented by the above formula (I), formula (I-1), or formula (I-2) in this disclosure.

In some embodiments of this disclosure, there are provided compounds (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)) or prodrugs, tautomers, stereoisomers, solvates, isotope derivatives or pharmaceutically acceptable salts thereof, wherein G is S.

In some embodiments of this disclosure, there are provided compounds (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)) or prodrugs, tautomers, stereoisomers, solvates, isotope derivatives or pharmaceutically acceptable salts thereof, wherein A is CH.

In some embodiments of this disclosure, there are provided compounds (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)) or prodrugs, tautomers, stereoisomers, solvates, isotope derivatives or pharmaceutically acceptable salts thereof, wherein A is N.

In some embodiments of this disclosure, there are provided compounds (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)) or prodrugs, tautomers, stereoisomers, solvates, isotope derivatives or pharmaceutically acceptable salts thereof, wherein Y is CR₄; or A is CH and Y is CR₄; or A is N and Y is CR₄; or A is N and Y is N.

In some embodiments of this disclosure, there are provided compounds (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)) or prodrugs, tautomers, stereoisomers, solvates, isotope derivatives or pharmaceutically acceptable salts thereof, wherein R₄ is independently selected from hydrogen, halogen, hydroxy, amino, cyano, optionally substituted C₁₋₆alkyl, and optionally substituted C₁₋₆alkoxyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and amino.

In an embodiment of the present disclosure, wherein R₄ is independently selected from hydrogen, halogen, amino, cyano, C₁₋₆alkyl, C₁₋₆alkoxyl, and C₁₋₆alkylamino; further preferably, wherein R₄ is independently selected from hydrogen, fluoro, chloro, amino, cyano, methyl, and methoxy; further preferably, wherein R₄ is Cl.

In an embodiment of the present disclosure, in the compound of formula (I): X is O or CH₂; L is CH; G is S; A is CH or N; R₃ is independently selected from amino, optionally substituted 8-12 membered heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, -CONH₂ and C₁₋₆alkyl; R₁ is independently selected from hydrogen, deuterium, and amino; R₂ is independently selected from halogen, -COR₅, -COOR₅, optionally substituted C₁₋₆alkyl, optionally substituted C₂₋₆alkenyl, and optionally substituted 3-10 membered heterocycloalkyl; wherein R₅ is independently selected from unsubstituted C₁₋₆alkyl and unsubstituted C₃₋₆cycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from and CR₄; R₄ is halogen; R₆ is independently selected from hydrogen, and deuterium; or

X is O or CH₂; L is CH; G is S; A is CH or N; R₃ is independently selected from amino, optionally substituted 8-10 membered bicyclic heterocyclyl, optionally substituted C₆ or C₁₀aryl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, -CONH₂ and C₁₋₆alkyl; R₁ is independently selected from hydrogen, deuterium, and amino; R₂ is independently selected from halogen, -COR₅, -COOR₅, optionally substituted C₁₋₆alkyl, optionally substituted C₂₋₆alkenyl, and optionally substituted 3-6 membered heterocycloalkyl; wherein R₅ is independently selected from unsubstituted C₁₋₆alkyl and unsubstituted C₃₋₆cycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from N and CR₄; R₄ is halogen; R₆ is independently selected from hydrogen and deuterium; or
X is O or CH₂; L is CH; G is S; A is CH or N; R₃ is independently selected from amino, and 5-6 membered heteroaryl that is optionally substituted by C₁₋₆alkyl; R₁ is independently selected from hydrogen, deuterium, and amino; R₂ is independently selected from halogen, -CORs, -COORs, optionally substituted C₂₋₆alkenyl, and optionally substituted 3-6 membered heterocycloalkyl; wherein R₅ is independently selected from unsubstituted C₁₋₆alkyl and unsubstituted C₃₋₆cycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from and CR₄; R₄ is halogen; R₆ is independently selected from hydrogen and deuterium; or
X is O or CH₂; L is CH; G is S; A is CH or N; R₃ is independently selected from amino, and 5-6 membered heteroaryl that is optionally substituted by C₁₋₆alkyl; R₁ is independently selected from hydrogen, deuterium, and amino; R₂ is independently selected from halogen, -CORs, -COORs, unsubstituted C₂₋₆alkenyl, and unsubstituted 3-6 membered heterocycloalkyl; wherein R₅ is independently selected from unsubstituted C₁₋₆alkyl and unsubstituted C₃₋₆cycloalkyl; Y is independently selected from N and CR₄; R₄ is halogen; R₆ is independently selected from hydrogen, and deuterium; or
X is O or CH₂; L is CH; G is S; A is CH or N; R₃ is independently selected from amino, and 5-membered heteroaryl that is optionally substituted by C₁₋₄alkyl; R₁ is independently selected from hydrogen, deuterium, and amino; R₂ is independently selected from -CORs and -COORs; wherein R₅ is independently selected from unsubstituted C₁₋₄alkyl and unsubstituted C₃₋₆cycloalkyl; Y is independently selected from and CR₄; R₄ is halogen, for example, Cl or F, preferably Cl; R₆ is independently selected from hydrogen and deuterium; or
X is O or CH₂; L is CH; G is S; when A is CH: Y is CR₄; R₄ is halogen, for example, Cl or F, preferably Cl; or when A is N: Y is independently selected from N and CR₄; R₄ is halogen, for example Cl or F, preferably Cl; R₃ is independently selected from amino, and 5 membered heteroaryl that is optionally substituted by C₁₋₂alkyl (e.g., methyl); R₁ is independently selected from hydrogen, deuterium, and amino; R₂ is independently selected from -CORs and -COORs; wherein R₅ is independently selected from unsubstituted C₁₋₂alkyl (e.g., methyl) and unsubstituted C₃₋₄cycloalkyl (e.g., cyclopropyl); R₆ is independently selected from hydrogen and deuterium.

In some embodiments of this disclosure, the compound of formula (I), the compound of formula (I-1), the compound of formula (I-1-1), the compound of formula (I-2), the compound of formula (I-A1), the compound of formula (I-1-A1), the compound of formula (I-2-A1), the compound of formula (I-A2), the compound of formula (I-A3), the compound of formula (I-2-A2), the compound of formula (I-2-A3), the compound of formula (I-B1), the compound of formula (I-1-B1), the compound of formula (I-2-B1), the compound of formula (I-B2), the compound of formula (I-B3), the compound of formula (I-2-B2), the compound of formula (I-2-B3), the compound of formula (I-A), the compound of formula (I-B), the compound of formula (I-1-A), the compound of formula (I-1-B), the compound of formula (I-2-A), the compound of formula (I-2-B), and the compound of formula (I-Ba), as described above, all do not incude the following compounds:

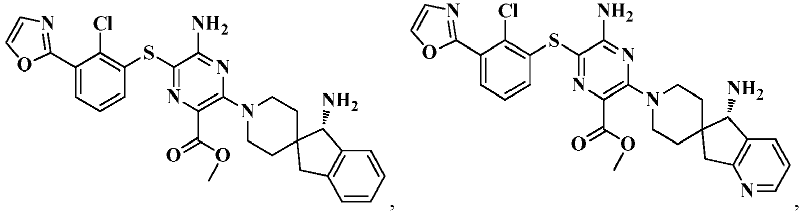

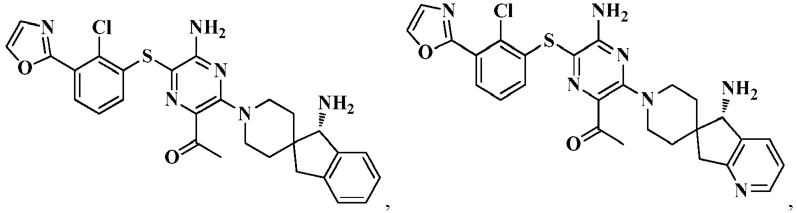

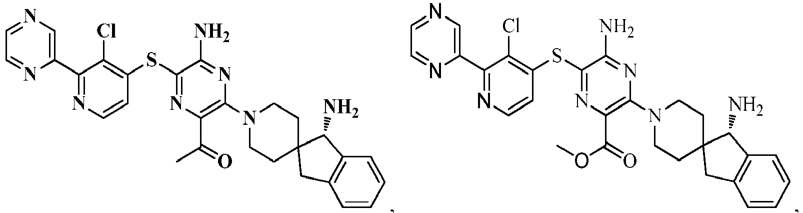

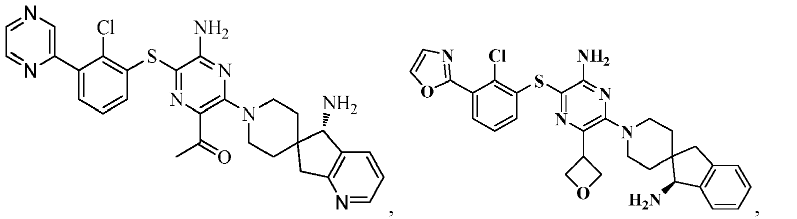

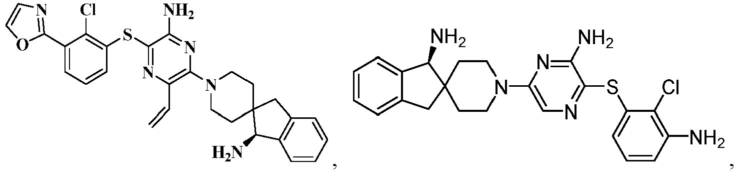

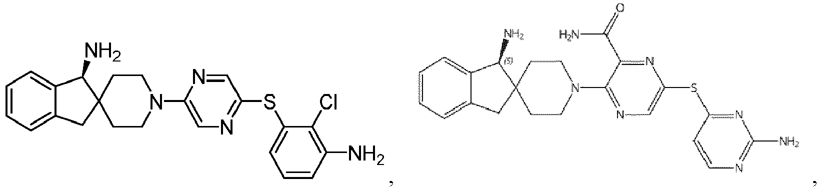

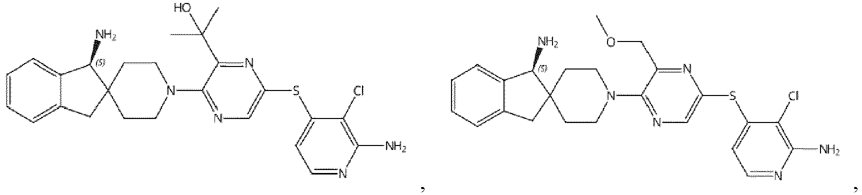

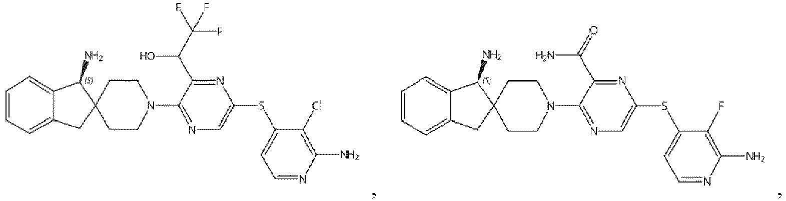

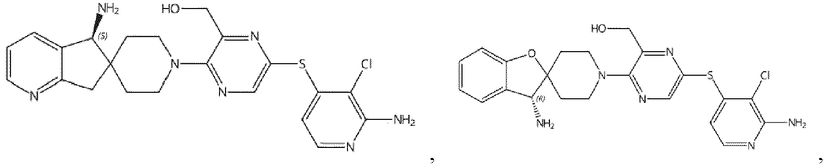

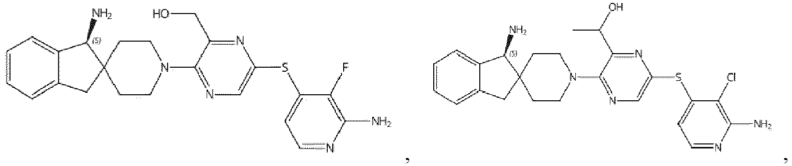

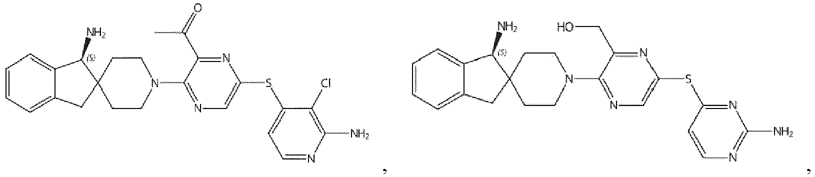

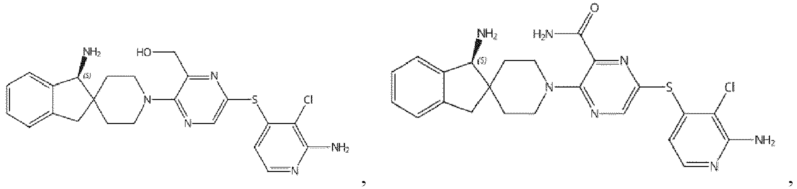

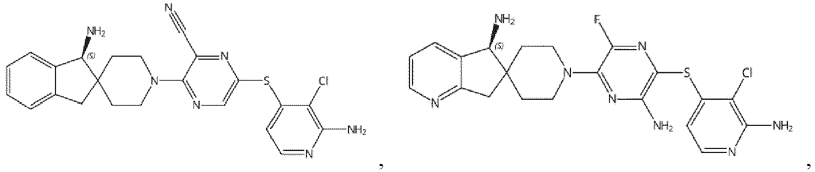

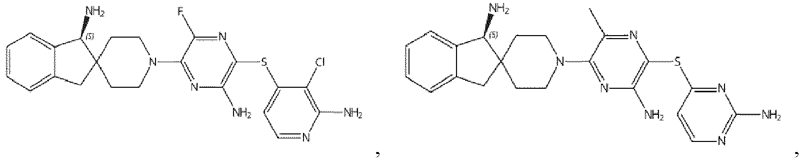

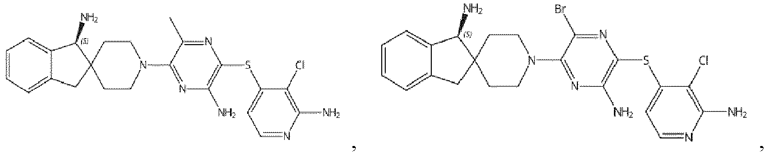

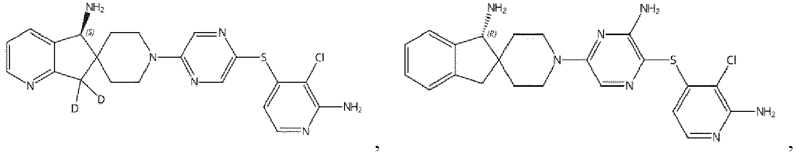

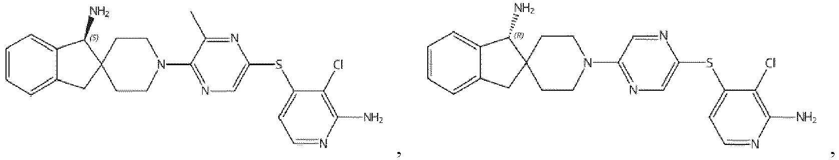

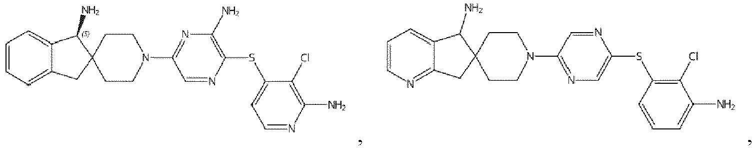

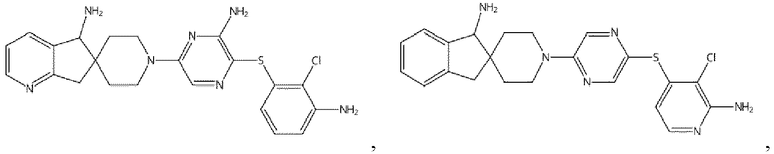

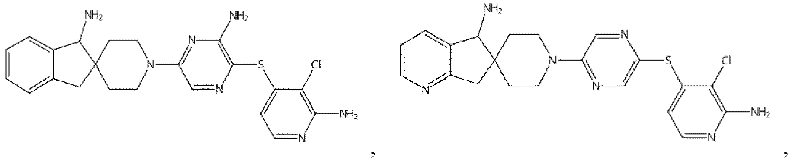

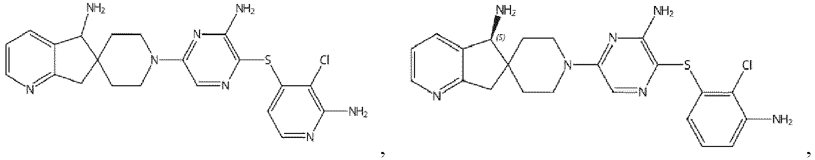

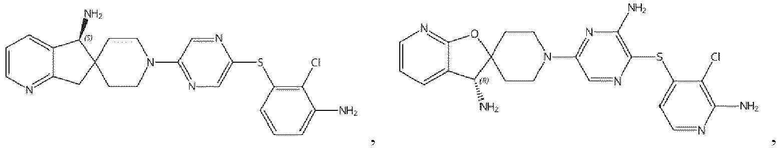

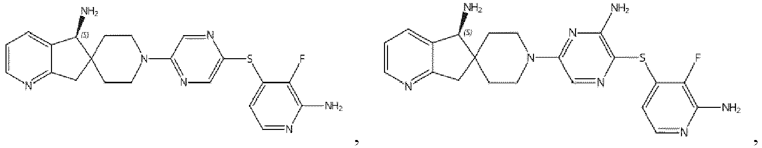

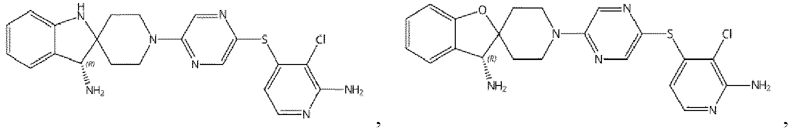

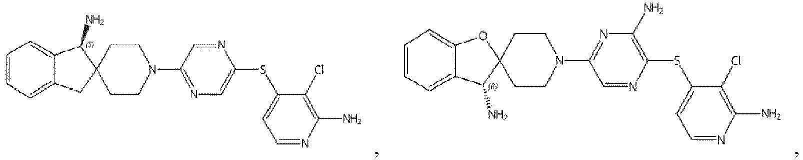

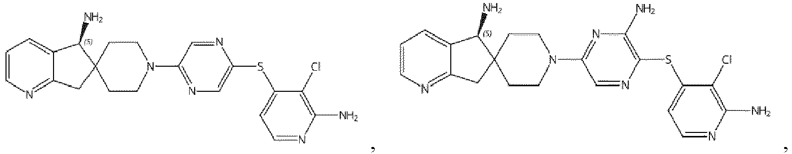

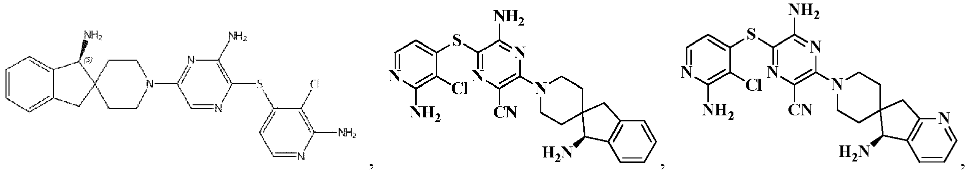

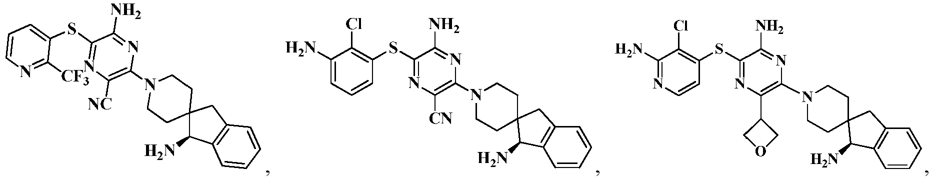

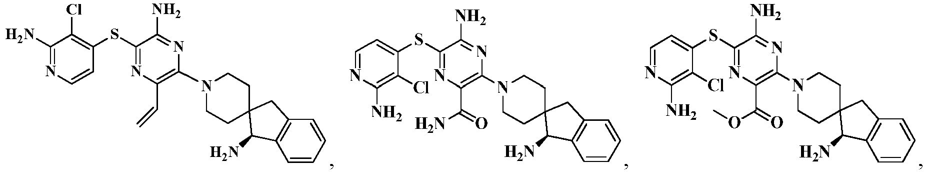

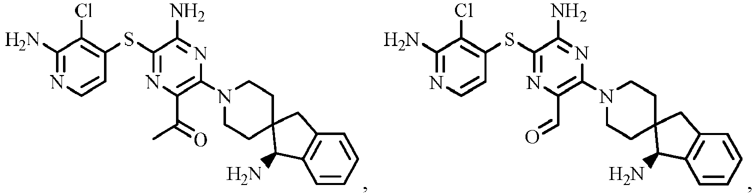

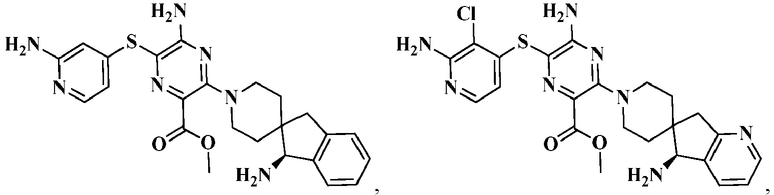

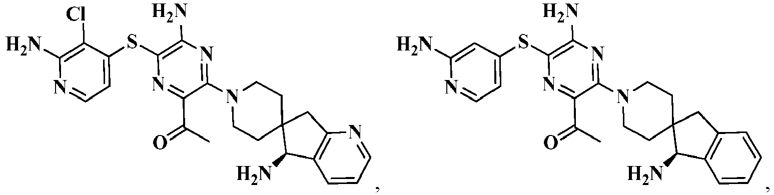

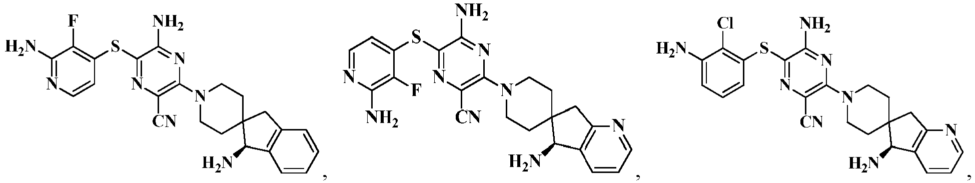

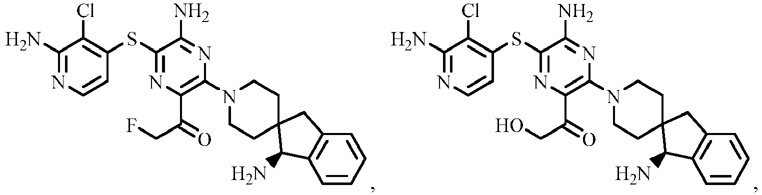

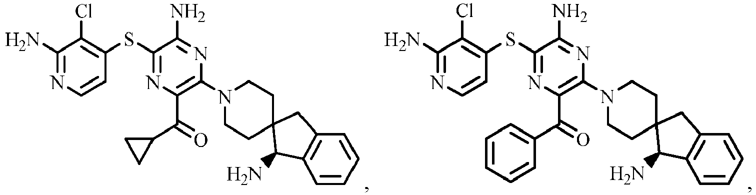

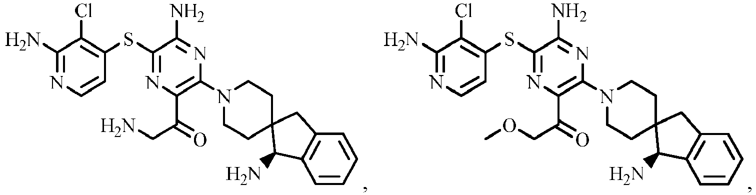

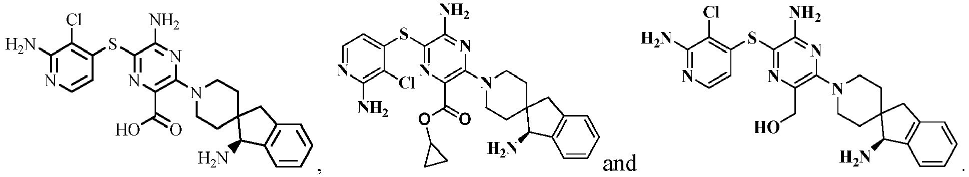

On the other hand, this disclosure provides a compound represented by formula (II), or a prodrug, tautomer, stereoisomer, solvate, isotope derivative, or pharmaceutically acceptable salt thereof, which compound has the following structure: 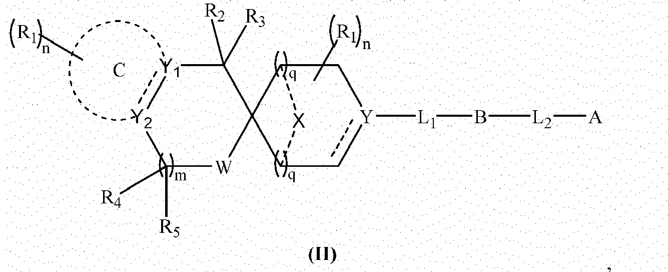 wherein,
each R₁ is independently selected from hydrogen, deuterium, halogen, -NH₂, -NHRₐ, -N(Rₐ)₂, -CN, -OH, - NO₂, oxo, =O, carboxyl, -C(O)-NH₂, -C(O)-NHRₐ, -C(O)Rₐ, -C(O)ORₐ, C₂₋₆alkenyl, C₂₋₆alkynyl, -C(O)-C(O)ORₐ-C₁₋₆alkoxyl, -C₁₋₆alkyl, -C₁₋₆haloalkyl, -C₃₋₁₀cycloalkyl, -C₁₋₆alkylene-N(Rₐ)₂, -C₁₋₆alkylene-O-C₁₋₆alkyl, -C₁₋₆alkylene-C(O)-ORₐ, -C₁₋₆alkylene-(3-10 membered heterocyclyl), -C₁₋₆alkylene-(5-10 membered heteroaryl), -C₁₋₆alkylene-C(O)-N(Rₐ)₂, -C₁₋₆alkylene-NRₐ-C(O)-N(Rₐ)₂, -C₁₋₆alkylene-NRₐ-C(O)-C₁₋₆alkyl, -C(O)-N(Rₐ)₂, -C(O)-C(O)-N(Rₐ)₂, -C₃₋₁₀ carbocyclyl, -5-10 membered heteroaryl, -3-10 membered heterocyclyl, -C(O)-C₁₋₆alkyl, -C(O)-C₃₋₆cycloalkyl, -C(O)-C₆₋₁₄aryl, -C(O)-C₅₋₁₀heteroaryl, - C(O)O-C₁₋₆alkyl, -C(O)-C₁₋₆alkylene-N(Rₐ)₂, -C(O)-NRₐ-(3-10 membered heterocyclyl), -C(O)-NRₐ-(3-10 membered heterocyclyl), -C(O)-(3-10 membered heterocyclyl), -O-C₁₋₆alkylene-C(O)-ORₐ, -O-C₁₋₆alkylene-C(O)-N(Rₐ)₂, -O-C₁₋₆alkylene-N(Rₐ)₂, -O-C₃₋₁₀carbocyclyl, -O-(3-10 membered heterocyclyl), - NRₐ-C(O)-C₁₋₆alkyl, -NRₐ-C(O)-N(Rₐ)₂, -NRₐ-C(O)-(5-10 membered heteroaryl), -NRₐ-C(O)-(5-10 membered heterocyclyl), -NRₐ-C(O)-C₃₋₈cycloalkyl, -NRₐ-C₁₋₆alkylene-N(Rₐ)₂, -NRₐ-C₁₋₆alkylene-(3-10 membered heterocyclyl), -NRₐ-C₁₋₆alkylene-(5-10 membered heteroaryl), -NRₐ-SO₂-C₁₋₆alkyl, -S-C₁₋₆alkyl, -SON(Rₐ)₂, -SO₂N(Rₐ)₂, -SO-C₁₋₆alkyl, -SO₂-C₁₋₆alkyl, -PO(C₁₋₆alkyl)₂, -PO(C₁₋₆alkyloxy)₂, -3-10 membered heterocyclyl, -5-14 membered aryl and -5-14 membered heteroaryl, each of which is independently optionally substituted;
or two adjacent R₁s cyclize to form C₆₋₁₀aryl, 5-10 membered heteroaryl, 3-10 membered carbocyclyl, or 3-10 membered heterocyclyl, each of which is independently optionally substituted;
or two adjacent Rₐs are attached together to form 5-14 membered aryl ring, 5-14 membered heteroaryl ring, 5-14 membered heterocyclic ring, or 5-14 membered carbocyclic ring, and each of said ring systems is independently optionally substituted;
each Rₐ is independently selected from hydrogen, deuterium, halogen, -NH₂, -CN, -OH, -NO₂, carboxyl, - C₁₋₆ alkoxyl, and -C₁₋₆alkyl, each of which is independently optionally substituted;
R₃ is deuterium;
R₂ is selected from hydrogen, deuterium, halogen, -NH₂, -CN, -OH, -NO₂, -COOH, -NH-C₁₋₆alkyl, -N(C₁₋₆alkyl)₂, -C₁₋₆alkoxyl and -C₁₋₆alkyl, and each of the above groups is independently optionally substituted or unsubstituted;
each of R₄ and R₅ is independently selected from hydrogen, deuterium, halogen, -NH₂, -CN, -OH, -NO₂, - COOH, -NH-C₁₋₆alkyl, -N(C₁₋₆alkyl)₂, -C₁₋₆alkoxyl and -C₁₋₆alkyl, and each of the above groups is independently optionally substituted or unsubstituted; or
R₄ and R₅, together with the carbon atom attached thereto, form 3-14 membered carbocyclic ring, 3-14 membered heterocyclic ring, 5-14 membered aryl ring, 5-14 membered heteroaryl ring or C=NR_{b}, and each of the above ring systems are independently optionally substituted or unsubstituted;
W is absent, -CH₂-, -CH(R_{b})-, -C(R_{b})₂-, -C(O)-, -NH-, -NR_{b}- or -O-;
Yis C, CH, -C(R_{b})- or N;
when Y is CH, -C(R_{b})- or N, its adjacent -̅ -̅ -̅ is a single bond; or when Y is C, its adjacent -̅ -̅ -̅ is a double bond;
each R_{b} is independently selected from hydrogen, deuterium, halogen, -NH₂, -CN, -OH, -C₁₋₆alkoxyl, and -C₁₋₆alkyl, and each of the above groups is independently optionally substituted or unsubstituted;
A is selected from aryl, heteroaryl, cycloalkyl, and heterocyclyl, which are unsubstituted or optionally substituted by one or more R_{1S};
B is selected from aryl, heteroaryl, cycloalkyl, and heterocyclyl, which are unsubstituted or optionally substituted by one or more R_{1S};
C is absent, or selected from aryl, heteroaryl, cycloalkyl, and heterocyclyl, which are unsubstituted or optionally substituted by one or more R_{1S};
when C is absent, Y₁ and Y₂ are each independently selected from -C(R₁)₂-, -C(R_{d})₂-, -NR_{d}-, -NR₁- and O; when -̅ -̅ -̅ in Y₁-̅ -̅Y₂ represents a single bond, Y₁ is -C(R₁)₂-, -C(R_{d})₂-, -NR_{d}-, -NR₁- or O, and Y₂ is -C(R₁)₂-, -C(R_{d})₂-, -NR_{d}-, -NR₁- or O; or, when -̅ -̅ -̅ in Y₁-̅ -̅Y₂ represents a double bond, Y₁ is CR₁, CR_{d} or N, and Y₂ is CR₁, CR_{d} or N;
when C is aryl ring, heteroaryl ring, cycloalkyl or heterocycloalkyl; when -̅ -̅ -̅ in Y₁-̅ -̅Y₂ represents a single bond, Y₁ is CR_{d}, C-R₁ or N, and Y₂ is CR_{d} or N; or, when -̅ -̅ -̅ in Y₁-̅ -̅Y₂ represents a double bond, Y₁ is C, and Y₂ is C;
each of L₁ and L₂ is independently selected from a covalent bond, -S-, -S(O)-, -S(O)₂-, -O-, -C(O)-, -CO-NR_{d}-, -NR_{d}-, -NR_{d}-C(O)-, -NR_{d}-C(O)-NR_{d}-, -C(O)O-, -NR_{d}SO₂-, -C(R_{d})₂-, -SO₂NR_{d}- and 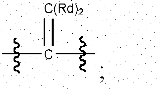 each R_{d} is independently selected from hydrogen, deuterium, halogen, -NH₂, -CN, -OH, -NO₂, -COOH, - CO-C₁₋₆alkyl, -COO-C₁₋₆alkyl, -C₁₋₆alkylene-O-C₁₋₆alkoxyl, -C₁₋₆alkoxyl and -C₁₋₆alkyl, and each of the above groups is independently optionally substituted or unsubstituted;
---represents the bond is present or absent; and
   i) when ---represents the bond is absent, X is absent;
   ii) when ---represents the bond is present, X is CH₂;
each of m, n, and q is independently selected from 0, 1, 2, 3, and 4; when m is 0, W is directly attached to Y2;
the above substitution is a substitution by one or more substituents independently selected from -OH, halogen, unsubstituted C₁₋₆ alkyl, -NH₂, -NO₂, unsubstituted -COC₁₋₆alkyl, -CN,=O, -COOH, -COCH₃-, - COOCH₃-, -CONH₂, unsubstituted C₃₋₁₀cycloalkyl, unsubstituted -NH-C₁₋₆alkyl, unsubstituted -N(C₁₋₆alkyl)(C₁₋₆alkyl), unsubstituted -NH-C₃₋₆cycloalkyl, unsubstituted -N(C₃₋₆cycloalkyl)(C₃₋₆cycloalkyl), unsubstituted 3-10 membered heterocycloalkyl, unsubstituted C₆₋₁₄aryl and unsubstituted 5-12 membered heteroaryl;
the substituent "oxo" or "=O" means that two hydrogen atoms on the same substitution site are substituted by an oxygen atom;
the heteroatoms in the above-mentioned heterocycloalkyl, heteroaryl, and heterocyclyl are independently selected from O, N, and S, and the number of heteroatoms is 1, 2, 3, or 4.

In an embodiment of this disclosure, in the compound represented by formula (II), said A is 3-14 membered cycloalkyl, 3-14 membered heterocycloalkyl, 5-14 membered aryl or 5-14 membered heteroaryl, each of which is optionally substituted or unsubstituted; preferably 5-12 membered cycloalkyl, 5-12 membered heterocycloalkyl, 5-12 membered aryl or 5-12 membered heteroaryl, each of which is optionally substituted or unsubstituted; further preferably 6-10 membered cycloalkyl, 6-10 membered heterocycloalkyl, 6-10 membered aryl or 6-10 membered heteroaryl, each of which is optionally substituted or unsubstituted; further preferably 6-10 membered aryl or 6-10 membered heteroaryl, each of which is optionally substituted or unsubstituted; further preferably phenyl, 8-10 membered polycyclic aryl, 5-6 membered monocyclic heteroaryl or 8-10 membered polycyclic heteroaryl, each of which is optionally substituted or unsubstituted; further preferably phenyl, 8-10 membered fused aryl, 8-10 membered bridged aryl, 8-10 membered spiro aryl, 5-6 membered monocyclic heteroaryl, 8-10 membered fused heteroaryl, 8-10 membered bridged heteroaryl or 8-10 membered spiro heteroaryl, each of which is optionally substituted or unsubstituted; further preferably phenyl, 8-10 membered fused aryl, 5-6 membered monocyclic heteroaryl or 8-10 membered fused heteroaryl, each of which is optionally substituted or unsubstituted; _wherein each of the above-mentioned heteroaryl and heterocyclic rings contains 1, 2, or 3 heteroatoms independently selected from N, O, and S;
further preferably, A is selected from the following groups that are optionally substituted or unsubstituted: 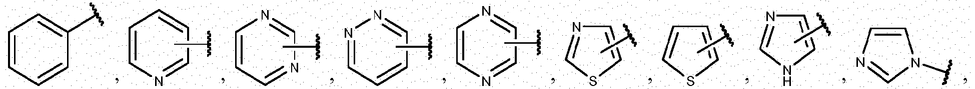 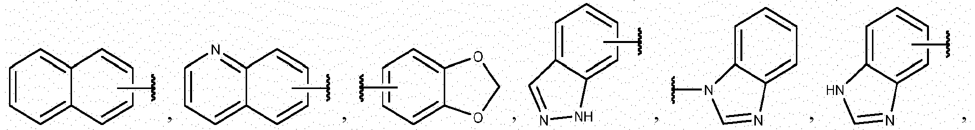 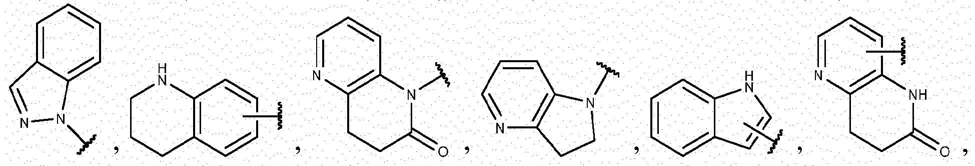 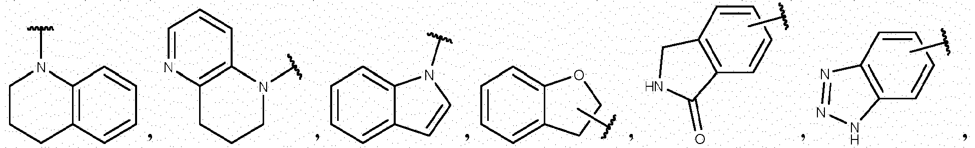 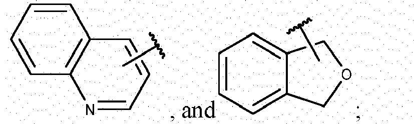 further preferably, A is selected from the following groups that are optionally substituted or unsubstituted: 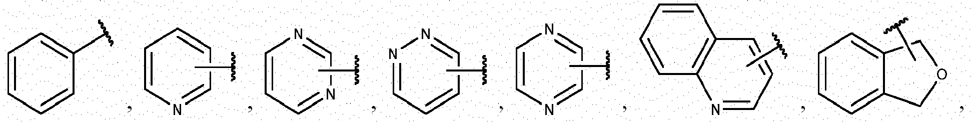 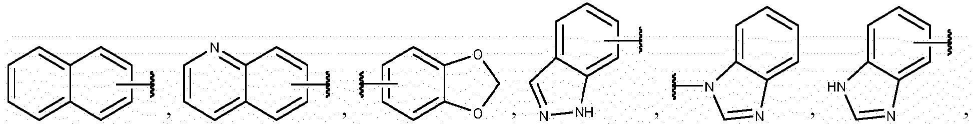 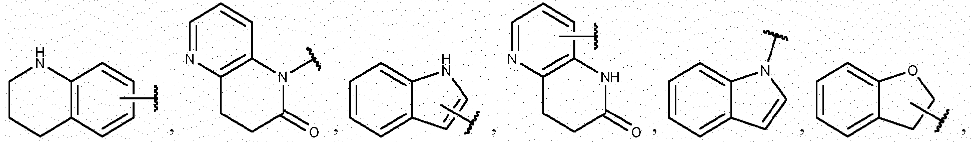 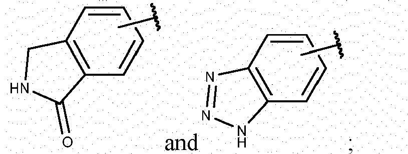 further preferably, A is selected from the following groups that are optionally substituted or unsubstituted: 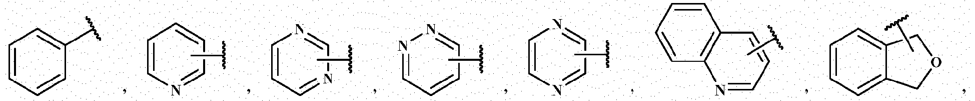 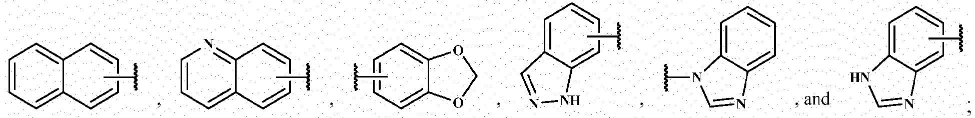 further preferably, A is selected from the following groups that are optionally substituted or unsubstituted: 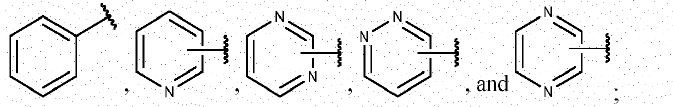 wherein said substitution is a substitution by one or more R_{1S}; 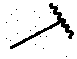 indicates the attachment to L₂.

In an embodiment of the compound represented by formula (II) of this disclosure, said L₂ is selected from covalent bond, -S-, -S(O)-, -S(O)₂-, -O-, -C(O)-, -NR_{d}- and -C(R_{d})₂-; preferably covalent bond, -S-, -O- and -NR_{d}-; further preferably covalent bond and -S-.

In an embodiment of the compound represented by formula (II) of this disclosure, said B is 3-14 membered cycloalkyl, 3-14 membered heterocycloalkyl, 5-14 membered aryl or 5-14 membered heteroaryl , each of which is optionally substituted or unsubstituted; preferably 5-12 membered cycloalkyl, 5-12 membered heterocycloalkyl, 5-12 membered aryl or 5-12 membered heteroaryl, each of which is optionally substituted or unsubstituted; further preferably 6-10 membered cycloalkyl, 6-10 membered heterocycloalkyl, 6-10 membered aryl or 6-10 membered heteroaryl, each of which is optionally substituted or unsubstituted; further preferably 6-10 membered aryl or 6-10 membered heteroaryl, each of which is optionally substituted or unsubstituted; further preferably phenyl, 8-10 membered polycyclic aryl, 5-6 membered monocyclic heteroaryl or 8-10 membered polycyclic heteroaryl, each of which is optionally substituted or unsubstituted; further preferably phenyl, 8-10 membered fused aryl, 8-10 membered bridged aryl, 8-10 membered spiro aryl, 5-6 membered monocyclic heteroaryl, 8-10 membered fused heteroaryl, 8-10 membered bridged heteroaryl or 8-10 membered spiro heteroaryl, each of which is optionally substituted or unsubstituted; further preferably phenyl, 8-10 membered fused aryl, 5-6 membered monocyclic heteroaryl or 8-10 membered fused heteroaryl, each of which is optionally substituted or unsubstituted; wherein each of the above-mentioned heteroaryl and heterocyclic rings contains 1, 2, or 3 heteroatoms independently selected from N, O, and S;
further preferably, B is selected from the following groups that are optionally substituted or unsubstituted: 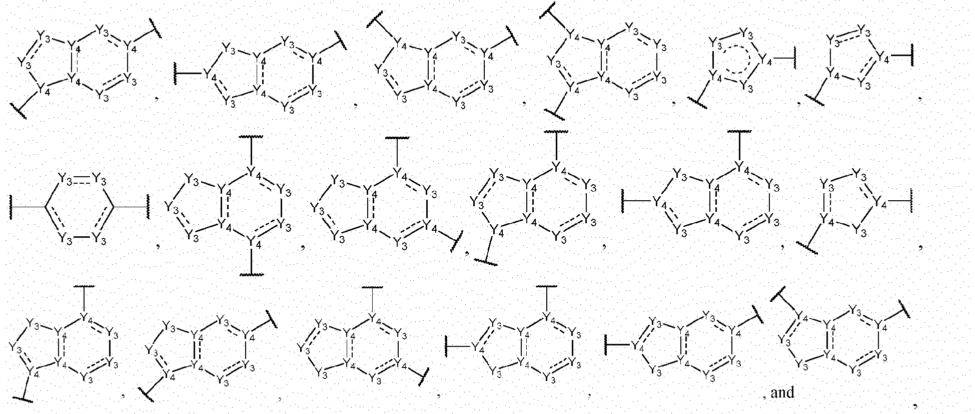 wherein,
each Y₃ is independently selected from C(R₁)₂, CR₁, NR₁, N, S and O;
each Y₄ is independently selected from CR₁ and NR₁;
when -̅ -̅ -̅ represents a single bond, Y₃ is C(R₁)₂, NR₁, S or O, and Y₄ is CR₁ or N; or, when -̅ -̅ -̅
represents a double bond, Y₃ is CR₁ or N, and Y₄ is C;
wherein said substitution is a substitution by one or more R_{1S}; 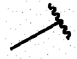 indicates the attachments to L₁ and L₂ respectively.

In an embodiment of the compound represented by formula (II) of this disclosure, B is a monocyclic ring such as substituted or unsubstituted pyridine, substituted or unsubstituted pyrazine, substituted or unsubstituted pyrimidine, substituted or unsubstituted thiazole, substituted or unsubstituted pyridinone, substituted or unsubstituted pyrimidinone, substituted or unsubstituted pyridazinone; or a two or three-ring fused ring system formed from pyridine ring, pyrazine ring, pyridazine ring, pyrimidine ring, thiazole ring, pyridinone ring, pyrimidinone ring, pyridazinone ring or the like, each of which is substituted or unsubstituted, with substituted or unsubstituted imidazole, substituted or unsubstituted triazole, substituted or unsubstituted pyrazole, substituted or unsubstituted indazole, substituted or unsubstituted oxazole or the like;
preferably, B is selected from the following groups that are optionally substituted or unsubstituted: 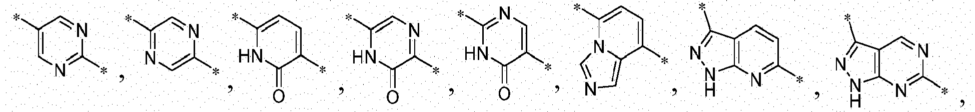 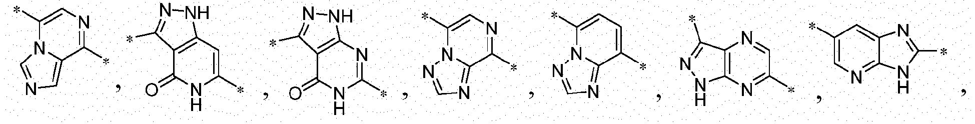 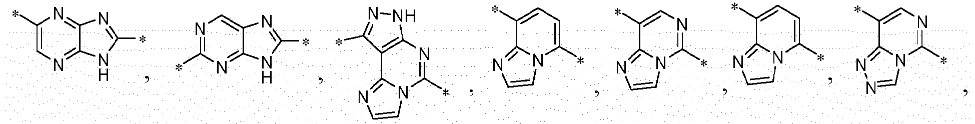 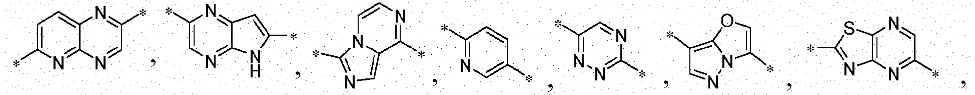 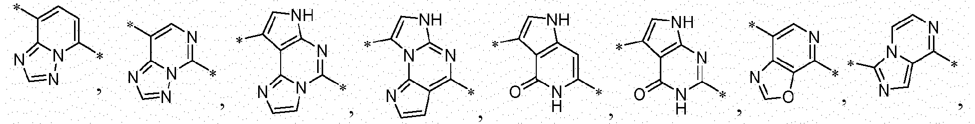 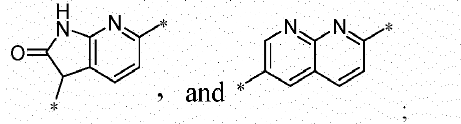 further preferably, B is selected from the following groups that are optionally substituted or unsubstituted: 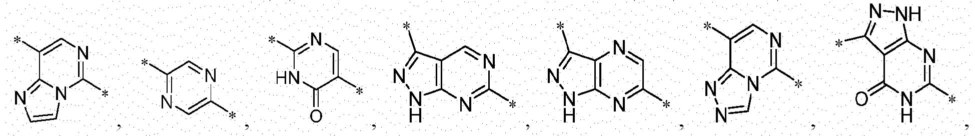 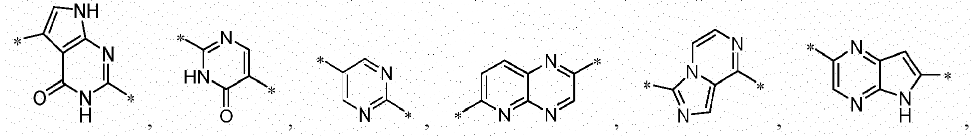 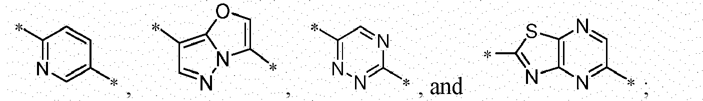 more further preferably 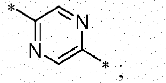 wherein said substitution is a substitution by one or more R_{1S}.

In an embodiment of the compound represented by formula (II) of this disclosure, said L₁ is selected from covalent bond, -S(O)₂-, -CO-NR_{d}-, -NR_{d}-C(O)-, -C(O)O-, -NR_{d}SO₂-, -SO₂NR_{d}- and 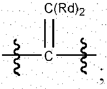 preferably covalent bond, -S(O)₂-, -CO-NR_{d}- and -NR_{d}-C(O)-; further preferably covalent bond.

In an embodiment of the compound represented by formula (II) of this disclosure, said Y is CH or N, and -̅ -̅ -̅ is a single bond; preferably N, and -̅ -̅ -̅ is a single bond.

In an embodiment of the compound represented by formula (II) of this disclosure, said W is -CH₂-, -CH(R_{b})-, -C(R_{b})₂- or -O-; preferably -CH₂- or -O-.

In an embodiment of the compound represented by formula (II) of this disclosure, said ring C is absent, or selected from 5-14 membered aryl, 5-14 membered heteroaryl, 5-14 membered carbocyclyl and 5-14 membered heterocyclyl, which are unsubstituted or optionally substituted by one or more R_{1S}; preferably, said ring C is absent, or selected from 5-12 membered aryl, 5-12 membered heteroaryl, 5-12 membered carbocyclyl and 5-12 membered heterocyclyl , which are unsubstituted or optionally substituted by one or more R_{1S}; further preferably, said ring C is absent, or selected from 5-10 membered aryl, 5-10 membered heteroaryl, 5-10 membered carbocyclyl and 5-10 membered heterocyclyl, which are unsubstituted or optionally substituted by one or more R_{1S}; further preferably, said ring C is absent, or selected from 6-10 membered aryl, 5-10 membered heteroaryl, 5-10 membered carbocyclyl and 5-10 membered heterocyclyl, which are unsubstituted or optionally substituted by one or more R_{1S}; further preferably, said ring C is absent, or selected from 6-10 membered monocyclic aryl, 6-10 membered fused aryl, 5-10 membered monocyclic heteroaryl, 5-10 membered fused heteroaryl, 5-7 membered monocyclic carbocyclyl, 7-10 membered fused carbocyclyl, 5-7 membered monocyclic heterocyclyl and 7-10 membered fused heterocyclyl, which are unsubstituted or optionally substituted by one or more R_{1S}; further preferably, said ring C is absent, or selected from phenyl, naphthyl, 5 membered monocyclic heteroaryl, 6 membered monocyclic heteroaryl, 7 membered monocyclic heteroaryl, 8 membered fused heteroaryl, 9 membered fused heteroaryl, 10 membered fused heteroaryl, 5 membered carbocyclyl, 6 membered carbocyclyl, 7 membered carbocyclyl, 8 membered fused carbocyclyl, 9 membered fused carbocyclyl, 10 membered fused carbocyclyl, 5 membered heterocyclyl, 6 membered heterocyclyl, 7 membered heterocyclyl, 8 membered fused heterocyclyl, 9 membered fused heterocyclyl and 10 membered fused heterocyclyl , which are unsubstituted or optionally substituted by one or more R₁s; wherein each of the above-mentioned heteroaryl and heterocyclic rings contains 1, 2, or 3 heteroatoms independently selected from N, O, and S;
further preferably, said ring C is absent, or selected from the following groups that are unsubstituted or optionally substituted by one or more R₁s: 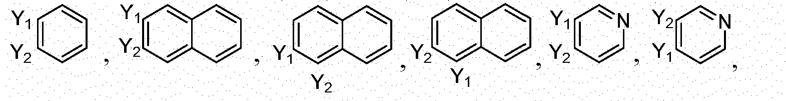 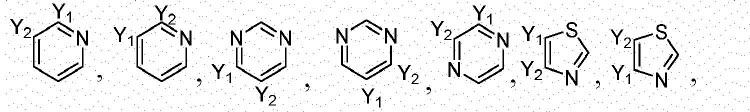 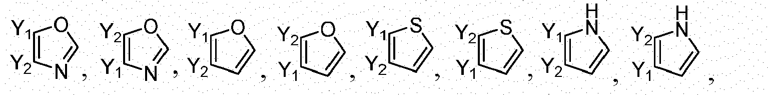 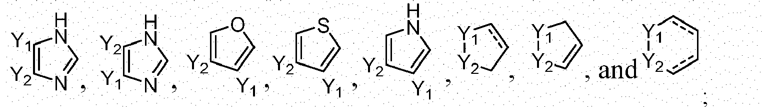 preferably the following groups that are unsubstituted or optionally substituted by one or more R₁s: 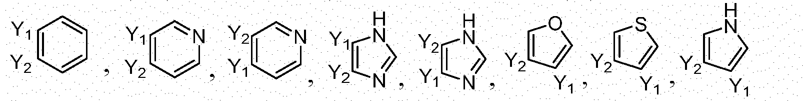 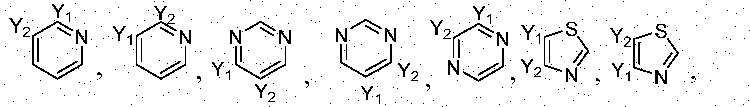 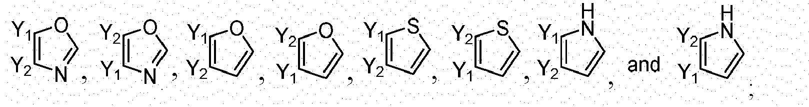 further preferably the following groups that are unsubstituted or optionally substituted by one or more R₁s: 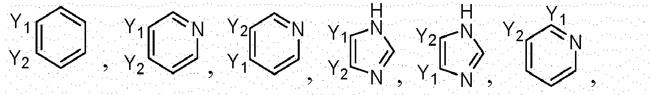 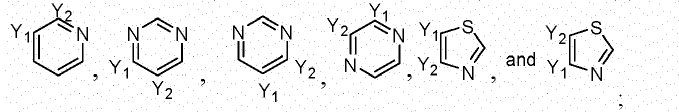 further preferably the following groups that are unsubstituted or optionally substituted by one or more R₁s: 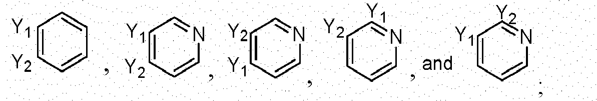 wherein -̅ -̅ -̅ represents a single bond or a double bond.

In an embodiment of the compound represented by formula (II) of this disclosure, when -̅ -̅ -̅ in Y₁-̅ -̅Y₂ represents a single bond, Y₁ is -CR_{d}- or -N-, and Y₂ is -CR_{d}- or -N-.

In an embodiment of the compound represented by formula (II) of this disclosure, when ring C is absent, Y₁ and Y₂ each independently are -C(R₁)₂- or -C(R_{d})₂-.

In an embodiment of the compound represented by formula (II) of this disclosure, each R₁ is independently selected from hydrogen, deuterium, halogen, -N(Rₐ)₂, -CN, -OH, -NO₂, oxo, =O, carboxyl, -C₁₋₆alkoxyl, - C₁₋₆alkyl, -C₁₋₆haloalkyl, -C₃₋₈cycloalkyl, -C₁₋₆alkylene-N(Rₐ)₂, -C₁₋₆alkylene-O-C₁₋₆alkyl, -C₁₋₆alkylene-C(O)-ORₐ, -C₁₋₆alkylene-(3-10 membered heterocyclyl), -C₁₋₆alkylene-(5-10 membered heteroaryl), -C₁₋₆alkylene-C(O)-N(Rₐ)₂, -C₁₋₆alkylene-NRₐ-C(O)-C₁₋₆alkyl, -C(O)-N(Rₐ)₂, -C(O)-C(O)-N(Rₐ)₂, -C₃₋₁₀carbocyclyl, -5-10 membered heteroaryl, -3-10 membered heterocyclyl, -C(O)-C₁₋₆alkyl, -C(O)O-C₁₋₆alkyl, -C(O)-C₁₋₆alkylene-N(Rₐ)₂, -C(O)-NRₐ-(3-10 membered heterocyclyl), -C(O)-NRₐ-(3-10 membered heterocyclyl), -C(O)-(3-10 membered heterocyclyl), -O-C₁₋₆alkylene-C(O)-ORₐ, -O-C₃₋₁₀carbocyclyl, -O-(3-10 membered heterocyclyl), -NRₐ-C(O)-C₁₋₆alkyl, -NRₐ-C(O)-(5-10 membered heteroaryl), -NRₐ-C(O)-C₃₋₈cycloalkyl, -NRₐ-C₁₋₆alkylene-N(Rₐ)₂, -NRₐ-C₁₋₆alkylene-(3-10 membered heterocyclyl), -NRₐ-C₁₋₆alkylene-(5-10 membered heteroaryl), -NRₐ-S(O)₂ C₁₋₆alkyl, -S(O)-N(Rₐ)₂, -S(O)₂-N(Rₐ)₂, -3-10 membered heterocyclyl and -5-10 membered heteroaryl; preferably, each R₁ is independently selected from -H, -F, -Cl, -Br, -NH₂, -N(CH₃)₂, -CN, -OH, oxo, =O, carboxyl, methoxy, ethyloxy, methyl, ethyl, isopropyl, tert-butyl, -CH₂NH₂, -CH₂CH₂OCH₃, -CH₂-C(O)OH, -CH₂NH-C(O)-NHCH₃, -C(O)-NH₂, -C(O)-N(CH₃)₂, -C(O)-NHOH, -C(O)-NHCH₂CH₂OH, -C(O)-CH₃, -C(O)O-CH₃, -S(O)₂-NH₂, -NHS(O)₂CH₃, -NH-C(O)-CH₃, -NH-C(O)-NHCH₃, 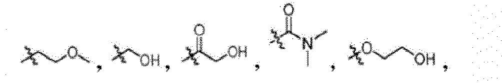 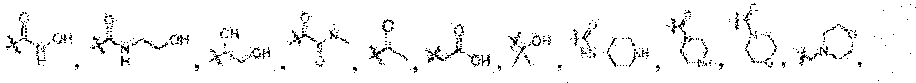 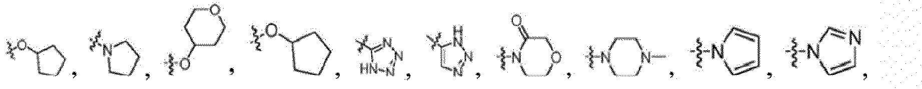 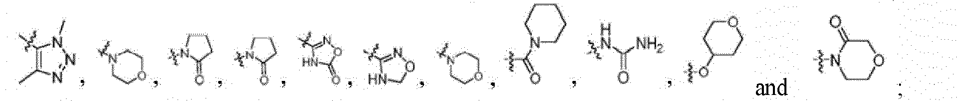
wherein each of the above R₁s is independently optionally substituted by 1, 2, or 3 substituents selected from -F, -Cl, -NH₂, -OH, oxo, =O, methyl, ethyl, and propyl;
or two adjacent Rₐs are attached together to form 5-14 membered aryl ring, 5-14 membered heteroaryl ring, 5-14 membered heterocyclic ring, or 5-14 membered carbocyclic ring, and each of said ring systems is independently optionally substituted; preferably, two adjacent Rₐs are attached together to form 6-10 membered aryl ring, 5-10 membered heteroaryl ring, 5-10 membered heterocyclic ring or 5-10 membered carbocyclic ring, and each of said ring systems is independently optionally substituted;
or Rₐ and R_{b}, together with the atom attached thereto, form 3-14 membered aryl ring, 3-14 membered heteroaryl ring, 3-14 membered heterocyclic ring or 3-14 membered carbocyclic ring; and each of said ring systems is independently optionally substituted; preferably, Rₐ and R_{b}, together with the atom attached thereto, form 6-10 membered aryl ring, 5-10 membered heteroaryl ring, 5-10 membered heterocyclic ring or 5-10 membered carbocyclic ring; and each of said ring systems is independently optionally substituted; each Rₐ is independently selected from hydrogen, deuterium, halogen, -NH₂, -CN, -OH, -NO₂, carboxyl, - C₁₋₆alkoxyl, and -C₁₋₆alkyl; each of which is independently optionally substituted; preferably, each Rₐ is independently selected from hydrogen, deuterium, halogen, -NH₂, -CN, -OH, -C₁₋₆alkoxyl, and -C₁₋₆alkyl; each of which is independently optionally substituted.

In an embodiment of the compound represented by formula (II) of this disclosure, R₁ on ring B is independently selected from hydrogen, deuterium, amino, halogen, cyano, -CONH₂, -COR₆, -COOR₆, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl; preferably hydrogen, halogen, amino, cyano, -CONH₂, -COR₆, -COOR₆, optionally substituted C₁₋₄alkyl, optionally substituted C₂₋₆alkenyl, optionally substituted C₃₋₆cycloalkyl, and optionally substituted 3-6 membered heterocycloalkyl; further preferably R₁ on ring B is independently selected from halogen, cyano, -CONH₂, -COR₆, -COOR₆, hydroxy-substituted C₁₋₄alkyl, C₂₋₆alkenyl, C₃₋₆cycloalkyl, and 3-6 membered heterocycloalkyl; wherein R₆ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl; preferably, R₆ is independently selected from hydrogen, optionally substituted C₁₋₆alkyl, optionally substituted C₃₋₆cycloalkyl, and optionally substituted 3-6 membered heterocycloalkyl; further preferably, R₅ is independently selected from hydrogen, C₁₋₆alkyl, C₃₋₆cycloalkyl, and 3-6 membered heterocycloalkyl;
said "optionally substituted" means that the hydrogen on the group to be substituted is unsubstituted or the hydrogen(s) on one or more substitution sites of the group to be substituted is/are independently substituted by a substituent selected from a group consisting of -OH, halogen, -NH₂, -NO₂, C₁₋₆alkyl, C₁₋₆alkoxyl, C₃₋₆cycloalkyl, -NH-C₁₋₆ alkyl, -N(C₁₋₆alkyl)-C₁₋₆alkyl, -NH-C₃₋₆cycloalkyl, -N(C₃₋₆cycloalkyl)-C₃₋₆cycloalkyl, 3-6 membered heterocycloalkyl, C₆₋₁₄aryl and 5-10 membered heteroaryl; preferably by a substituent selected from a group consisting of halogen, hydroxy, amino, nitro, oxo, cyano, C ₁₋₆alkyl, and C₁₋₆alkoxyl.

In an embodiment of the compound represented by formula (II) of this disclosure, R₁ on ring A is independently selected from optionally substituted 8-12 membered heterocyclyl, optionally substituted C₆₋₁₀aryl, optionally substituted 5-14 membered heteroaryl; said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, -COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl; In an embodiment of the compound represented by formula (II) of this disclosure, R₁ on ring C is independently selected from hydrogen, halogen, -NH₂, -NHR₇, -N(R₇)₂, -CN, -NO₂,=O, -C(O)-OR₇, -C(O)-NH₂, -CO-NHR₇, -CO-N(R₇)₂, -C(O)R₇, -COOH, -OH, -SC₁₋₆alkyl, -SOC₁₋₆alkyl, -S(O)₂C₁₋₆alkyl, substituted or unsubstituted C₁₋₆alkyl, and substituted or unsubstituted C₁₋₆alkoxyl; wherein R₇ is each independently C₁₋₆alkyl; the substitution refers to a substitution by one or more substituents independently selected from -OH, halogen, C₁₋₆alkyl, -NH₂, -NO₂, -COCH₃, -CN and =O, and it means where the substituent is "=O" that two hydrogen atoms on the same substitution site are substituted by an oxygen atom.

In an embodiment of the compound represented by formula (II) of this disclosure, R₂ is selected from hydrogen, deuterium, halogen, -NH₂, -OH, -NH-C₁₋₆alkyl, -N(C₁₋₆alkyl)₂, -C₁₋₆alkoxyl and -C₁₋₆alkyl, and each of the above groups is independently optionally substituted or unsubstituted; preferably R₂ is selected from hydrogen, deuterium, halogen, -NH₂, -OH, -C₁₋₃alkoxyl and -C₁₋₃alkyl, and each of the above groups is independently optionally substituted or unsubstituted; further preferably R₂ is selected from hydrogen, - NH₂ and -OH, and each of the above groups is independently optionally substituted or unsubstituted; further preferably R₂ is selected from -NH₂.

In an embodiment of the compound represented by formula (II) of this disclosure, each of R₄ and R₅ is independently selected from hydrogen, deuterium, halogen, -NH₂, -CN, -OH, -C₁₋₆alkoxyl and -C₁₋₆alkyl, and each of the above groups is independently optionally substituted or unsubstituted; preferably, each of R₄ and R₅ is independently selected from hydrogen, deuterium, halogen, -NH₂, -CN, -OH, -C₁₋₃alkoxyl and -C₁₋₃alkyl, and each of the above groups is independently optionally substituted or unsubstituted; further preferably, each of R₄ and R₅ is independently selected from hydrogen, halogen, -OH, -CH₃ and -OCH₃; further preferably, R₄ and R₅ are both hydrogen.

In an embodiment of the compound represented by formula (II) of this disclosure, m is 0 or 1; q is 1; n is 1, 2, or 3; when m is 0, W is directly attached to Y₂.

In an embodiment of the compound represented by formula (II) of this disclosure, - - - represents the bond is absent.

In an embodiment of the compound represented by formula (II) of this disclosure, the above substitution is a substitution by one or more substituents independently selected from -OH, halogen, unsubstituted C₁₋₆ alkyl, -NH₂, -NO₂, unsubstituted -COC₁₋₆alkyl, -CN,=O, -C(O)OH, -C(O)-CH₃-, -C(O)OCH₃-, -C(O)-NH₂, unsubstituted C₃₋₁₀cycloalkyl, unsubstituted -NH-C₁₋₆alkyl, unsubstituted -N(C₁₋₆alkyl)(C₁₋₆alkyl), unsubstituted -NH-C₃₋₆cycloalkyl, unsubstituted -N(C₃₋₆cycloalkyl)(C₃₋₆cycloalkyl), unsubstituted 3-10 membered heterocycloalkyl, unsubstituted C₆₋₁₄aryl, and unsubstituted 5-12 membered heteroaryl; preferably -OH, halogen, unsubstituted C₁₋₆alkyl, -NH₂, -NO₂, unsubstituted -COC₁₋₆alkyl, -CN,=O, - C(O)OH, -C(O)-CH₃-, -C(O)OCH₃-, -C(O)-NH₂, unsubstituted -NH-C₁₋₆alkyl, and unsubstituted -N(C₁₋₆alkyl)(C₁₋₆alkyl).

In an embodiment of the compound represented by formula (II) of this disclosure, each R_{b} is independently selected from hydrogen, deuterium, halogen, -NH₂, -CN, -OH, -C₁₋₆alkoxyl and -C₁₋₆alkyl; preferably each R_{b} is independently selected from hydrogen, deuterium, halogen, -NH₂, -CN, -OH, -C₁₋₃alkoxyl and -C₁₋₃alkyl.

In an embodiment of the compound represented by formula (II) of this disclosure, each R_{d} is independently selected from hydrogen, deuterium, halogen, -NH₂, -CN, -OH, -NO₂, -C(O)OH, -C(O)-C₁₋₆alkyl, -C(O)-O-C₁₋₆alkyl, -C₁₋₆alkylene-O-C₁₋₆alkoxyl, -C₁₋₆alkoxyl and -C₁₋₆alkyl; preferably each R_{d} is independently selected from hydrogen, deuterium, halogen, -NH₂, -CN, -OH, -NO₂, -C(O)OH, -C(O)-C₁₋₃alkyl, -C(O)-O-C₁₋₃alkyl, -C₁₋₃alkylene-O-C₁₋₃alkoxyl, -C₁₋₃alkoxyl and -C₁₋₃alkyl; further preferably each R_{d} is independently selected from hydrogen, deuterium, halogen, -NH₂, -CN, -OH, -NO₂, -C(O)OH, -C(O)-C₁₋₃alkyl, and -C₁₋₃alkylene-O-C₁₋₃alkoxyl.

In some embodiments of this disclosure, there are provided the following compounds or prodrugs, tautomers, stereoisomers, solvates, isotope derivatives, or pharmaceutically acceptable salts thereof:

| Comp. No. | Structure | Comp. No. | Structure |
|---|---|---|---|
| 1 | 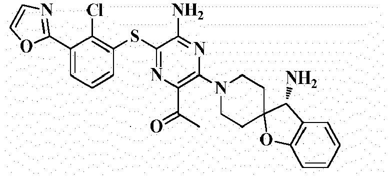 | 2 | 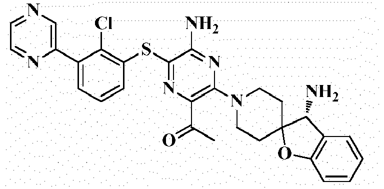 |
| 3 | 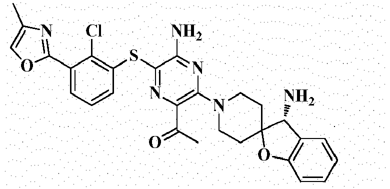 | 4 | 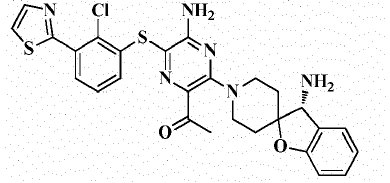 |
| 5 | 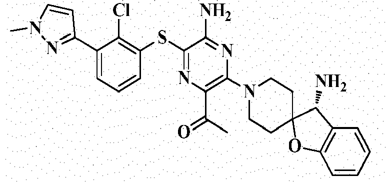 | 6 | 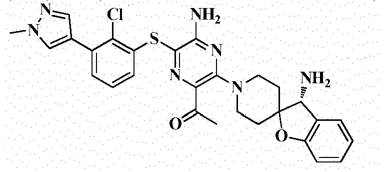 |
| 7 | 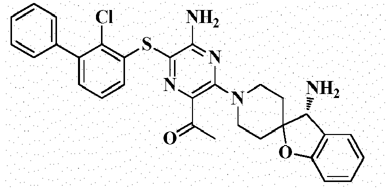 | 8 | 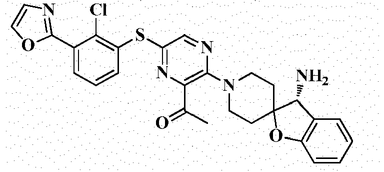 |
| 9 | 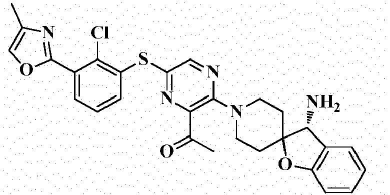 | 10 | 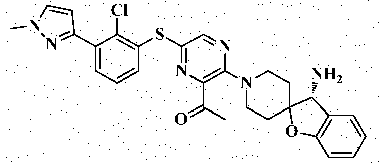 |
| 11 | 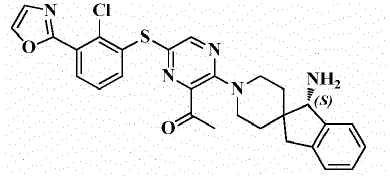 | 11A | 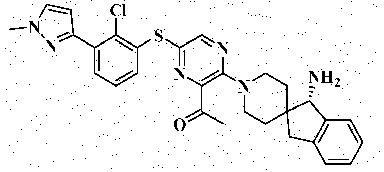 |
| 12 | 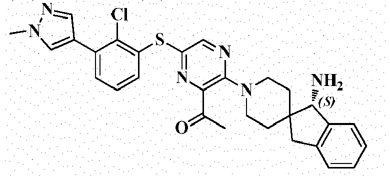 | 13 | 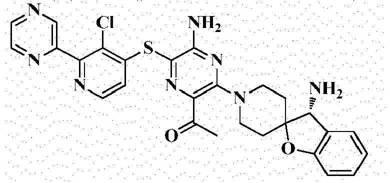 |
| 14 | 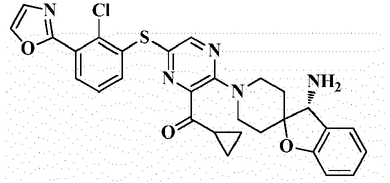 | 15 | 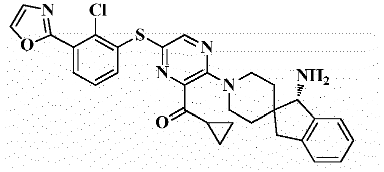 |
| 16 | 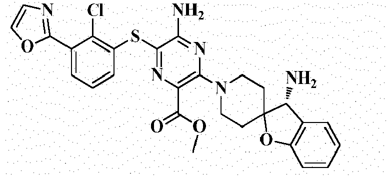 | 17 | 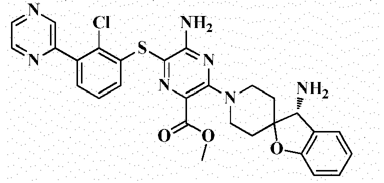 |
| 18 | 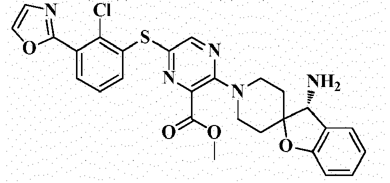 | 19 | 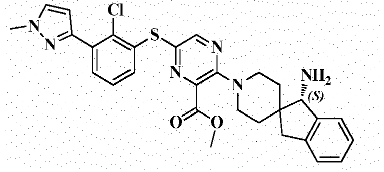 |
| 20 | 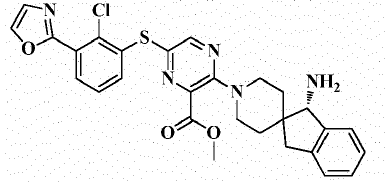 | 21 | 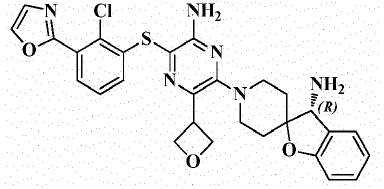 |
| 22 | 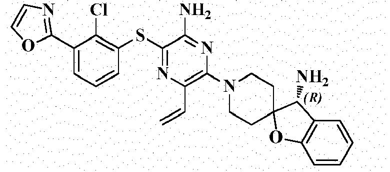 | 23 | 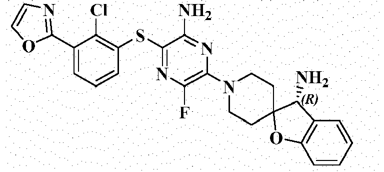 |
| 24 | 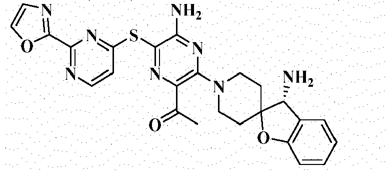 | 25 | 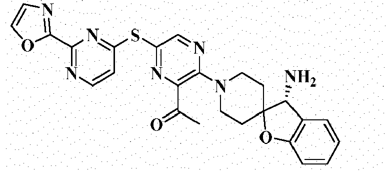 |
| 26 | 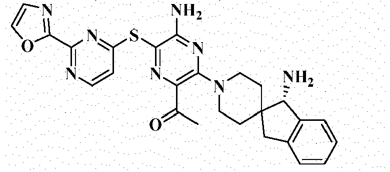 | 27 | 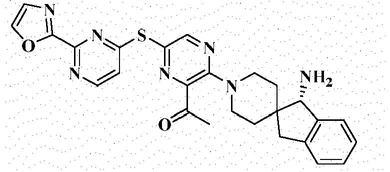 |
| 28 | 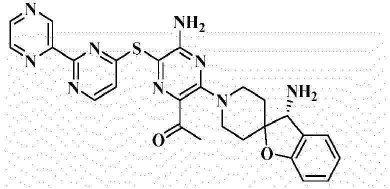 | 29 | 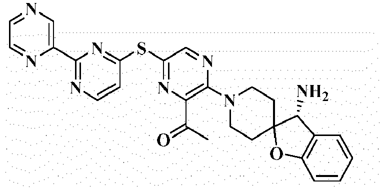 |
| 30 | 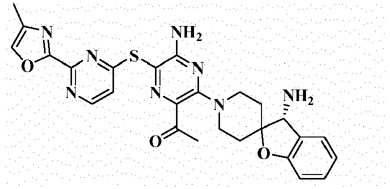 | 31 | 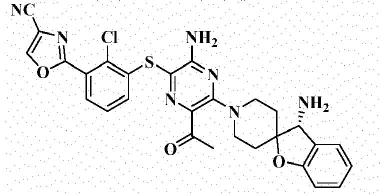 |
| 32 | 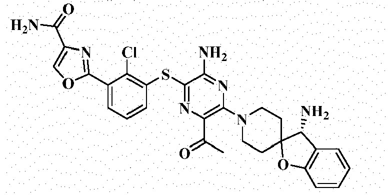 | 33 | 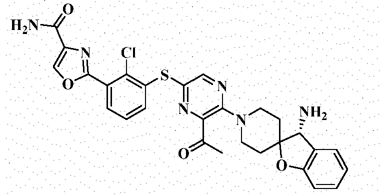 |
| 34 | 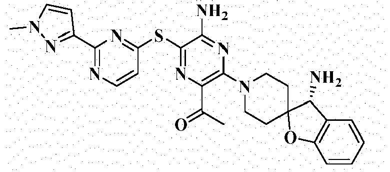 | 35 | 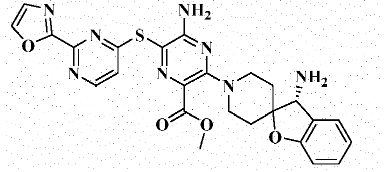 |
| 36 | 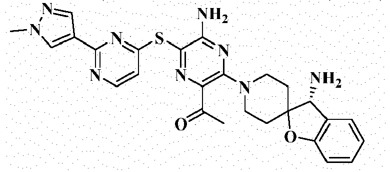 | 37 | 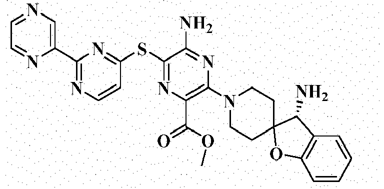 |
| 38 | 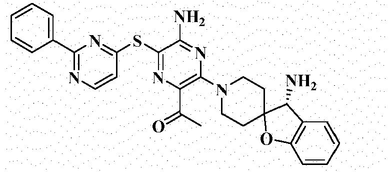 | 39 | 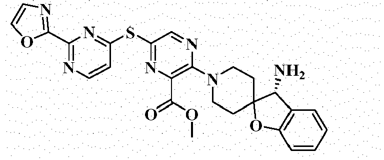 |
| 40 | 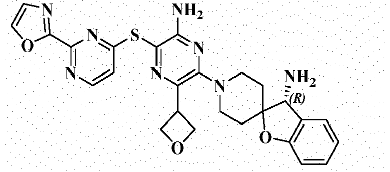 | 41 | 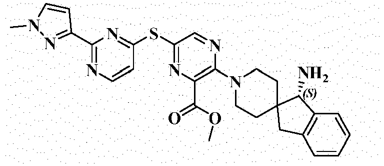 |
| 42 | 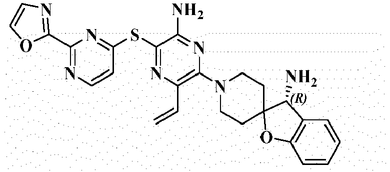 | 43 | 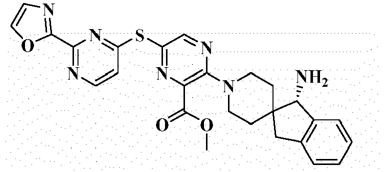 |
| 44 | 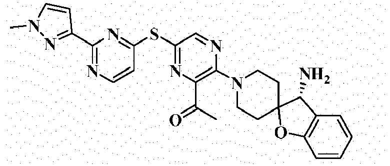 | 45 | 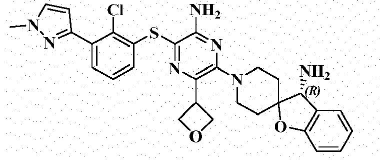 |
| 46 | 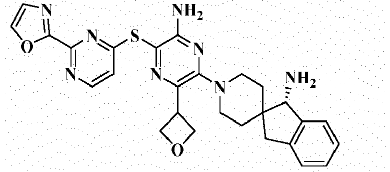 | 47 | 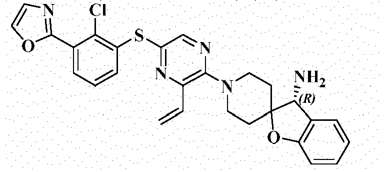 |
| 48 | 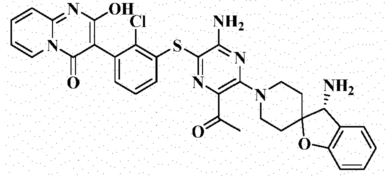 | 49 | 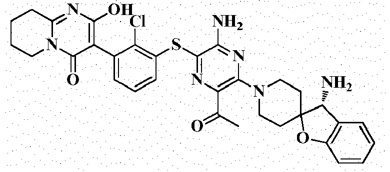 |
| 50 | 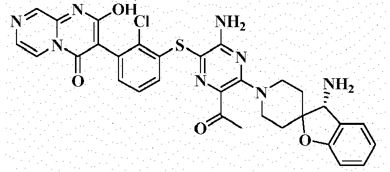 | 51 | 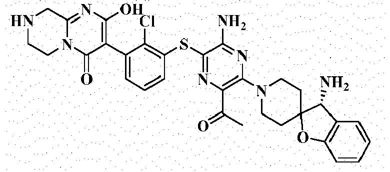 |
| 52 | 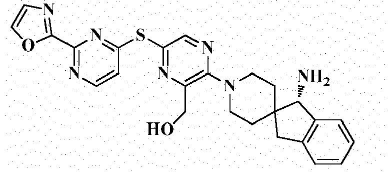 | 53 | 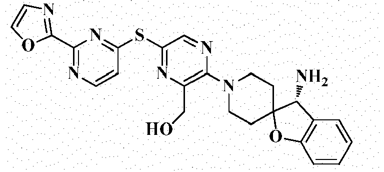 |
| 54 | 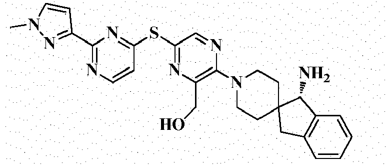 | 55 | 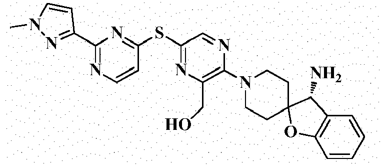 |
| 56 | 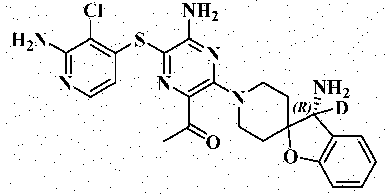 | 57 | 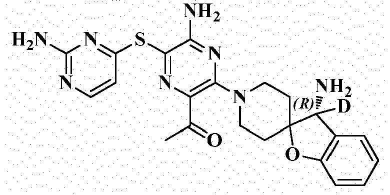 |
| 58 | 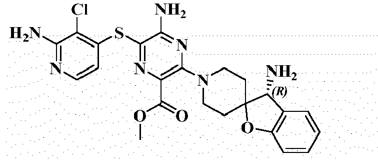 | 59 | 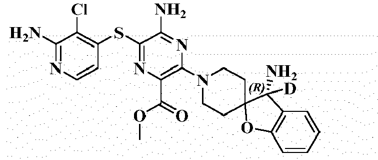 |
| 60 | 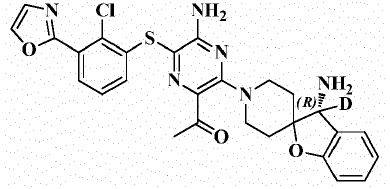 | 61 | 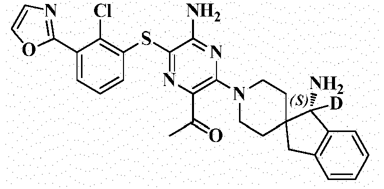 |
| 56A | 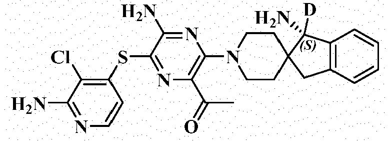 | 62 | 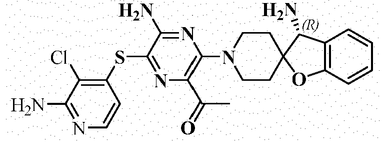 |

In some preferable embodiments of this disclosure, there are provided the following compounds or prodrugs, tautomers, stereoisomers, solvates, isotope derivatives, or pharmaceutically acceptable salts thereof:

| Comp. No. | Structure | Comp. No. | Structure |
|---|---|---|---|
| 1 | 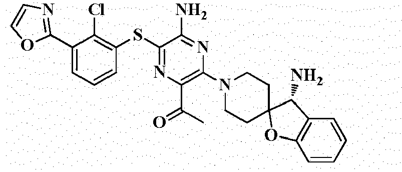 | 8 | 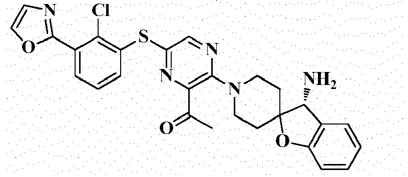 |
| 16 | 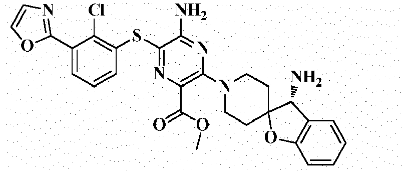 | 19 | 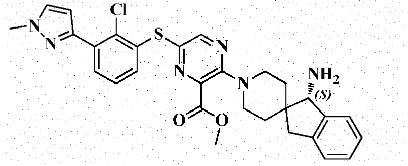 |
| 22 | 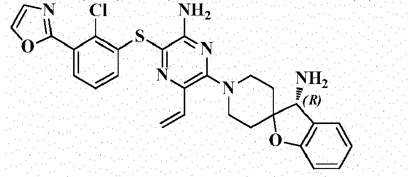 | 21 | 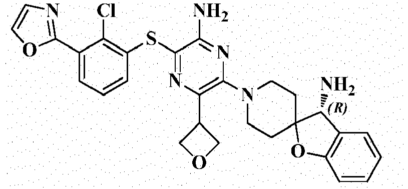 |
| 24 | 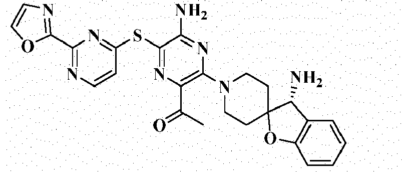 | 23 | 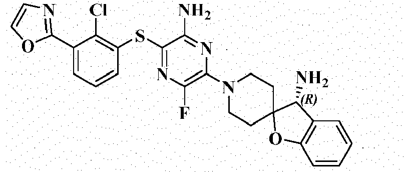 |
| 56 | 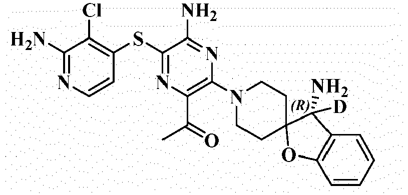 | 57 | 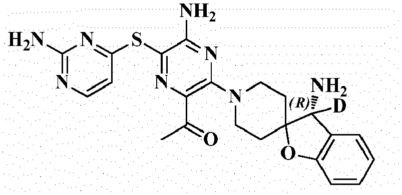 |
| 58 | 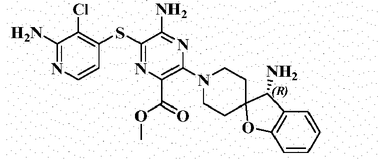 | 59 | 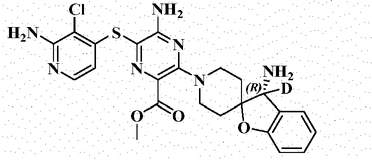 |
| 60 | 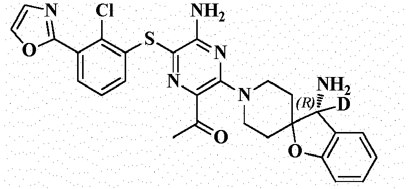 | 61 | 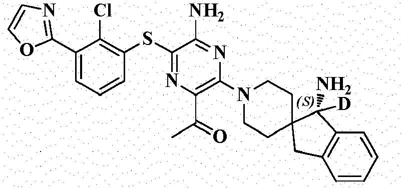 |
| 62 | 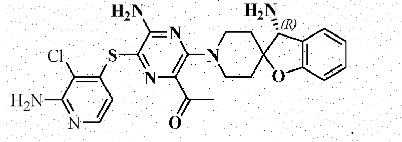 | | |

In some preferable embodiments of this disclosure, there are provided the following compounds or prodrugs, tautomers, stereoisomers, solvates, isotope derivatives, or pharmaceutically acceptable salts thereof:

| Comp. No. | Structure | Comp. No. | Structure |
|---|---|---|---|
| 1 | 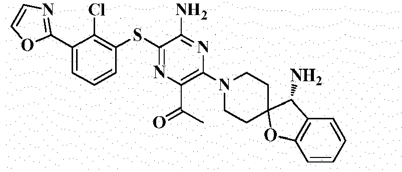 | 8 | 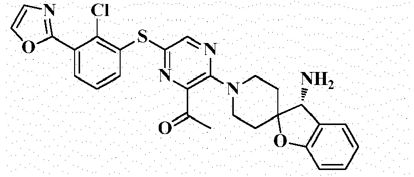 |
| 16 | 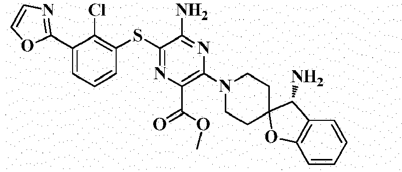 | 19 | 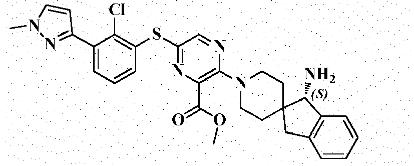 |
| 56 | 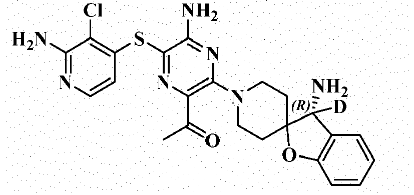 | 57 | 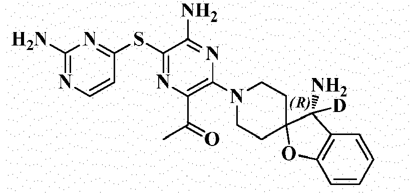 |
| 59 | 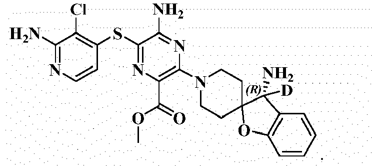 | | |

In some preferable embodiments of this disclosure, there are provided the following compounds or prodrugs, tautomers, stereoisomers, solvates, isotope derivatives, or pharmaceutically acceptable salts thereof:

| Comp. No. | Structure | Comp. No. | Structure |
|---|---|---|---|
| 1 | 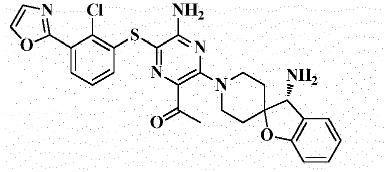 | 56 | 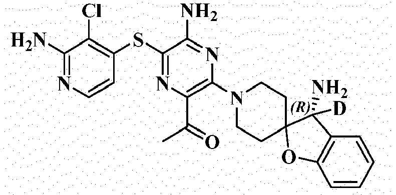 |

On the other hand, this disclosure also provides a principal process for preparing the above-mentioned compound of formula (I), which can be prepared, for example, using the methods shown in the following synthesis scheme I and synthesis scheme II.

### Synthesis scheme I

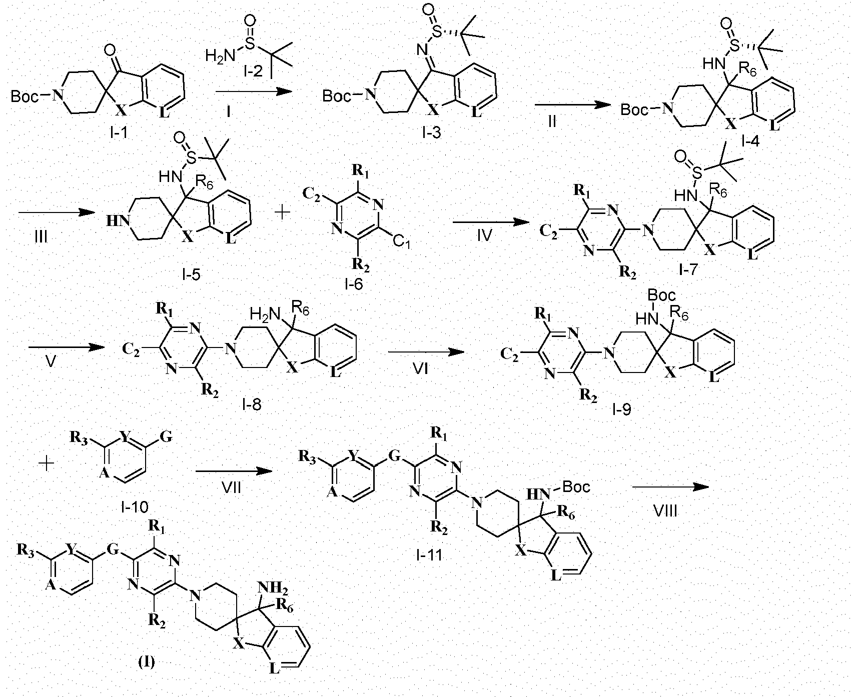

C₁ and C₂ may be Cl or Br
wherein A, Y, G, X, L, R₁, R₂, R₃, and R₆ are as defined in the compound of formula (I),
   I) Compounds 1-1 and I-2 undergo a chemical conversion to produce compound 1-3;
      for example, compounds I-1 and 1-2, Ti(OEt)₄, and the like are used as basic raw materials to produce compound I-3;
   II) Compound I-3 undergoes a chemical conversion to produce compound I-4;
      for example, compound I-3 as basic raw material is reacted in the presence of a reducer to produce compound I-4;
   III) Compound I-4 undergoes a chemical conversion to produce compound 1-5;
      for example, compound I-4 undergoes a conversion in an acidic condition to produce compound I-5;
   IV) Compound I-5 undergoes a chemical conversion to produce compound I-7;
      for example, compounds I-5 and I-6 and the like as basic raw materials are reacted in a basic condition to produce compound I-7;
   V) Compound I-7 undergoes a chemical conversion to produce compound I-8;
      for example, compound I-7 is reacted in an acidic condition to produce compound I-8;
   VI) Compound I-8 undergoes a chemical conversion to produce compound I-9;
      for example, compound I-8, (Boc)₂O, and the like are used as basic raw materials to produce compound I-9;
   VII) Compound I-9 undergoes a chemical conversion to produce compound I-11;
      for example, compounds I-9 and I-10 and the like as basic raw materials are reacted under the palladium catalysis in a basic condition to produce compound I-11;
   VIII) Compound I-11 undergoes a chemical conversion to produce the compound of general formula (I); for example, compound I-11 and the like as basic raw materials are reacted in an acidic condition to produce the compound of general formula (I).

### Synthesis scheme II

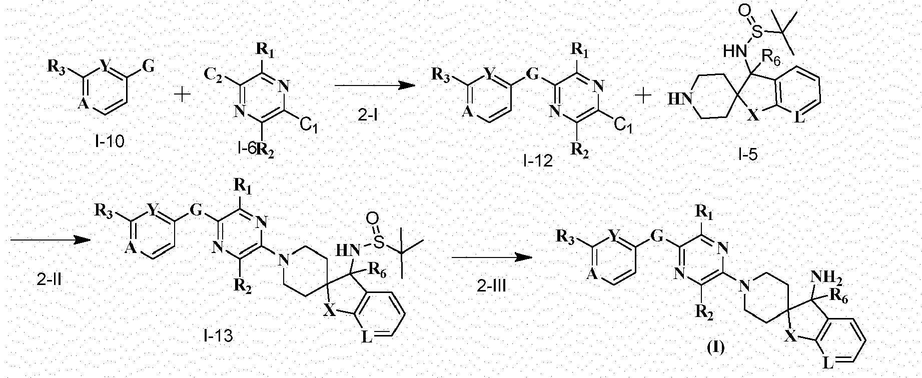

C₁ and C₂ may be Cl or Br
wherein A, Y, G, X, L, R₁, R₂, R₃, and R₆ are as defined in the compound of formula (I),
2-I) Compounds I-10 and I-6 undergo a chemical conversion to produce compound I-12;
   for example, compounds I-10 and I-6 and the like as basic raw materials are reacted under the palladium catalysis in a basic condition to produce compound 1-12;
2-II) Compounds 1-12 and I-5 undergo a chemical conversion to produce compound 1-13;
   for example, compounds I-12 and I-5 and the like as basic raw materials are reacted under the palladium catalysis in a basic condition to produce compound 1-13;
2-III) Compound 1-13 undergoes a chemical conversion to produce the compound of general formula (I);
   for example, compound I-13 undergoes a chemical conversion in an acidic condition to produce the compound of general formula (I).

On the other hand, this disclosure also provides a pharmaceutical composition, which contains (e.g., a therapeutically effective amount) of the compound described in this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof.

Further, the pharmaceutical composition described in this disclosure contains the compound described in this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof, and a pharmaceutically acceptable adjuvant.

The compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof may be administered in their pure forms or in the form of suitable pharmaceutical compositions through any acceptable administration routes of a medicament providing a similar use.

The pharmaceutical composition of this disclosure may be prepared by combining the compound of this disclosure with a suitable pharmaceutically acceptable adjuvant. The pharmaceutical composition of the present application may be formulated into solid, semi-solid, liquid, or gaseous formulations, such as tablets, pills, capsules, powders, granules, ointments, emulsions, suspensions, solutions, suppositories, injections, inhalants, gels, microspheres, aerosols, and the like. Generally, the above pharmaceutical compositions can be prepared by conventional preparation processes using conventional adjuvants in the field of preparations. On the other hand, this disclosure also provides use of the compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof or the pharmaceutical composition of this disclosure in manufacture of a medicament for preventing and/or treating diseases, disorders and conditions that are mediated by SHP2 activity.

Further, for the use provided in this disclosure, said disease, disorder and condition is tumor, cancer metastasis, cardiovascular disease, immune disorder, or visual disorder. Further, for the use provided in this disclosure, the tumor includes solid tumor and hematological malignancy.

Further, for the use provided in this disclosure, the solid tumor includes lung cancer, and the hematological malignancy includes leukemia, preferably acute myeloid leukemia. In some contexts in this field, the cancer may also be called a malignant tumor.

In another aspect, this disclosure provides a method for preventing and/or treating diseases, disorders and conditions that are mediated by SHP2 activity, which method comprises administering to an individual in need thereof the compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3), compound of formula (I-A), compound of formula (I-B), compound of formula (I-1-A), compound of formula (I-1-B), compound of formula (I-2-A), compound of formula (I-2-B), compound of formula (I-Ba)), or compound of formula (II)) or a prodrug, tautomer, stereoisomer, solvate, isotope derivative, or pharmaceutically acceptable salt thereof or the pharmaceutical composition of this disclosure; preferably, said disease, disorder and condition is tumour, cancer metastasis, cardiovascular disease, immune disorder, or visual disorder; more preferably, the tumor includes solid tumor and hematological malignancy; more preferably, the solid tumor includes lung cancer, the hematological malignancy includes leukemia, preferably acute myeloid leukemia. on the other hand, this disclosure provides the compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof or the pharmaceutical composition of this disclosure for preventing and/or treating diseases, disorders and conditions that are mediated by SHP2 activity; preferably, said disease, disorder and condition is tumour, cancer metastasis, cardiovascular disease, immune disorder, or visual disorder; more preferably, the tumor includes solid tumor and hematological malignancy; more preferably, the solid tumor includes lung cancer, the hematological malignancy includes leukemia, preferably acute myeloid leukemia.

Further, for the method or the use provided in this disclosure, the compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (I-2-B), or compound of formula (I-Ba)) or a prodrug, tautomer, stereoisomer, solvate, isotope derivative, or pharmaceutically acceptable salt thereof or the pharmaceutical composition of this disclosure is used in combination with one, two or more other compounds having tumor inhibitory activity.

In another aspect, this disclosure provides the compound of this disclosure (compound of formula (I), compound of formula (I-1), compound of formula (I-1-1), compound of formula (I-2), compound of formula (I-A1), compound of formula (I-1-A1), compound of formula (I-2-A1), compound of formula (I-A2), compound of formula (I-A3), compound of formula (I-2-A2), compound of formula (I-2-A3), compound of formula (I-B1), compound of formula (I-1-B1), compound of formula (I-2-B1), compound of formula (I-B2), compound of formula (I-B3), compound of formula (I-2-B2), compound of formula (I-2-B3)), compound of formula (II), compound of formula (I-A), compound of formula (I-B), compound of formula (I-1-A), compound of formula (I-1-B), compound of formula (I-2-A), compound of formula (1-2-B), or compound of formula (I-Ba)) or a prodrug, tautomer, stereoisomer, solvate, isotope derivative, or pharmaceutically acceptable salt thereof or the pharmaceutical composition of this disclosure for inhibiting SHP2 activity.

### Definition

The term "optional" or "optionally" refers to events or conditions described later that may, but need not, occur, and this description includes situations in which the events or conditions occur and situations in which the events or conditions do not occur.

Unless specified otherwise, the term "optionally substituted" means the group can be unsubstituted or substituted, wherein the substituent(s) is one or more groups independently selected from hydroxy, halogen, amino, nitro, mercapto, cyano, azido, oxo, carboxyl, -C(O)C₁₋₆alkyl, -C(O)O-C₁₋₆alkyl, -OC(O)-C₁₋₆alkyl, -NH(C₁₋₆alkyl), -N(C₁₋₆alkyl)(C₁₋₆alkyl), -C(O)NH-C₁₋₆alkyl, -NHC(O)-C₁₋₆alkyl, C₂₋₆alkenyl, C₂₋₆alkynyl, C₁₋₆alkyl, C₁₋₆alkoxyl, C₃₋₁₀cycloalkyl, C₃₋₁₀cycloalkylsulfonyl, 3-10 membered heterocycloalkyl, C₆₋₁₄aryl, and 5-12 membered heteroaryl; wherein the C₂₋₆alkenyl, C₂₋₆alkynyl, C₁₋₆alkyl, C₁₋₆alkoxyl, C₃₋₁₀cycloalkyl, C₃₋₁₀cycloalkylsulfonyl, 3-10 membered heterocycloalkyl, C₆₋₁₄aryl or 5-12 membered heteroaryl can be optionally substituted by one or more substituents selected from halogen, hydroxy, amino, cyano, C₁₋₆alkyl and C₁₋₆alkoxyl.

The term "oxo" means that two hydrogen atoms on the same substitution site are replaced by the same oxygen atom to form a double bond, i.e., =O.

Unless specified otherwise, the term "alkyl" refers to a monovalent saturated aliphatic hydrocarbon group, including a linear or branched group containing 1 to 20 carbon atoms, preferably 1, 2, 3, 4, 5, 6, 7, 8, 9 or 10 carbon atoms (i.e. C₁₋₁₀ alkyl), further preferably 1 to 8 carbon atoms (C₁₋₈ alkyl), more preferably 1 to 6 carbon atoms (i.e. C₁₋₆ alkyl). For example, "C₁₋₆ alkyl" means that the group is an alkyl group, and the number of carbon atoms in the carbon chain is between 1 and 6 (specifically 1, 2, 3, 4, 5, or 6). Examples include but are not limited to methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, and the like.

Unless specified otherwise, the term "alkenyl" refers to a linear or branched unsaturated aliphatic hydrocarbon group consisting of carbon atoms and hydrogen atoms and having at least one double bond. The alkenyl can contain 2-20 carbon atoms, preferably 2, 3, 4, 5, 6, 7, 8, 9 or 10 carbon atoms (i.e., C₂₋₁₀alkenyl), further preferably 2-8 carbon atoms (C₂₋₈alkenyl), more preferably 2-6 carbon atoms (i.e., C₂₋₆alkenyl), 2-5 carbon atoms (i.e., C₂₋₅alkenyl), 2-4 carbon atoms (i.e., C₂₋₄alkenyl), 2-3 carbon atoms (i.e., C₂₋₃alkenyl), 2 carbon atoms (i.e., C2alkenyl). For example, "C₂₋₆alkenyl" means that the group is an alkenyl group, and the number of carbon atoms in the carbon chain is between 2 and 6 (specifically 2, 3, 4, 5, or 6). Non-limiting examples of alkenyl groups include but are not limited to, ethenyl, 1-propenyl, 2-propenyl, 1-butenyl, isobutenyl, 1,3-butadienyl, and the like.

Unless specified otherwise, the term "alkynyl" refers to a linear or branched unsaturated aliphatic hydrocarbon group consisting of carbon atoms and hydrogen atoms and having at least one triple bond. The alkynyl can contain 2-20 carbon atoms, preferably 2, 3, 4, 5, 6, 7, 8, 9 or 10 carbon atoms (i.e., C₂₋₁₀alkynyl), further preferably 2-8 carbon atoms (C₂₋₈alkynyl), more preferably 2-6 carbon atoms (i.e., C₂₋₆alkynyl), 2-5 carbon atoms (i.e., C₂₋₅alkynyl), 2-4 carbon atoms (i.e., C₂₋₄alkynyl), 2-3 carbon atoms (i.e., C₂₋₃alkynyl), 2 carbon atoms (i.e., C2alkynyl). For example "C₂₋₆alkynyl" means that the group is an alkynyl group, and the number of carbon atoms in the carbon chain is between 2 and 6 (specifically 2, 3, 4, 5, or 6). Non-limiting examples of alkynyl groups include but are not limited to, ethynyl, 1-propynyl, 2-propynyl, 1-butynyl, and the like.

Unless specified otherwise, the term "cycloalkyl", "carbocyclyl" or "carbocycle" refers to a monocyclic saturated aliphatic hydrocarbon group with a specific number of carbon atoms, including 3, 4, 5, 6, 7, 8, 9, 10, 11, 12, 13 or 14 carbon atoms (i.e., C₃₋₁₄cycloalkyl), preferably 3-12 carbon atoms (i.e., C₃₋₁₂cycloalkyl), more preferably 3-10 carbon atoms (C₃₋₁₀cycloalkyl), further preferably 3-7 carbon atoms (C₃₋₇cycloalkyl), 4-6 carbon atoms (C₄₋₆cycloalkyl), 5-6 carbon atoms (C₅₋₆cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, and the like. In some embodiments of this disclosure, "cycloalkyl" refers to a monocyclic saturated aliphatic hydrocarbon group with a specific number of carbon atoms and consisting of carbon atoms and hydrogen atoms. In some embodiments of this disclosure, cycloalkyl also preferably contains 3-4 carbon atoms (C₃₋₄cycloalkyl), 3-5 carbon atoms (C₃₋₅cycloalkyl), or 4-5 carbon atoms (C₄₋scycloalkyl).

Unless specified otherwise, the term "alkoxyl" refers to -O-alkyl, said alkyl is defined as above, that is to say, contains 1-20 carbon atoms, preferably 1, 2, 3, 4, 5, 6, 7, 8, 9 or 10 carbon atoms, more preferably 1-8 carbon atoms, still more preferably 1-6 carbon atoms (specifically 1, 2, 3, 4, 5 or 6). Representative examples include but are not limited to methoxy, ethyloxy, propoxy, isopropoxy, butoxy, 1-methylpropoxy, 2-methylpropoxy, tert-butyloxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy and the like.

Unless specified otherwise, the term "carboxyl" refers to the group -COOH.

The term "halogen" or "halo" refers to F, Cl, Br, and I.

The term "haloalkyl" refers to such an alkyl in which one, two, or more hydrogen atoms or all hydrogen atoms in an alkyl group as defined above are substituted by a halogen atom(s). Representative examples of haloalkyl include CCl₃, CF₃, CHCl₂, CH₂Cl, CH₂Br, CH₂I, CH₂CF₃, CF₂CF₃ and the like.

Unless specified otherwise, the term "heterocyclyl" or "heterocycle " refers to a saturated or partially unsaturated monocyclic, bicyclic, or polycyclic non-aromatic substituent having at least one ring carbon atom and 1-4 ring heteroatoms, and it contains 3-20 ring atoms, wherein 1, 2, 3, or more ring atoms are selected from N, O, and S and the remaining ring atoms are C. Preferably it contains 3, 4, 5, 6, 7, 8, 9, 10, 11, 12, 13, or 14 ring atoms (i.e., 3-14 membered heterocyclyl), preferably 3-12 ring atoms (3-12 membered heterocyclyl), further preferably 3-10 ring atoms (3-10 membered heterocyclyl), or 3-8 ring atoms (3-8 membered heterocyclyl), or 3-6 ring atoms (3-6 membered heterocyclyl), or 4-6 ring atoms (4-6 membered heterocyclyl), or 5-6 ring atoms (5-6 membered heterocyclyl). The number of heteroatoms is preferably 1-4, more preferably 1-3 (i.e., 1, 2 or 3). Examples of monocyclic heterocyclyl include oxiranyl, pyrrolidinyl, N-methylpyrrolidinyl, pyrazolidinyl, imidazolidinyl, tetrahydrofuranyl, dihydropyrrolyl, piperidinyl, piperazinyl, pyranyl, morpholino, thiomorpholino, tetrahydrothienyl and the like. Polycyclic heterocyclyl includes spiro, fused, and bridged heterocyclyl. "Heterocyclyl" may be monocyclic ("monocyclic heterocyclyl") or fused ("fused heterocyclyl" or "hetero fused cyclyl"), bridged ("hetero bridged cyclyl" or "bridged heterocyclyl") or spiro ("hetero spiro cyclyl" or "spiro heterocyclyl") ring system, such as bicycle system ("bicyclic heterocyclyl"), and may be saturated or may be partially unsaturated. The bicycle system of the heterocyclyl may include one or more heteroatoms in one or two rings. "Heterocyclyl" also includes such a ring system in which the ring of the heterocyclyl as defined above is fused with one or more carbocyclyl groups, wherein the attachment point is on the ring of the carbocyclyl or heterocyclyl, or "heterocyclyl" also includes such a ring system in which the ring of the heterocyclyl as defined above is fused with one or more aryl or heteroaryl groups, or such a ring system the ring of the cycloalkyl as defined above is fused with one or more heteroaryl groups, wherein the attachment point is on the ring of the heterocyclyl or the ring of the cycloalkyl, and in such cases, the member number in the ring system of the heterocyclyl is the number of the ring atoms of the resulting fused ring system. In some embodiments, each example of heterocyclyl can be independently optionally substituted, for example, unsubstituted (an "unsubstituted heterocyclyl") or substituted by one or more substituents (a "substituted heterocyclyl"). An exemplary 3-membered heterocyclyl group containing 1 heteroatom includes but is not limited to, aziridinyl, oxiranyl, and thiiranyl. An exemplary 4-membered heterocyclyl group containing 1 heteroatom includes but is not limited to, azetidinyl, oxetanyl, and thietanyl. An exemplary 5-membered heterocyclyl group containing 1 heteroatom includes but is not limited to, tetrahydrofuranyl, dihydrofuranyl, tetrahydrothiaphenyl, dihydrothiaphenyl, pyrrolidinyl, dihydropyrrolyl, and pyrrolidine-2,5-dionyl. An exemplary 5-membered heterocyclyl group containing 2 heteroatoms includes but is not limited to, dioxolanyl, oxathiolanyl, dithiolanyl, and oxazolidin-2-onyl. An exemplary 5-membered heterocyclyl group containing 3 heteroatoms includes but is not limited to, triazolinyl, oxadiazolinyl, and thiadiazolinyl. An exemplary 6-membered heterocyclyl group containing 1 heteroatom includes but is not limited to, piperidinyl, tetrahydropyranyl, dihydropyridyl, and thianyl. An exemplary 6-membered heterocyclyl group containing 2 heteroatoms includes but is not limited to, piperazinyl, morpholinyl, dithianyl, and dioxanyl. An exemplary 6-membered heterocyclyl group containing 3 heteroatoms includes but is not limited to, triazinanyl, oxadiazinanyl, thiadiazinanyl, oxathiazinanyl, and dioxazinanyl. An exemplary 7-membered heterocyclyl group containing 1 heteroatom includes but is not limited to, azepanyl, oxepanyl, and thiepanyl. An exemplary 8-membered heterocyclyl group containing 1 heteroatom includes but is not limited to, azocaneyl, oxocaneyl, and thiocanyl. An exemplary 5-membered heterocyclyl group fused with one C₆aryl ring (herein also called 5,6-bicyclic heterocycle) includes but is not limited to, indolinyl, isoindolinyl, dihydrobenzofuranyl, dihydrobenzothienyl, benzoxazolinonyl and the like. An exemplary 6-membered heterocyclyl group fused with one C₆aryl ring (also referred to herein as 6,6-bicyclic heterocycle) includes but is not limited to, tetrahydroquinolinyl, tetrahydroisoquinolinyl, and like.

Unless specified otherwise, the term "heterocycloalkyl" refers to a monocyclic, saturated "heterocyclyl" or "heterocycle" as defined above, wherein the ring atoms are defined as above. That is to say, it contains 3-20 ring atoms ("3-20 membered heterocycloalkyl"), and the number of heteroatoms is 1-4 (1, 2, 3, or 4), preferably 1-3 (1, 2 or 3), and the heteroatom is each independently selected from N, O and S. Preferably it contains 3, 4, 5, 6, 7, 8, 9, 10, 11 or 12 ring atoms ("3-12 membered heterocycloalkyl"), further preferably 3-10 ring atoms ("3-10 membered heterocycloalkyl"), more further preferably 3-8 ring atoms ("3-8 membered heterocycloalkyl"), more further preferably 4-7 ring atoms ("4-7 membered heterocycloalkyl"), more further preferably 5-10 ring atoms ("5-10 membered heterocycloalkyl"), or more further preferably 5-6 ring atoms ("5-6 membered heterocycloalkyl"). In some embodiments, each example of heterocycloalkyl can be independently optionally substituted, for example, unsubstituted (an "unsubstituted heterocycloalkyl") or substituted by one or more substituents (a "substituted heterocycloalkyl"). A part of exemplary "heterocycloalkyl" groups have been given in the above description for "heterocyclyl" or "heterocycle", and the "heterocycloalkyl" group also includes but is not limited to, aziridinyl, oxiranyl, thiiranyl, azetidinyl, oxetanyl, thietanyl, tetrahydrofuranyl, tetrahydropyranyl, piperidinyl, piperazinyl, morpholinyl, thiamorpholinyl, oxathianyl, oxazolidinyl, dioxanyl, dithianyl, thiazolidinyl, pyrrolidinyl, pyrazolidinyl, imidazolidinyl, and the like.

Unless specified otherwise, the term "aryl" or "aromatic ring group" refers to monocyclic, bicyclic, and tricyclic aromatic carbocycle system containing 6-16 carbon atoms, or 6-14 carbon atoms, or 6-12 carbon atoms, or 6-10 carbon atoms, preferably 6-10 carbon atoms. The term "aryl" may be used interchangeably with the term "aromatic ring". Examples of aryl groups may include but are not limited to, phenyl, naphthyl, anthryl, phenanthrenyl, pyrenyl, and the like. Polycyclic aryl groups include fused aryl groups and bridged aryl groups, and "fused" and "bridged" are defined in a similar way as those in the definition of polycyclic heterocyclyl.

Unless specified otherwise, the term "heteroaryl" or "heteroaromatic ring group" refers to an aromatic monocyclic or polycycle system containing a 5, 6, 7, 8, 9, 10, 11, 12, 13 or 14-membered structure, or preferably a 5-10 membered structure, or a 5-8 membered structure, or more preferably a 5-6 membered structure, wherein 1, 2, 3 or more ring atom(s) is/are heteroatom(s) and the remaining atom(s) is/are carbon, the heteroatom is independently selected from O, N, and S, and the heteroatom number is preferably 1, 2 or 3. Examples of heteroaryl groups includ but are not limited to, furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidyl, [1,2,4]triazolo[1,5-a]pyridinyl, and the like. Polycyclic heteroaryl groups include fused heteroaryl groups and bridged heteroaryl groups, and "fused" and "bridged" are defined in a similar way as those in the definition of polycyclic heterocyclyl.

When a substituent may be attached to more than one atom on a ring, this substituent may be bonded to any atom (including heteroatom, e.g. NH) on the ring. For example, 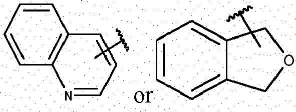 represents that the attachment site can be located at any position on the benzene or pyridine ring, or any position on the benzene or tetrahydrofuran ring.

Unless specified otherwise, the term "pharmaceutically acceptable salt" refers to those salts which are, within the scope of sound medical judgment, suitable for use in contact with the tissues of mammals, particularly humans without undue toxicity, irritation, allergic response, and the like, and are commensurate with a reasonable benefit/risk ratio. For example, pharmaceutically acceptable salts of amines, carboxylic acids, and other types of compounds are well-known in the art. The salts can be prepared in situ during the final isolation and purification of the compounds according to the present invention, or separately by reacting the free base or acid with a suitable reagent. The pharmaceutically acceptable salts of this disclosure include acid addition salts or base addition salts of the compound described in this disclosure, which are obtained by reacting the compounds of this disclosure with inorganic or organic acids or inorganic or organic bases well-known to those skilled in the art.

Unless specified otherwise, the term "isotope derivative" means that compounds of this disclosure can exist in isotope labeled or enriched form containing one or more atoms having an atomic mass or mass number different from the atomic mass or mass number most abundantly found in nature. Isotopes can be radioactive or non-radioactive isotopes. Isotopes commonly used as isotopic labels are: hydrogen isotopes: ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O and ¹⁸O; and sulfur isotope: ³⁵S. These isotope-labeled compounds can be used to study the distribution of pharmaceutical molecules in tissues. In particular, ²H and ¹³C are more widely used due to their ease of labeling and ease of detection. The substitution with certain heavy isotopes, such as heavy hydrogen (²H), can enhance the stability of metabolism, and prolong the half-life, so as to achieve the purpose of reducing dosage and providing therapeutic advantages. Isotope labeled compounds are generally synthesized starting from labeled starting materials in the same way as non-isotope labeled compounds using known synthetic techniques.

Unless specified otherwise, the term "solvate" means a physical association of a compound of this disclosure with one or more solvent molecules, whether organic or inorganic. This physical association includes hydrogen bonding. In certain instances, the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include but are not limited to, hydrates, ethanolates, methanolates, and isopropanolates. Methods of solvation are generally known in the art.

Unless specified otherwise, the term "stereoisomer" refers to compounds that have identical chemical constitutions, but differ with regard to the arrangement of the atoms or groups in space. Stereoisomers include enantiomers, diastereomers, conformers (rotamers), geometric (cis/trans) isomers, atropisomers, and the like. Any resulting mixtures of isomers can be separated on the basis of the physicochemical differences of the constituents, into pure or substantially pure geometric isomers, enantiomers, and diastereomers, for example, by chromatography and/or fractional crystallization.

Unless specified otherwise, the term "tautomer" refers to structural isomers of different energies which are interconvertible via a low-energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons.

Unless specified otherwise, structures depicted herein are also meant to include all isomeric (e.g., enantiomeric, diastereomeric, and geometric (or conformational)) forms of the structure, for example, the R and S configurations for each asymmetric center, (Z) and (E) double bond isomers, and (Z) and (E) conformational isomers. Therefore, single stereochemical isomers as well as enantiomeric, diastereomeric, or geometric (or conformational) mixtures of the present compounds are within the scope of this disclosure. Unless specified otherwise, the term "prodrug" refers to a drug that is converted into its parent drug in vivo. The prodrug is usually useful, which may improve some certain and undesirable physical or biological properties. The physical properties usually refer to related solubility (excessive or insufficient solubility in lipid or water) or stability, while problematic biological properties include overquick metabolism or poor bioavailability, which may itself be related to physical and chemical properties. For example, they can be bioavailable by oral administration, while the parent drugs cannot. Compared with the parent drug, the solubility of the prodrug in a pharmaceutical composition is also improved. An exemplary example of prodrug may be, but is not limited to, any compound of the present disclosure, which is administered as an ester ("prodrug") to facilitate transport through cell membranes, and subsequently hydrolyzed to a carboxylic acid (i.e. active entity), wherein the water solubility thereof is detrimental to the transport but beneficial once it gets into the cell. Another exemplary example of the prodrug may be a short peptide (polyamino acid) attached to a carboxyl group, wherein the peptide is metabolized to release the active moiety.

Unless specified otherwise, the term "treatment," covers any treatment of a disease, disorder, and condition in a patent, and includes: (a) inhibiting the symptom of the disease, disorder, and condition, i.e., arresting its development; or (b) relieving the symptom of the disease, disorder and condition, i.e., causing the subsidence of the disease or symptom; or (c) ameliorating or eliminating the disease, disorder and condition, or one or more symptoms related to the disease.

Unless specified otherwise, the term "therapeutically effective amount" refers to the dosage of the compound in this disclosure that (i) treats a specific disease, disorder, or condition, (ii) alleviates, ameliorates, or eliminates one or more symptoms of a specific disease, disorder or condition, or (iii) delays the onset of one or more symptoms of a specific disease, disorder or condition described in this disclosure. The amount of compound in this disclosure that constitutes the "therapeutically effective amount" varies depending on the compound, the disease status and the severity thereof, the manner of administration, and the age of the mammal to be treated, but can be routinely determined by those skilled in the art based on their own knowledge and the content of this disclosure.

Unless specified otherwise, the term "pharmaceutically acceptable adjuvant" refers to those adjuvants which do not cause significant stimulation to an organism (e.g., human), and will not impair the bioactivity and properties of an active compound. Suitable adjuvants are well known to those skilled in the art, for example, carbohydrate, wax, water-soluble and/or water-swellable polymers, hydrophilic or hydrophobic material, gelatin, oil, solvent, or water. The "pharmaceutical acceptable adjuvant" also refers to an inert substance that is administered together with an active ingredient and is beneficial to the administration thereof, including, but not limited to, any adjuvant such as glidant, sweetening agent, diluent, preservative, dye/colorant, flavoring agent, surfactant, wetting agent, dispersant, disintegrant, suspending agent, stabilizer, isotonic agent, solvent and emulsifier, which have been approved by the United States Food and Drug Administration as being acceptable for use in humans or animals (such as livestock).

Beneficial effects of the present invention:
This invention designs a class of structurally novel compounds that provide a new direction for the treatment of tumors, cancer, cancer metastasis, cardiovascular diseases, immune disorders, or visual disorders. The compounds of this invention have one or more of the following advantages: (1) they have a relatively strong SHP2 kinase inhibitory effect; (2) they have relatively high exposure and good absorption; (3) they have no significant inhibitory effect on hERG channels and may have relatively low cardiac toxicity; (4) they have good tolerance and high drug safety; and (5) they have high tumor inhibition rate in vivo. In addition, this invention discloses a specific synthesis process, which is simple in process, convenient in operation, and conducive to large-scale industrial production and application.

### Detailed description

The following will further elaborate on various aspects of this invention in conjunction with specific examples. It should be understood that these examples are only used to illustrate the present invention and not to limit the scope of various aspects described in this disclosure. If the specific experimental method is not specified in the examples, it is usually carried out under conventional conditions or according to the conditions recommended by the manufacturer. Unless otherwise defined, all professional and scientific terms used in this disclosure have the same meaning as those familiar to those skilled in the art. In addition, any methods and materials similar or equivalent to those described in this disclosure may be used in the methods of the present invention. The preferred embodiments and materials shown in this disclosure are illustrative only.

The abbreviations used in the preparation examples, the examples, and other parts of this disclosure are:
- DCM: dichloromethane
- TEA: triethylamine
- EA: ethyl acetate
- DMSO: dimethyl sulfoxide
- DMF: N,N-dimethyl formamide
- TFA: trifluoroacetic acid
- DIPEA: N,N-diisopropylethylamine
- Pd(dppf)Cl₂: 1,1'-bis(diphenylphosphino)ferrocene dichloropalladium
- Pd₂(dba)₃: tris(dibenzylideneacetone)dipalladium
- Pd(PPh₃)₄: tetrakis(triphenylphosphine)palladium (0)
- XantPhos: 4,5-bis(diphenylphosphino)-9,9-dimethylxanthene
- (Boc)₂O: di-tert-butyl dicarbonate
- NBS: N-bromosuccinimide
- Dtbpy: 4,4'-bis(tert-butyl)bipyridine
- DMA: N,N-dimethylacetamide
- TBAI: tetrabutylammonium iodide
- EDCI: 1-ethyl-(3-dimethylaminopropyl)carbodiimide hydrochloride.

### Intermediate Preparation Example 1: 1-(5-amino-3-chloropyrazine-2-yl)ethanone (Intermediate 1)

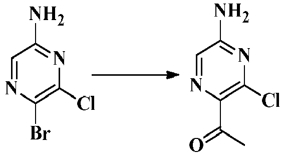

To a solution of 2-amino-5-bromo-6-chloropyrazine (20.0g, 96.67mmol, 1.0eqv) in 1,4-dioxane (240ml) were added tributyl(1-ethoxyvinyl)stannane (52.36g, 144.98mmol, 1.5eqv), CuI (1.84g, 9.667mmol, 0.1eqv) and Pd(Ph₃P)₂Cl₂ (6.78g, 9.667mmol, 0.1eqv). Under the protection of nitrogen gas, the reaction mixture was stirred at 100°C overnight. After cooling down to room temperature, silica gel was added to the reaction solution. The resulting system was concentrated under reduced pressure to dryness, and purified by column chromatography (n-hexane:EA=10:1-5:1) to produce 1-(5-amino-3-chloropyrazine-2-yl)ethanone (5.1g, yield: 31%). (ESI, *m*/*z):* 171.97[M+H]⁺.

### Intermediate Preparation Example 2: 3-chloro-5-((2,4-dimethoxybenzyl)amino)pyrazine-2-carboxylic acid methyl ester (Intermediate 2)

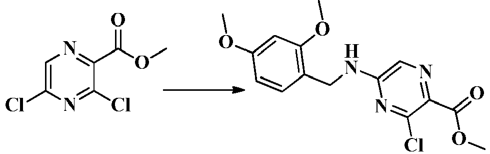

To a solution of 3,5-dichloropyrazine-2-carboxylic acid methyl ester (3.8 g, 18.36 mmol, 1 eqv) and 2,4-dimethoxybenzylamine (3.07 g, 18.36 mmol, 1 eqv) in DMSO (40 mL) was added CsF (8.36 g, 55.07 mmol, 3 eqv) under the protection of nitrogen gas. Then the resulting mixture was warmed up to 75°C and reacted for 3 hours. After cooling down to room temperature, the reaction solution was poured into water (200ml) and EA (100ml), and separated into two phases. The aqueous phase was extracted with EA (2×50ml). The organic phases were combined. The combined organic phase was washed with brine (100ml), and concentrated under reduced pressure. The residue was purified by column chromatography (n-hexane:EA=10:1-1:1) to produce 3-chloro-5-((2,4-dimethoxybenzyl)amino)pyrazine-2-carboxylic acid methyl ester (4 g, yield: 54.06%). (ESI, m/z): 338.1 [M+H]⁺; ¹H NMR (400 MHz, CDCl₃): δ ppm 7.82 (s, 1H) 7.23 (d, *J*=8.4 Hz, 1H) 6.41 -6.51 (m, 2H) 5.66 (br s, 1H) 4.55 (d, *J*=5.6 Hz, 2H) 3.94 (s, 3H) 3.85 (s, 3H) 3.81 (s, 3H).

### Intermediate Preparation Example 3: 2-chloro-3-(oxazole-2-yl)benzenethiol (Intermediate 3)

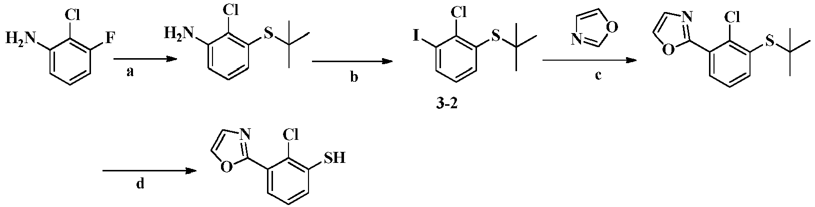

**Step a:** A solution of 2-chloro-3-fluoroaniline (5.00 g, 33.663 mmol, 1eqv), tert-butylthiol (3.64 g, 40.396 mmol, 1.2eqv), and Cs₂CO₃ (32.90 g, 100.989 mmol, 3eqv) in DMF (50ml) was reacted at 130°C for 24 hours. After cooling down to room temperature, the reaction solution was poured into water (300ml), and extracted with EA (2×300ml). The organic phases were combined. The combined organic phase was washed with brine (2×500ml), and concentrated under reduced pressure. The residue was purified by column chromatography (n-hexane:EA=100:1-20:1) to produce 3-(tert-butylsulfanyl)-2-chloroaniline (7.0 g, yield: 94.46%). (ESI, m/z): 216 [M+H]⁺.

**Step b:** A solution of 3-(tert-butylsulfanyl)-2-chloroaniline (7.0 g, 32.55mmol, 1eqv) in concentrated hydrochloric acid (15ml) was cooled down to -10 to -5°C. Then, a solution of sodium nitrite (3.369g, 48.83mmol, 1.5eqv) in water (105ml) was added dropwise. After the completion of the dropwise addition, the system was maintained at the same temperature and stirred for 30 minutes. At the same temperature, a solution of potassium iodide (6.484g, 39.06mmol, 1.2eqv) in water (65ml) was added dropwise. After the completion of the dropwise addition, the system was maintained at the same temperature and stirred for 20 minutes. To the reaction solution were added ethyl acetate (100ml) and saturated sodium thiosulfate solution (100ml). The resulting mixture was stirred for 10min, and separated into two phases. The aqueous phase was extracted with EA (2×50ml). The organic phases were combined. The combined organic phase was washed with brine (100 mL), and concentrated under reduced pressure. The residue was purified by column chromatography (n-hexane) to produce tert-butyl(2-chloro-3-iodophenyl)sulfane (6.52g, yield: 61.45%). ¹H-NMR (400 MHz, DMSO-d6) δ7.99 (dd, J= 7.9, 1.5 Hz, 1H), 7.68 (dd, J= 7.7, 1.5 Hz, 1H), 7.10 (t, J= 7.8 Hz, 1H), 1.29 (s, 9H).

**Step c:** A solution of tert-butyl(2-chloro-3-iodophenyl)sulfane (6.5g, 19.94mmol, 1eqv), oxazole (2.75g, 39.88mmol, 2eqv), lithium tert-butanolate (1.92g, 23.93mmol, 1.2eqv), and CuI (380mg, 1.994mmol, 0.1eqv) in DMF (50ml) was reacted at 140°C for 2 hours under the protection of nitrogen gas. The TLC detection indicated the completion of the reaction. After cooling down to room temperature, the reaction solution was poured into water (300ml), and extracted with EA (2×150ml). The organic phases were combined. The combined organic phase was washed with brine (2×100ml), and concentrated under reduced pressure. The residue was purified by column chromatography (n-hexane:EA=25:1-10:1) to produce 2-(3-(tert-butylsulfanyl)-2-chlorophenyl)oxazole (3.12 g, yield: 58.58%). (ESI, m/z): 267.93 [M+H]⁺. ¹H-NMR (400 MHz, CDCl₃) δ7.91 (dd, J= 7.8, 1.7Hz, 1H), 7.80 (d, J= 0.7 Hz, 1H), 7.78 (dd, J= 7.7, 1.7Hz, 1H), 7.36-7.31 (m, 2H), 1.37 (s, 9H).

**Step d:** To a solution of 2-(3-(tert-butylsulfanyl)-2-chlorophenyl)oxazole (3.12 g, 11.68mmol, 1eqv) in toluene (60ml) was added aluminum trichloride (6.23g, 46.72mmol, 4eqv) at 0°C. The reaction mixture was stirred at room temperature overnight. After cooling down to 0°C, water (60ml) was added dropwise. The resulting mixture was stirred for 30min, and separated into two phases. The aqueous phase was extracted with toluene (30ml). The organic phases were combined. The combined organic phase was concentrated under reduced pressure. The residue was purified by column chromatography (n-hexane:EA=20:1-5:1) to produce 2-chloro-3-(oxazole-2-yl)benzenethiol (1.726 g, yield: 70%). (ESI, m/z): 211.89 [M+H]⁺.

### Intermediate Preparation Examples 4-5:

The steps of Intermediate Preparation Example 3 and the following corresponding starting materials were used to synthesize Intermediates 4-5:

| Intermediate Preparation Example No. | Main Starting Materials | Intermediate Structure and No. | Characterization Data |
|---|---|---|---|
| 4 | 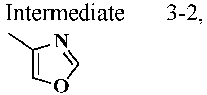 | 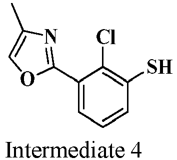 | (ESI, m/z): 225.91[M+H]⁺ |
| 5 | 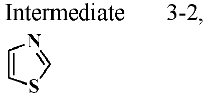 | 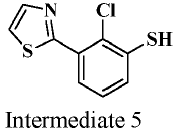 | (ESI, m/z): 227.92[M+H]⁺ |

### Intermediate Preparation Example 6: 2-chloro-3-(pyrazine-2-yl)benzenethiol (Intermediate 6)

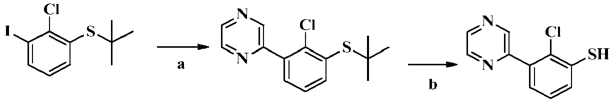

**Step a:** A solution oftert-butyl(2-chloro-3-iodophenyl)sulfane (3.26g, 10mmol, 1eqv), pyrazine-2-boronic acid (1.49g, 12mmol, 1.2 eqv), K₂CO₃ (4.14g, 30mmol, 3 eqv) and Pd(dppf)Cl₂ (731.7mg, 1mmol, 0.1 eqv) in 1,4-dioxane (80ml) and water (20ml) was reacted at 100°C overnight under the protection of nitrogen gas. The reaction solution was concentrated under reduced pressure. The residue was purified by column chromatography (n-hexane:EA=50:1-25:1) to produce 2-(3-(tert-butylsulfanyl)-2-chlorophenyl)pyrazine (1.21g, yield: 43.5%). (ESI, m/z): 279.00 [M+H]⁺.

**Step b:** To a solution of 2-(3-(tert-butylsulfanyl)-2-chlorophenyl)pyrazine (1.21g, 4.35mmol, 1eqv) in toluene (60ml) was added aluminum trichloride in portions (2.32g, 17.4mmol, 4eqv) at 0°C. The reaction mixture was stirred at room temperature overnight. After cooling down to 0°C, water (60ml) was added dropwise. The resulting mixture was stirred for 30min, and separated into two phases. The aqueous phase was extracted with toluene (30ml). The organic phases were combined. The combined organic phase was concentrated under reduced pressure. The residue was purified by column chromatography (n-hexane:EA=20:1-5:1) to produce 2-chloro-3-(pyrazine-2-yl)benzenethiol (726mg, yield: 75.2%). (ESI, m/z): 222.89 [M+H]⁺.

### Intermediate Preparation Examples 7-9

The steps of Intermediate Preparation Example 6 and the following corresponding starting materials were used to synthesize Intermediates 7-9:

| Intermediate Preparation Example No. | Main Starting Materials | Intermediate Structure and No. | Characterization Data |
|---|---|---|---|
| 7 | 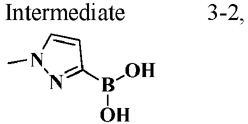 | 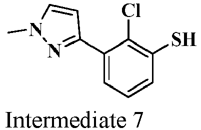 | (ESI, m/z): 224.95[M+H]⁺ |
| 8 | 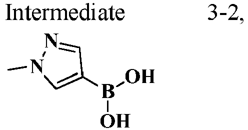 | 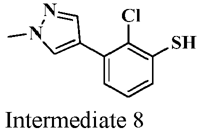 | (ESI, m/z): 224.94[M+H]⁺ |
| 9 | 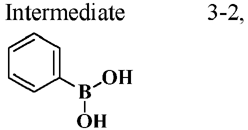 | 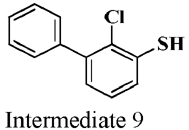 | (ESI, m/z): 221.00[M+H]⁺ |

### Intermediate Preparation Example 10: 3-chloro-2-(pyrazine-2-yl)pyridine-4-thiol (Intermediate 10)

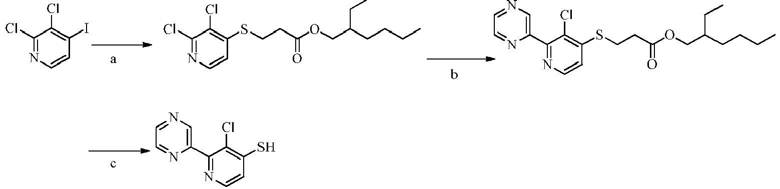

**Step a:** A solution of 2,3-dichloro-4-iodopyridine (2.00 g, 7.33 mmol, 1.00 eqv), 3-mercaptopropanoic acid 2-ethylhexyl ester (2.07 g, 9.51mmol, 1.3 eqv), DIPEA (2.862 g, 21.99 mmol, 3.00eqv), Pd₂(dba)₃ (1.342 g, 1.466 mmol, 0.2 eqv) and XantPhos (1.696 g, 2.932mmol, 0.4eqv) in 1,4-dioxane (50ml) was stirred at 96°C for 8 hours under the protection of nitrogen gas. After cooling down to room temperature, the reaction solution was filtered by suction with diatomite. The filtrate was concentrated under reduced pressure. The residue was purified by column chromatography (n-hexane:EA=20:1) to produce 3-(2-(2,3-dichloropyridine-4-yl)sulfanyl)propanoic acid 2-ethylhexyl ester (2.076g, yield: 78%). (ESI, *m*/*z*): 364.06[M+H] ⁺.

**Step b:** A solution of 3-(2-(2,3-dichloropyridine-4-yl)sulfanyl)propanoic acid 2-ethylhexyl ester (666mg, 1.834mmol, 1.0eqv), pyrazine-2-boronic acid (250mg, 2.02mmol, 1.1eqv), Pd(dppf)Cl₂ (134mg, 0.183mmol, 0.1eqv) and potassium carbonate (760mg, 5.503mmol, 3eqv) in 1,4-dioxane (60 mL) and water (10ml) was reacted at 96 °C overnight under the protection of nitrogen gas. The resulting reaction mixture was cooled down, and concentrated under reduced pressure. To the residue were added water (200ml) and EA (100ml), and the resulting mixture was separated into two phases. The aqueous phase was extracted with EA (2×50ml). The organic phases were combined. The combined organic phase was washed with brine (100ml), and concentrated under reduced pressure. The residue was purified by column chromatography (n-hexane:EA=30:1-10:1) to produce 3-((3-chloro-2-(pyrazine-2-yl)pyridine-4-yl)sulfanyl)propanoic acid 2-ethylhexyl ester (637mg, yield: 85.3%). (ESI, *m*/*z*): 408.15[M+H]⁺.

**Step c:** A solution of 3-((3-chloro-2-(pyrazine-2-yl)pyridine-4-yl)sulfanyl)propanoic acid 2-ethylhexyl ester (637mg, 1.565mmol, 1.0eqv), and NaOH (66mg, 1.643mmol, 1.05eqv) in methanol (60ml) was reacted at 50°C for 3 hours under the protection of nitrogen gas. After cooling down to about 0°C, the reaction system was adjusted to pH=3-4 by the dropwise addition of 4M HCl/MeOH solution, concentrated under reduced pressure, purified with column chromatography (n-hexane:EA=10:1-5:1) to produce 3-chloro-2-(pyrazine-2-yl)pyridine-4-thiol (320mg, yield: 91.7%). (ESI, m/z): 223.92[M+H]⁺.

### Intermediate Preparation Example 11: (R)-2-methyl-N-((R)-3H-spiro[benzofuran-2,4'-piperidine]-3-yl)propane-2-sulfinamide (Intermediate 11)

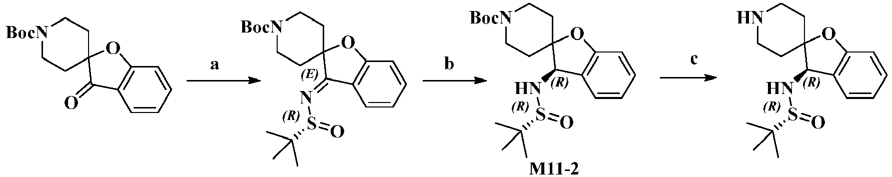

**Steps a and b:** These steps were performed according to the process for preparing Compound 8 from Compound 7 in WO2021061706A1.

**Step c:** The product M11-2 (5.0g, 12.238mmol) was dissolved in DCM (90ml), and trifluoroacetic acid (30ml) was added dropwise at 0°C. The reaction mixture was stirred at room temperature for 3 hours. The reaction system was concentrated under reduced pressure. To the residue was added water (50ml). The resulting mixture was adjusted with 25% aqueous ammonia to pH=10. The aqueous phase was extracted with a mixed solution of EA:THF=1:1 (60ml×4). The organic phases were combined, dried over anhydrous sodium sulfate, and concentrated under reduced pressure to produce (R)-2-methyl-N-((R)-3H-spiro[benzofuran-2,4'-piperidine]-3-yl)propane-2-sulfinamide (3.2g). (ESI, m/z): 309.10[M+H]⁺.

### Intermediate Preparation Example 11A: (R)-N-((S)-1,3-dihydrospiro[indene-2,4'-piperidine]-1-yl)-2-methylpropane-2-sulfinamide (Intermediate 11A)

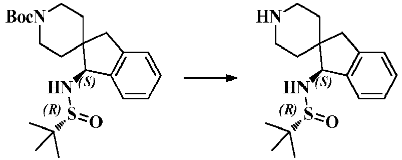

A solution of (S)-tert-butyl 1-((R)-1,1-dimethylethylsulfinylamino)-1,3-dihydrospiro[indene-2,4'-piperidine]-1'-carboxylate (48.00 g)and TFA (40ml) in DCM (160ml) was reacted at room temperature overnight. The reaction solution was concentrated under reduced pressure. To the residue were added EA (200ml) and water (200ml). The resulting mixture was adjusted with 25% aqueous ammonia to pH=10, and separated into two phases. The aqueous phase was extracted with EA (100ml×2). The organic phases were combined. The combined organic phase was washed with water (100ml), and brine (100ml), dried over anhydrous sodium sulfate, and concentrated to produce (R)-N-((S)-1,3-dihydrospiro[indene-2,4'-piperidine]-1-yl)-2-methylpropane-2-sulfinamide (crude, Intermediate 11A)(32g). (ESI, m/z): 307.09 [M+H]⁺.

### Intermediate Preparation Example 12: (R)-(1'-(3-acetyl-6-amino-5-bromopyrazine-2-yl)-3H-spiro[benzofuran-2,4'-piperidine]-3-yl)carbamic acid tert-butyl ester (Intermediate 12)

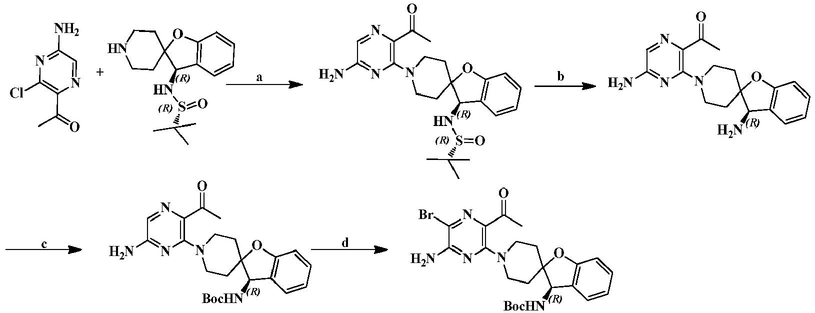

**Step a:** A solution of 1-(5-amino-3-chloropyrazine-2-yl)ethanone (266mg, 1.555mmol, 1.0eqv), (R)-2-methyl-N-((R)-3H-spiro[benzofuran-2,4'-piperidine]-3-yl)propane-2-sulfinamide (575mg, 1.866mmol, 1.2eqv), and DIPEA (603mg, 4.6655mmol, 3.0eqv) in DMSO (10ml) was reacted at 110 °C for 8 hours, and cooled down to room temperature. Water (50ml) was added. The resulting mixture was extracted with EA (3×30ml). The organic phases were combined. The combined organic phase was washed with H₂O (100ml) and brine (100ml), concentrated under reduced pressure, and purified by silica gel column chromatography (eluent: DCM:MeOH=80:1-50:1) to produce (R)-N-((R)-1'-(3-acetyl-6-aminopyrazine-2-yl)-3H-spiro[benzofuran-2,4'-piperidine]-3-yl)-2-methylpropane-2-sulfinamide (321mg, yield: 46%). (ESI, m/z):444.16[M+H]⁺.

**Step b:** To a reaction bottle containing (R)-N-((R)-1'-(3-acetyl-6-aminopyrazine-2-yl)-3H-spiro[benzofuran-2,4'-piperidine]-3-yl)-2-methylpropane-2-sulfinamide (321mg, 0.724mmol, 1.0eqv) was added HCl/CH₃OH solution (10mL). The resulting mixture was reacted at room temperature for 4 hours, and then the reaction solution was rotary dried to obtain a crude product (R)-1-(5-amino-3-(3-amino-3H-spiro[benzofuran-2,4'-piperidine]-1'-yl)pyrazine-2-yl)ethanone. (ESI, m/z):340.10[M+H]⁺.

**Step c:** TEA (220mg, 2.175mmol, 3.0eqv) was added to a solution of (R)-1-(5-amino-3-(3-amino-3H-spiro[benzofuran-2,4'-piperidine]-1'-yl)pyrazine-2-yl)ethanone (246mg, 0.724mmol, 1.0eqv) in DCM (50ml) at room temperature. Then (Boc)₂O (316mg, 1.448mmol, 2.0eqv) was added. The reaction was stirred overnight at room temperature. To the reaction solution was added water (50ml), and the resulting mixture was separated into two phases. The aqueous phase was extracted with DCM (20ml×2). The organic phases were combined. The combined organic phase was washed with H₂O (20mL), and brine (20mL), concentrated under reduced pressure, and purified by silica gel column chromatography (n-hexane: EA=5:1-1:5) to produce (R)-(1'-(3-acetyl-6-aminopyrazine-2-yl)-3H-spiro[benzofuran-2,4'-piperidine]-3-yl)carbamic acid tert-butyl ester (221mg, yield: 69.5%). (ESI, m/z): 440.15[M+H]⁺.

**Step d:** NBS (94mg, 0.528mmol, 1.05eqv) was added to a solution containing (R)-(1'-(3-acetyl-6-aminopyrazine-2-yl)-3H-spiro[benzofuran-2,4'-piperidine]-3-yl)carbamic acid tert-butyl ester (221mg, 0.503mmol, 1.0eqv) in DCM (20ml) at -15°C. The reaction was stirred at a constant temperature for 10 minutes. The reaction solution was quenched by adding a saturated aqueous sodium bicarbonate solution (10ml). The resulting mixture was separated into two phases. The aqueous phase was extracted with DCM (10ml×2). The organic phases were combined. The combined organic phase was washed with H₂O (20ml), and brine (20ml), concentrated under reduced pressure, and purified by silica gel column chromatography (n-hexane:EA=5:1-1:1) to produce (R)-(1'-(3-acetyl-6-amino-5-bromopyrazine-2-yl)-3H-spiro[benzofuran-2,4'-piperidine]-3-yl)carbamic acid tert-butyl ester (132mg, yield: 50.75%). (ESI, m/z):518.09[M+H]⁺.

### Intermediate Preparation Example 13: (R)-5-amino-6-bromo-3-(3-((tert-butyloxycarbonyl)amino)-3H-spiro[benzofuran-2,4'-piperidine]-1'-yl)pyrazine-2-carboxylic acid methyl ester (Intermediate 13)

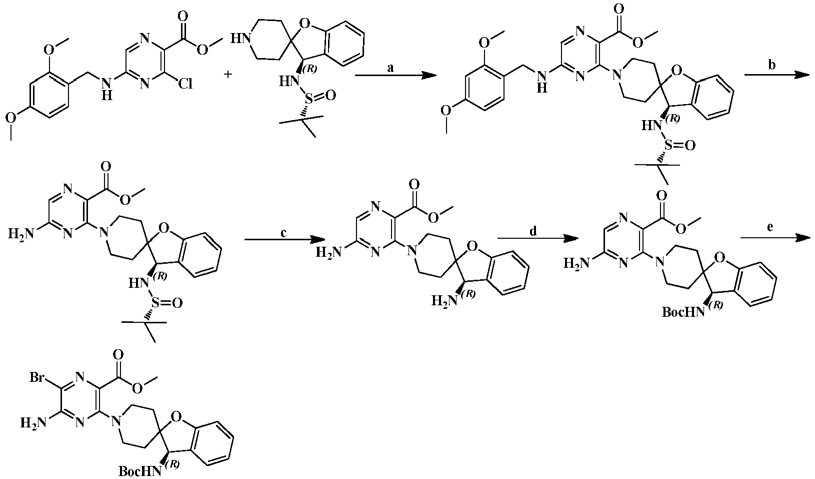

**Step a:** A solution of 3-chloro-5-((2,4-dimethoxybenzyl)amino)pyrazine-2-carboxylic acid methyl ester (200mg, 0.593mmol, 1.0eqv), (R)-2-methyl-N-((R)-3H-spiro[benzofuran-2,4'-piperidine]-3-yl)propane-2-sulfinamide (201mg, 0.653mmol, 1.1eqv) and DIPEA (230mg, 1.779mmol, 3.0eqv) in DMSO (10ml) was reacted at 110°C for 10 hours, and cooled down to room temperature. To the reaction solution were added water (60ml) and EA (60ml). The resulting mixture was separated into two phases. The aqueous phase was extracted with EA (3×20ml). The organic phases were combined. The combined organic phase was washed with H₂O (100m1) and brine (50ml), concentrated under reduced pressure, and purified by elution by silica gel column chromatography (eluent: EA:MeOH=80:1-20:1) to produce 5-((2,4-dimethoxybenzyl)amino)-3-((R)-3-((R)-1,1-dimethylethylsulfinylamino)-3H-spiro[benzofuran-2,4'-piperidine]-1'-yl)pyrazine-2-carboxylic acid methyl ester (305mg, yield: 84.42%). (ESI, *m*/*z*):610.20[M+H]⁺.

**Step b:** 5-((2,4-dimethoxybenzyl)amino)-3-((R)-3-((R)- 1, 1 -dimethylethylsulfinylamino)-3H-spiro[benzofuran-2,4'-piperidine]-1'-yl)pyrazine-2-carboxylic acid methyl ester (305mg, 0.501mmol, 1.0eqv) was dissolved in CF₃COOH (6ml). The reaction mixture was stirred under stirring at room temperature for 3 hours. The reaction solution was rotary dried to obtain a crude product of 5-amino-3-((R)-3-((R)-1,1-dimethylethylsulfinylamino)-3H-spiro[benzofuran-2,4'-piperidine]-1'-yl)pyrazine-2-carboxylic acid methyl ester (230mg). (ESI, *m*/*z*):460.15[M+H]⁺*.*

**Step c:** 5-amino-3-((R)-3-((R)-1,1-dimethylethylsulfinylamino)-3H-spiro[benzofuran-2,4'-piperidine]-1'-yl)pyrazine-2-carboxylic acid methyl ester (crude, 230mg) was dissolved in HCl/CH₃OH solution (10mL), and the resulting mixture was reacted at room temperature for 2 hours, and then the reaction solution was rotary dried to obtain a crude product (R)-5-amino-3-(3-amino-3H-spiro[benzofuran-2,4'-piperidine]-1'-yl)pyrazine-2-carboxylic acid methyl ester (178mg). (ESI, *m*/*z*):356.11[M+H]⁺.

**Step d:** To TEA (253.3mg, 2.505mmol, 5.0eqv) was added a solution of (R)-5-amino-3-(3-amino-3H-spiro[benzofuran-2,4'-piperidine]-1'-yl)pyrazine-2-carboxylic acid methyl ester (178mg, 0.501mmol, 1.0eqv) in DCM (20ml) at room temperature. Then (Boc)₂O (142mg, 0.651mmol, 1.3eqv) was added. The reaction mixture was stirred overnight at room temperature. To the reaction solution was added water (25ml), and the resulting mixture was separated into two phases. The aqueous phase was extracted with DCM (10ml×2). The organic phases were combined. The combined organic phase was washed with H₂O (20ml), and brine (20ml), concentrated under reduced pressure, and purified by silica gel column chromatography (eluent: DCM:MeOH=100:1-20:1) to produce (R)-5-amino-3-(3-((tert-butyloxycarbonyl)amino)-3H-spiro[benzofuran-2,4'-piperidine]-1'-yl)pyrazine-2-carboxylie acid methyl ester (133mg, yield: 58.3%). (ESI, m/z):456.16[M+H]⁺.

**Step e:** NBS (57mg, 0.321mmol, 1.1eqv) was added to a solution of (R)-5-amino-3-(3-((tert-butyloxycarbonyl)amino)-3H-spiro[benzofuran-2,4'-piperidine]-1'-yl)pyrazine-2-carboxylie acid methyl ester (133mg, 0.292mmol, 1.0eqv) in DCM (30ml) at -15°C. The system was maintained at the same temperature for 10 minutes. To the reaction solution was added a saturated aqueous sodium bicarbonate solution (20ml), and the resulting mixture was separated into two phases. The aqueous phase was extracted with DCM (10ml×2). The organic phases were combined. The combined organic phase was washed with H₂O (20ml), and brine (20ml), concentrated under reduced pressure, and purified by silica gel column chromatography (eluent: n-hexane:EA=5:1-1:2) to produce (R)-5-amino-6-bromo-3-(3-((tert-butyloxycarbonyl)amino)-3H-spiro[benzofuran-2,4'-piperidine]-1'-yl)pyrazine-2-carboxylie acid methyl ester (100mg, yield: 64.3%). (ESI, m/z):534.09[M+H]⁺.

### Intermediate Preparation Example 14: (R)-6-bromo-3-(3-((tert-butyloxycarbonyl)amino)-3H-spiro[benzofuran-2,4'-piperidine]-1'-yl)pyrazine-2-carboxylic acid methyl ester (Intermediate 14)

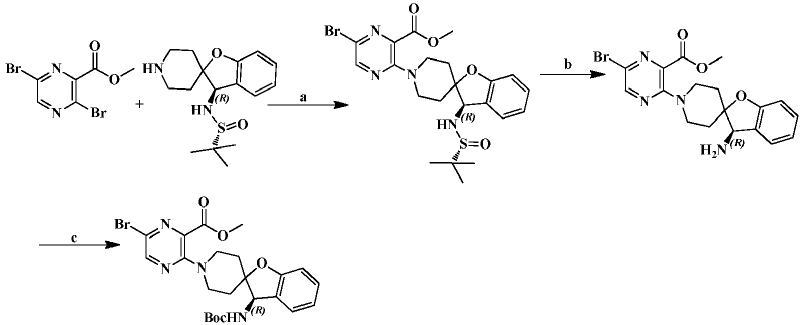

**Step a:** A solution of 3,6-dibromopyrazine-2-carboxylic acid methyl ester (460mg, 1.555mmol, 1.0eqv), (R)-2-methyl-N-((R)-3H-spiro[benzofuran-2,4'-piperidine]-3-yl)propane-2-sulfinamide (575mg, 1.866mmol, 1.2eqv), and DIPEA (603mg, 4.6655mmol, 3.0eqv) in DMSO (10ml) was reacted at 110 °C for 6 hours. After cooling down to room temperature, water (50ml) was added. The resulting mixture was extracted with EA (3×30ml). The organic phases were combined. The combined organic phase was washed with H₂O (100ml) and brine (100m1), concentrated under reduced pressure, and purified by silica gel column chromatography (eluent: DCM:MeOH=100:1-40:1) to produce 6-bromo-3-((R)-3-((R)-1,1-dimethylethylsulfinylamino)-3H-spiro[benzofuran-2,4'-piperidine]-1-yl)pyrazine-2-carboxylic acid methyl ester (375mg, yield: 46.2%). (ESI, *m*/*z*):523.01[M+H]⁺.

**Step b:** 6-bromo-3-((R)-3-((R)-1,1-dimethylethylsulfinylamino)-3H-spiro[benzofuran-2,4'-piperidine]-1'-yl)pyrazine-2-carboxylic acid methyl ester (375mg, 0.718mmol, 1.0eqv) was dissolved in HCl/CH₃OH solution (10ml). The resulting mixture was reacted under stirring at room temperature for 4 hours. The reaction solution was rotary dried to obtain a crude product (R)-3-(3-amino-3H-spiro[benzofuran-2,4'-piperidine]-1'-yl)-6-bromopyrazine-2-carboxylic acid methyl ester. (ESI, *m*/*z*):419.02[M+H]⁺.

**Step c:** At room temperature, TEA(218mg, 2.155mmol, 3.0eqv) was added to a solution of (R)-3-(3-amino-3H-spiro[benzofuran-2,4'-piperidine]-1'-yl)-6-bromopyrazine-2-carboxylic acid methyl ester (300mg, 0.718mmol, 1.0eqv) in DCM (50ml). Then (Boc)₂O (299mg, 1.437mmol, 2.0eqv) was added. The reaction mixture was stirred overnight at room temperature. To the reaction solution was added water (50ml), and the resulting mixture was separated into two phases. The aqueous phase was extracted with DCM (20ml×2). The organic phases were combined. The combined organic phase was washed with H₂O (20mL) and brine (20mL), concentrated under reduced pressure, and purified by silica gel column chromatography (n-hexane:EA=5:1-1:5) to produce (R)-6-bromo-3-(3-((tert-butyloxycarbonyl)amino)-3H-spiro[benzofuran-2,4'-piperidine]-1'-yl)pyrazine-2-carboxylic acid methyl ester (279mg, yield: 75%). (ESI, m/z): 519.09[M+H]⁺.

### Intermediate Preparation Example 14A:

The preparation of Intermediate 14A was identical to the preparation of Intermediate 14, the following corresponding starting materials were used to synthesize Intermediate 14A:

| Intermediate Preparation Example No. | Main Starting Materials | Intermediate Structure and No. | Characterization Data |
|---|---|---|---|
| 14A | 3,6-dibromopyrazine-2-carboxylic acid methyl ester, Intermediate 11A | 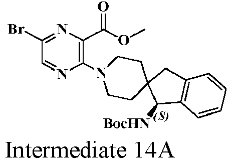 | (ESI, m/z): 517.08[M+H] ⁺ |

### Intermediate Preparation Example 15: (R)-(1'-(3-acetyl-5-bromopyrazine-2-yl)-3H-spiro[benzofuran-2,4'-piperidine]-3-yl)carbamic acid tert-butyl ester (Intermediate 15)

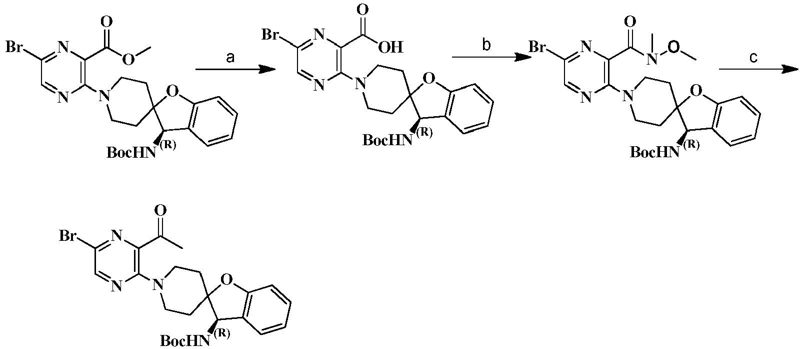

**Step a:** A solution of (R)-6-bromo-3-(3-((tert-butyloxycarbonyl)amino)-3H-spiro[benzofuran-2,4'-piperidine]-1'-yl)pyrazine-2-carboxylic acid methyl ester (500mg, 0.965mmol, 1eqv), LiOH·H₂O (81mg, 1.93mmol, 2eqv) in THF (10ml) and water (10ml) was reacted at room temperature overnight. The reaction mixture was adjusted with 1N HCl solution to pH=3-4, and concentrated under reduced pressure. To the residue was added water (60ml) and EA (60ml), and the resulting mixture was separated into two phases. The aqueous phase was extracted with EA (3×20ml). The organic phases were combined. The combined organic phase was washed with H₂O (100m1) and brine (50ml), concentrated under reduced pressure, and purified by silica gel column chromatography (eluent: DCM:MeOH=80:1-20:1) to produce (R)-6-bromo-3-(3-((tert-butyloxycarbonyl)amino)-3H-spiro[benzofuran-2,4'-piperidine]-1'-yl)pyrazine-2-carboxylic acid (440mg, yield: 90.45%). (ESI, *m*/*z*):505.02[M+H]⁺.

**Step b:** To a solution of (R)-6-bromo-3-(3-((tert-butyloxycarbonyl)amino)-3H-spiro[benzofuran-2,4'-piperidine]-1'-yl)pyrazine-2-carboxylic acid (400mg, 0.793mmol, 1eqv) in anhydrous DMF (10ml) were added N-methoxymethylamine (58mg, 0.952mmol, 1.2eqv), EDCI (182.4mg, 0.952mmol, 1.2eqv), HOBt (128.6mg, 0.952mmol, 1.2eqv) and Et₃N (160mg, 1.586mmol, 2eqv) at 0°C under the protection of nitrogen gas. Then the reaction mixture was stirred at room temperature for 6 hours. To the reaction were added water (60ml) and EA (60ml), and the resulting mixture was separated into two phases. The aqueous phase was extracted with EA (3×20ml). The organic phases were combined. The combined organic phase was washed with H₂O (100m1) and brine (50ml), concentrated under reduced pressure, and purified by silica gel column chromatography (eluent: DCM:MeOH=100:1-25:1) to produce (R)-(1'-(5-bromo-3-(methoxy(methyl)carbamoyl)pyrazine-2-yl)-3H-spiro[benzofuran-2,4'-piperidine]-3-yl)carbamic acid tert-butyl ester (227mg, yield: 52.32%). (ESI, *m*/*z*):548.10[M+H]⁺.

**Step c:** To a solution of (R)-(1'-(5-bromo-3-(methoxy (methyl)carbamoyl)pyrazine-2-yl)-3H-spiro[benzofuran-2,4'-piperidine]-3-yl)carbamic acid tert-butyl ester (227mg, 0.415mmol, 1eqv) in anhydrous THF (10ml) was added dropwise magnesium methyl bromide (3.0M, 0.276mL, 2eqv) at -78°C under the protection of nitrogen gas. The reaction mixture was naturally warmed up to 0°C and stirred for 2 hours. The reaction was quenched with a saturated NH₄Cl solution. To the reaction mixture were added water (60ml) and EA (60ml), and the resulting mixture was separated into two phases. The aqueous phase was extracted with EA (3×20ml). The organic phases were combined. The combined organic phase was washed with H₂O (100m1) and brine (50ml), concentrated under reduced pressure, and purified by silica gel column chromatography (eluent: DCM:MeOH=50:1-20:1) to produce (R)-(1'-(3-acetyl-5-bromopyrazine-2-yl)-3H-spiro[benzofuran-2,4'-piperidine]-3-yl)carbamic acid tert-butyl ester (100mg, yield: 48%). (ESI, m/z):503.07[M+H]⁺.

### Intermediate Preparation Examples 15A-15C:

The preparation of Intermediates 15A-15C was identical to the preparation of Intermediate 15, the following corresponding starting materials were used to synthesize Intermediates 15A-15C:

| Intermediate Preparation Example No. | Main Starting Materials | Intermediate Structure and No. | Characterization Data |
|---|---|---|---|
| 15A | Intermediate 14A, magnesium methyl bromide | 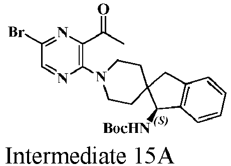 | (ESI, m/z): 501.08[M+H]⁺ |
| 15B | Intermediate 14, magnesium cyclopropyl bromide | 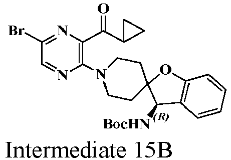 | (ESI, m/z): 529.08[M+H] ⁺ |
| 15C | Intermediate 14A, magnesium cyclopropyl bromide | 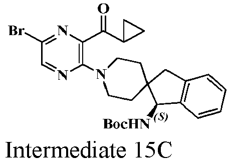 | (ESI, m/z): 527.11[M+H]⁺ |

### Intermediate Preparation Example 16: 6-chloro-3-((2-chloro-3-(oxazole-2-yl)phenyl)sulfanyl)-5-(oxetane-3-yl)pyrazine-2-amine (Intermediate 16)

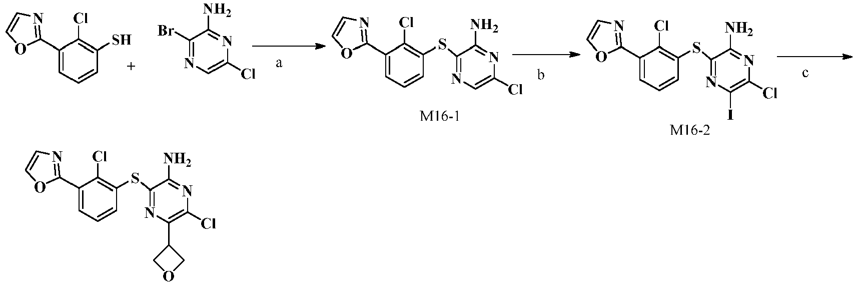

**Step a:** A solution of 3-bromo-6-chloro-pyrazine-2-amine (5.00 g, 23.988 mmol, 1.00 equiv), 2-chloro-3-(oxazole-2-yl)benzenethiol (6.70g, 28.786mmol, 1.2 eqv), DIPEA (9.30 g, 71.963 mmol, 3.0 eqv), Pd₂(dba)₃.CHCl₃ (0.62 g, 0.600 mmol, 0.025 eqv) and XantPhos (0.69 g, 1.199 mmol, 0.05 eqv) in 1,4-dioxane (100 mL) was reacted overnight at 95°C under the protection of nitrogen gas. After cooling down to room temperature, the reaction solution was filtered by suction with diatomite. The filtrate was concentrated under reduced pressure. The residue was purified by column chromatography (n-hexane:EA=2:1) to produce 6-chloro-3-((2-chloro-3-(oxazole-2-yl)phenyl)sulfanyl)pyrazine-2-amine (Intermediate M16-1)(5.86g, yield: 72.3%). (ESI, m/z):338.90[M+H]⁺.

**Step b:** To a solution of 6-chloro-3-((2-chloro-3-(oxazole-2-yl)phenyl)sulfanyl)pyrazine-2-amine (5.50 g, 26.085mmol, 1.00 eqv), and K₂CO₃ (7.20g, 52.17mmol, 2.00 eqv) in MeOH (160ml) was added dropwise a solution of ICl (6.35 g, 39.128 mmol, 1.50 eqv) in DCM (160ml) at 0°C under the protection of nitrogen gas. The reaction mixture was stirred under controlling the temperature for 2 hours and then stirred at room temperature overnight. The reaction solution was quenched by adding 500ml of 10% Na₂SO₃. The resulting mixture was separated into two phases. The aqueous phase was extracted with DCM (3×250ml). The organic phases were combined. The combined organic phase was washed with saturated NaCl (250ml), dried over anhydrous sodium sulfate, and concentrated under reduced pressure to produce 6-chloro-3-((2-chloro-3-(oxazole-2-yl)phenyl)sulfanyl)-5-iodopyrazine-2-amine (Intermediate M16-2)(8.71g, yield: 72%). (ESI, *m*/*z*):464.80 [M+H]⁺.

**Step c:** To a solution of dtbpy (551mg, 1.832mmol, 0.10 eqv) and DMA (200ml). NiBr₂.DME (724mg, 1.832mmol, 0.10 eqv) was added under the protection of nitrogen gas. The resulting mixture was stirred at room temperature for 0.5 hours. Then 6-chloro-3-((2-chloro-3-(oxazole-2-yl)phenyl)sulfanyl)-5-iodopyrazine-2-amine (8.5g, 18.324mmol, 1.00 eqv), 3-bromooxetane (5.624g, 36.647 mmol, 2.00 eqv), TBAI (758.2mg, 1.832mmol, 0.10 eqv), and Zn (2.686g, 36.647mmol, 2.00 eqv) were added. The resulting mixture was reacted at 75°C overnight under the protection of nitrogen gas, and filtered by suction with diatomite. The filtrate was poured into water (1000ml). The resulting mixture was extracted with EA (2×500ml). The organic phases were combined. The combined organic phase was washed with brine, dried over anhydrous sodium sulfate, and concentrated under reduced pressure. The residue was purified by Prep-HPLC (eluting with 20-50% of aqueous acetonitrile solution in gradient and modifying with 0.1% NH₄HCO₃) to produce 6-chloro-3-((2-chloro-3-(oxazole-2-yl)phenyl)sulfanyl)-5-(oxetane-3-yl)pyrazine-2-amine (810mg, yield: 11.22%). (ESI, *m*/*z*):394.92 [M+H]⁺.

### Intermediate Preparation Example 17: 6-chloro-3-((2-chloro-3-(oxazole-2-yl)phenyl)sulfanyl)-5-vinylpyrazine-2-amine (Intermediate 17)

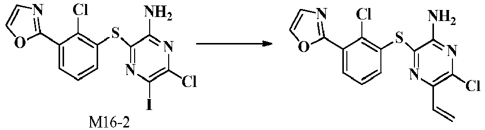

A solution of 6-chloro-3-((2-chloro-3-(oxazole-2-yl)phenyl)sulfanyl)-5-iodopyrazine-2-amine (197mg, 0.425mmol, 1eqv), potassium vinyltrifluoroboronate (116mg, 0.85mmol, 2eqv), Pd(PPh₃)₄ (50mg, 0.0425mmol, 0.1eqv), and potassium carbonate (176mg, 1.275mmol, 3eqv) in toluene (60ml), ethanol (30ml) and water (15ml) was reacted at 80 °C for 5 hours under the protection of nitrogen gas. The reaction solution was concentrated under reduced pressure. To the residue was added water (100ml). The resulting mixture was extracted with EA (2×50ml). The organic phases were combined. The combined organic phase was washed with brine (50ml), and concentrated under reduced pressure. The residue was purified by column chromatography (CH₂Cl₂:CH₃OH=100:1) to produce 6-chloro-3-((2-chloro-3-(oxazole-2-yl)phenyl)sulfanyl)-5-vinylpyrazine-2-amine (101mg, yield: 65.3%). (ESI, m/z): 364.93[M+H]⁺.

### Intermediate Preparation Example 18: 4-(oxazole-2-yl)pyrimidine-2-thiol (Intermediate 18)

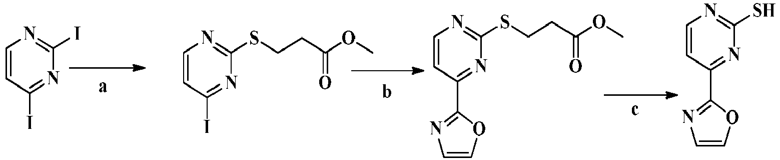

**Step a:** A solution of 2,4-diiodopyrimidine (7.96g, 23.99 mmol, 1eqv), 3-mercaptopropanoic acid methyl ester (3.02g, 25.19 mmol, 1.05eqv), DIPEA(6.20 g, 47.97 mmol, 2 eqv), Pd(OAc)₂ (538.53 mg, 2.40 mmol, 0.1 eqv) and Xantphos (2.08 g, 3.60 mmol, 0.15 eqv) in 1,4-dioxane (160ml) was reacted at 95°C overnight under the protection of nitrogen gas. After cooling down to room temperature, the reaction solution was filtered by suction with diatomite. The filtrate was concentrated under reduced pressure. The residue was purified by column chromatography (n-hexane:EA=100:1-20:1) to produce 3-((4-iodopyrimidine-2-yl)sulfanyl)propanoic acid methyl ester (5 g, yield: 64.34%). (ESI, *m*/*z*): 324.90[M+H]⁺.

**Step b:** A solution of 3-((4-iodopyrimidine-2-yl)sulfanyl)propanoic acid methyl ester (595mg, 1.834mmol, 1.0eqv), oxazole-2-ylboronic acid (228mg, 2.02mmol, 1.1eqv), Pd(dppf)Cl₂ (134mg, 0.183mmol, 0.1eqv) and potassium carbonate (760mg, 5.503mmol, 3eqv) in 1,4-dioxane (50 mL) and water (10ml) was reacted at 96 °C overnight under the protection of nitrogen gas. The resulting mixture was cooled down, and concentrated under reduced pressure. To the residue were added water (200ml) and EA (100ml), and the resulting mixture was separated into two phases. The aqueous phase was extracted with EA (2×50ml). The organic phases were combined. The combined organic phase was washed with brine (100ml), and concentrated under reduced pressure. The residue was purified by column chromatography (n-hexane:EA=30:1-10:1) to produce 3-((4-(oxazole-2-yl)pyrimidine-2-yl)sulfanyl)propanoic acid methyl ester (256mg, yield: 52.65%). (ESI, m/z): 266[M+H]⁺.

**Step c:** A solution of 3-((4-(oxazole-2-yl)pyrimidine-2-yl)sulfanyl)propanoic acid methyl ester (256mg, 0.966mmol, 1.0eqv), and NaOH (40.6mg, 1.014mmol, 1.05eqv) in methanol (10ml) was reacted at 50°C for 4 hours under the protection of nitrogen gas. After cooling down to 0°C, the resulting mixture was adjusted by the dropwise addition of 4M HCl/MeOH solution to pH=3-4. Silica gel was added. The system was purified by column chromatography (n-hexane:EA=10:1-5:1) to produce 4-(oxazole-2-yl)pyrimidine-2-thiol (130mg, yield: 75.2%). (ESI, *m*/*z*): 179.92[M+H]⁺.

### Intermediate Preparation Examples 19-22

The preparation of Intermediates 19-22 was identical to the preparation of Intermediate 18, the following corresponding starting materials were used to synthesize intermediates 19-22:

| Intermediate Preparation Example No. | Starting Materials | Intermediate Structure and No. | Characterization Data |
|---|---|---|---|
| 19 | 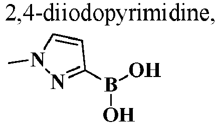 | 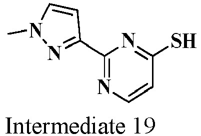 | (ESI, m/z): 193[M+H]⁺ |
| 20 | 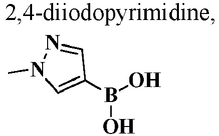 | 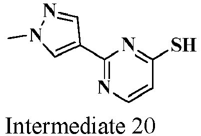 | (ESI, m/z): 193.01[M+H]⁺ |
| 21 | 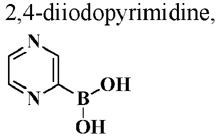 | 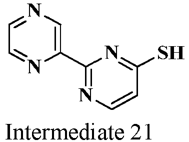 | (ESI, m/z): 191[M+H]⁺ |
| 22 | 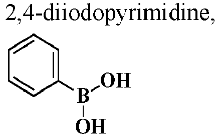 | 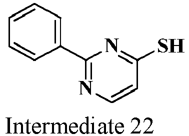 | (ESI, m/z): 189[M+H]⁺ |

### Intermediate Preparation Example 23: (R)-2-methyl-N-((R)-3-deuterium-spiro[benzofuran-2,4'-piperidine]-3-yl)propane-2-sulfinamide (M-23)

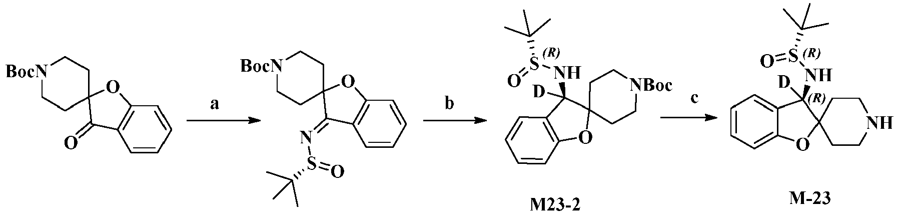

**Steps a and b:** To a solution of 3-oxospiro[1-benzofuran-2,4'-piperidine]-1'-carboxylic acid tert-butyl ester (10.00 g, 32.965mmol, 1.00 eqv) and (R)-(+)-tert-butyl sulfinamide (7.99g, 65.93mmol, 2eqv) in 2-MeTHF (40ml) was added Ti (OEt)₄ (100ml) under the protection of nitrogen gas, and the resulting mixture was reacted at 80 °C for 48 hours. After cooling down to -5°C, NaBD₄ (1.66g, 39.55 mmol, 1.2 eqv) was added. The reaction mixture was stirred at room temperature overnight. After cooling down to 0°C, methanol (30ml) was added dropwise to the reaction solution. Water (1000ml) and EA (1000ml) were added. The resulting mixture was stirred for 30 minutes, and filtered. The filtrate was separated into two phases. The organic phase was washed with brine (1000ml), dried over anhydrous sodium sulfate, and concentrated under reduced pressure. The residue was purified by column chromatography (n-hexane:EA=6:1-2:1) to produce M23-2 (10.5g, yield: 77.78%), (ESI, *m*/*z*): 410.13[M+H]⁺. ¹H-NMR (600MHz, CDCl₃) δ7.285-7.273 (m, 1H), 7.250-7.224 (m, 1H), 6.929-6.904 (m, 1H), 6.817 (d, J= 7.8Hz, 1H), 4.086 (br s, 2H), 3.673 (s, 1H), 3.178 (s, 2H), 2.011 (s, 1H), 1.856 (s, 1H), 1.770-1.748 (m, 1H), 1.716-1.690 (m, 1H), 1.465 (s, 9H), 1.255 (s, 9H).

**Step c:** M23-2 (10.5g) was dissolved in DCM (20ml). Trifluoroacetic acid (20ml) was added dropwise at 0°C. The resulting mixture was reacted at room temperature for 6 hours, and concentrated under reduced pressure. To the residue was added water (50ml), and the resulting mixture was adjusted with 25% aqueous ammonia to pH=10. The aqueous phase was extracted with EA (50ml×3). The organic phases were combined, dried over anhydrous sodium sulfate, and concentrated under reduced pressure to produce (R)-2-methyl-N-((R)-3-deuteriumspiro[benzofuran-2,4'-piperidine]-3-yl)propane-2-sulfinamide (M-23)(crude, 7.84g) as a crude product. (ESI, *m*/*z*): 310.07[M+H]⁺.

### Intermediate Preparation Example 24:

The preparation of Intermediate 24 was identical to the preparation of Intermediate 23, the following corresponding starting materials were used to synthesize Intermediate 24:

| Intermediate Preparation Example No. | Starting Materials | Intermediate Structure and No. | Characterization Data |
|---|---|---|---|
| 24 | 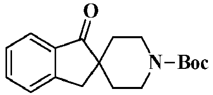 | 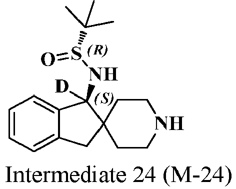 | (ESI, m/z): 308.15[M+H]⁺ |

### Intermediate Preparation Example 25: (R)-(1'-(3-acetyl-6-amino-5-bromopyrazine-2-yl)-3-deuterium - spiro[benzofuran-2,4'-piperidine]-3-yl)carbamic acid tert-butyl ester (M-25)

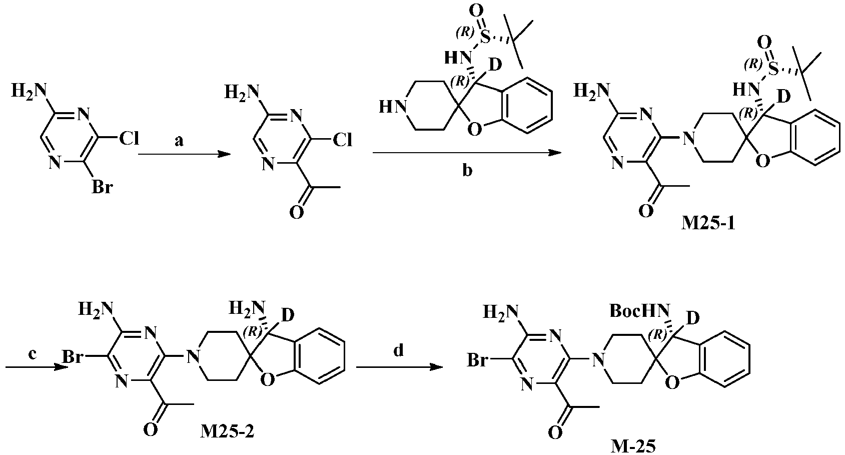

**Steps a and b:** A solution of 2-amino-5-bromo-6-chloropyrazine (20.0g, 96.67mmol, 1.0eqv), tributyl(1-ethoxyvinyl)stannane (52.36g, 144.98mmol, 1.5eqv), CuI (1.84g, 9.667mmol, 0.1eqv), and Pd(Ph₃P)₂Cl₂ (6.78g, 9.667mmol, 0.1eqv) in 1,4-dioxane (240ml) was reacted at 100°C overnight under the protection of nitrogen gas. The TLC detection indicated the completion of the reaction. A solution of (R)-2-methyl-N-((R)-3-deuterium-spiro[benzofuran-2,4'-piperidine]-3-yl)propane-2-sulfinamide (M-23) (crude, 35.86g, 115.987mmol, 1.2eqv) in 1,4-dioxane (200ml) was added to the reaction solution. Then DIPEA (37.48g, 289.97mmol, 3.0eqv) was added, and the resulting mixture was reacted at 100°C overnight. The reaction system was cooled down to room temperature, concentrated under reduced pressure to dryness, and purified by silica gel column chromatography (eluent: EA:n-hexane=1: 1-2:1) to produce M25-1 (24.05g, yield: 56.01%). (ESI, *m*/*z*):445.07[M+H]⁺.

**Step c:** To a solution of M25-1 (24.05g, 54.14mmol, 1.0eqv) in DCM (250ml) was added NBS (11.563g, 64.97mmol, 1.2eqv) at 0°C. The reaction mixture was stirred overnight at room temperature. Then 4M HCl/CH₃OH solution (10ml) was added and the resulting mixture was stirred for 1 hour. To the reaction solution was added a saturated aqueous sodium bicarbonate solution (100ml), and the resulting mixture was separated into two phases. The aqueous phase was extracted with DCM (50ml×2). The organic phases were combined. The combined organic phase was washed with H₂O (200ml) and brine (200ml), dried over anhydrous sodium sulfate, and filtered to produce a solution of the crude M25-2 in DCM as a crude product. (ESI, m/z):419.00[M+H]⁺.

**Step d:** To the solution of M25-2 in DCM obtained from Step c was added (Boc)₂O (14.2g) at 0°C, and added dropwise Et₃N (10.96g). After the completion of the dropwise addition, the reaction mixture was stirred at room temperature overnight. Water (200ml) was added, and the resulting mixture was separated into two phases. The aqueous phase was extracted with DCM (100ml×2). The organic phases were combined. The combined organic phase was washed with H₂O (200ml) and brine (200ml), concentrated under reduced pressure to dryness, and purified by silica gel column chromatography (eluent: EA:n-hexane=1:3-2:1) to produce M-25 (16.43g). (ESI, *m*/*z*):519.09[M+H]⁺.

### Intermediate Preparation Example 25A: M-25A

The preparation of Intermediate 25A was identical to the preparation of Intermediate 25, the following corresponding starting materials were used to synthesize Intermediate 25-A:

| Intermediate Preparation Example No. | Starting Materials | Intermediate Structure and No. | Characterization Data |
|---|---|---|---|
| 25A | 2-amino5-bromo-6-chloropyrazine, M-24 | 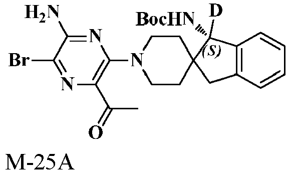 | (ESI, m/z): 517.10[M+H]⁺ |

### Intermediate Preparation Example 26:

The preparation of Intermediate 26 was identical to the preparation of Intermediate 13, the following corresponding starting materials were used to synthesize Intermediate 26:

| Intermediate Preparation Example No. | Main Starting Materials | Intermediate Structure and No. | Characterization Data |
|---|---|---|---|
| 26 | Intermediate 2, Intermediate 23 (M-23) | 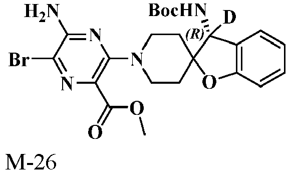 | (ESI, m/z): 535.09[M+H]⁺ |

### Intermediate Preparation Example 27: M-27

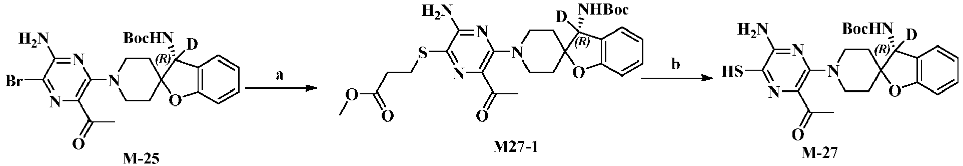

**Step a:** A solution of M-25 (3.00g, 5.78mmol, 1.0eqv), 3-mercaptopropanoic acid methyl ester (1.04g, 8.67mmol, 1.5eqv), Pd(OAc)₂ (250mg, 1.16mmol, 0.2eqv), Xantphos (1.34g, 2.31mmol, 0.4eqv), and DIPEA (2.25g, 7.34mmol, 3eqv) in 1,4-dioxane (60ml) was reacted at 97°C overnight under the protection of nitrogen gas. The reaction system was cooled down, concentrated under reduced pressure, and purified by silica gel column chromatography (eluent: EA:n-hexane=1:3-2:1) to produce M27-1 (3.00g, yield: 93%), (ESI, m/z):559.17 [M+H]⁺.

**Step b:** To a solution of M27-1 (3.00g, 5.37mmol, 1.0eqv) in MeOH (30ml) was added NaOH (242mg, 5.907mmol, 1. 1eqv). The resulting mixture was stirred at 55°C for 2 hours, and concentrated under reduced pressure. To the concentrated mixture was added water (100ml), and the resulting mixture was adjusted with 1 N HCl to pH=3-4, and extracted with EA (100ml×2). The organic phases were combined. The combined organic phase was washed with H₂O (20ml) and brine (20ml), dried over anhydrous sodium sulfate, and filtered. The filtrate was concentrated under reduced pressure to produce M-27 (1.33g, yield: 78.4%)(ESI, *m*/*z):* 473.10[M+H] ⁺.

### Intermediate Preparation Examples 28-30:

The preparation of Intermediates 28-30 was identical to the preparation of Intermediate 27, and the following corresponding starting materials were used to synthesize intermediates 28-30:

| Intermediate Preparation Example No. | Starting Materials | Intermediate Structure and No. | Characterizati on Data |
|---|---|---|---|
| 28 | Intermediate 24 (M-24), 3-mercaptopropanoic acid methyl ester | 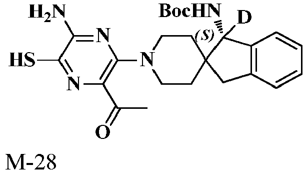 | (ESI, m/z): 471.16[M+H]⁺ |
| 29 | Intermediate 13, 3-mercaptopropanoic acid methyl ester | 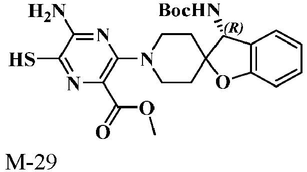 | (ESI, m/z): 488.12[M+H]⁺ |
| 30 | Intermediate 26, 3-mercaptopropanoic acid methyl ester | 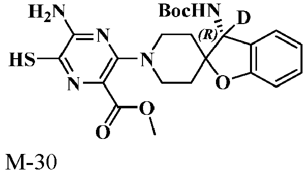 | (ESI, m/z): 489.15[M+H]⁺ |

Examples of preparing the exemplary compounds of this disclosure are provided below.

### Preparation Example 1: (R)-1-(5-amino-3-(3-amino-3H-spiro[benzofuran-2,4'-piperidine]-1'-yl)-6-((2-chloro-3-(oxazole-2-yl)phenyl)sulfanyl)pyrazine-2-yl)ethanone (Compound 1)

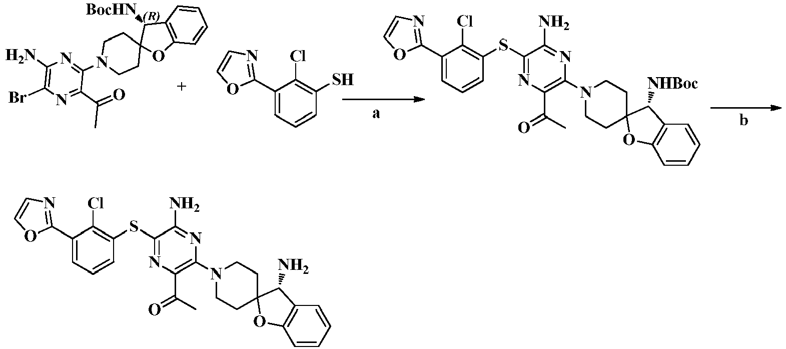

**Step a:** A solution of (R)-(1'-(3-acetyl-6-amino-5-bromopyrazine-2-yl)-3H-spiro[benzofuran-2,4'-piperidine]-3-yl)carbamic acid tert-butyl ester (140 mg, 0.27 mmol, 1.0eqv), 2-chloro-3-(oxazole-2-yl)benzenethiol (69mg, 0.325mmol, 1.2 eqv), DIPEA (123mg, 0.954 mmol, 3.5eqv), Pd₂(dba)₃ (50 mg, 0.054 mmol, 0.2eqv) and Xantphos (63mg, 0.109mmol, 0.4 eqv) in 1,4-dioxane (10mL) was reacted at 96°C for 6 hours under the protection of nitrogen gas. After cooling down to room temperature, silica gel was added to the reaction solution. The reaction system was purified by silica gel column chromatography (eluent: n-hexane:EA=10:1-1:2) to produce (R)-(1'-(3-acetyl-6-amino-5-((2-chloro-3-(oxazole-2-yl)phenyl)sulfanyl)pyrazine-2-yl)-3H-spiro[benzofuran-2,4'-piperidine]-3-yl)carbamic acid tert-butyl ester (152mg, yield: 86.85%). (ESI, m/z):649.14[M+H]⁺.

**Step b:** To a solution of (R)-(1'-(3-acetyl-6-amino-5-((2-chloro-3-(oxazole-2-yl)phenyl)sulfanyl)pyrazine-2-yl)-3H-spiro[benzofuran-2,4'-piperidine]-3-yl)carbamic acid tert-butyl ester (152mg) in dichloromethane (10ml) was added trifluoroacetic acid (5ml). The resulting mixture was stirred at room temperature for 3 hours. The reaction solution was concentrated under reduced pressure. To the residue were added EA (50ml) and water (50ml). The resulting mixture was adjusted with 25% aqueous ammonia to pH=10, and separated into two phases. The aqueous phase was extracted with EA (20ml). The organic phases were combined. The combined organic phase was washed with water (50ml), and brine (50ml), and concentrated under reduced pressure. The residue was purified by column chromatography (CH₂Cl₂:CH₃OH=20:1-15:1) to produce (R)-1-(5-amino-3-(3-amino-3H-spiro[benzofuran-2,4'-piperidine]-1'-yl)-6-((2-chloro-3-(oxazole-2-yl)phenyl)sulfanyl)pyrazine-2-yl)ethanone (120mg, yield: 93.4%). (ESI, *m*/*z*):549.10[M+H]⁺. ¹H-NMR (400MHz, DMSO-d6) δ8.33. (s, 1H), 7.750 (d, J= 6.6 Hz, 1H), 7.476 (s, 1H), 7.416 (t, J= 7.8 Hz, 1H), 7.321 (d, J= 7.2 Hz, 1H), 7.148-7.060 (m, 4H), 6.859 (t, J= 7.2 Hz, 1H), 6.772 (d, J= 7.2 Hz, 1H), 4.105 (s, 1H), 3.876-3.854 (m, 1H), 3.762-3.740 (m, 1H), 3.309-3.290 (m, 1H), 2.320 (s, 3H), 2.033-1.985 (m, 3H), 1.867-1.705 (m, 4H).

### Preparation Examples 2-15 and 11A:

The preparation of Examples 2-15 and 11A was identical to the preparation of Example 1, using the corresponding intermediates as the starting materials.

| Preparation Example No. | Main Starting Materials | Compound Structure | Characterization Data |
|---|---|---|---|
| 2 | Intermediate 6, Intermediate 12 | 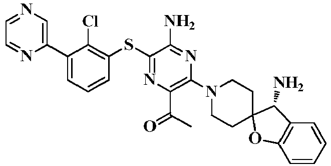 | (ESI, m/z): 560.10[M+H] ⁺ |
| 3 | Intermediate 4, Intermediate 12 | 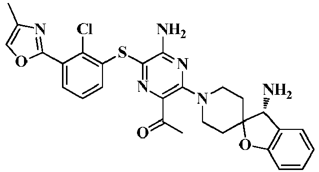 | (ESI, m/z): 563.10[M+H] ⁺ |
| 4 | Intermediate 5, Intermediate 12 | 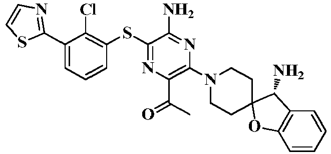 | (ESI, m/z): 565.02[M+H] ⁺ |
| 5 | Intermediate 7, Intermediate 12 | 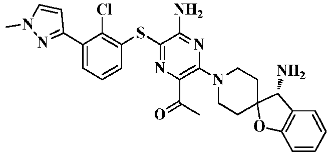 | (ESI, m/z): 562.11[M+H] ⁺ |
| 6 | Intermediate 8, Intermediate 12 | 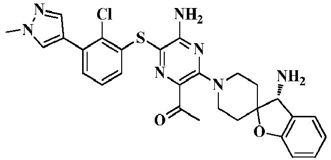 | (ESI, m/z): 562.10[M+H] ⁺ |
| 7 | Intermediate 9, Intermediate 12 | 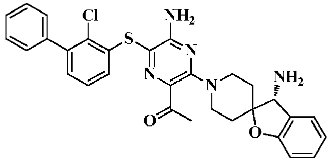 | (ESI, m/z): 558.12[M+H] ⁺ |
| 8 | Intermediate 3, Intermediate 15 | 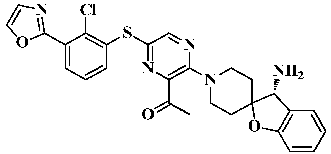 | (ESI, m/z): 534.09[M+H] ⁺ |
| 9 | Intermediate 4, Intermediate 15 | 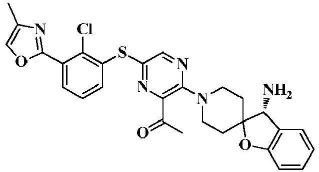 | (ESI, m/z): 548.11[M+H] ⁺ |
| 10 | Intermediate 7, Intermediate 15 | 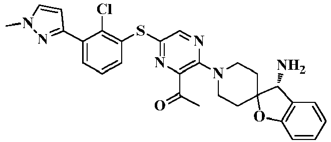 | (ESI, m/z): 547.10[M+H] ⁺ |
| 11 | Intermediate 3, Intermediate 15A | 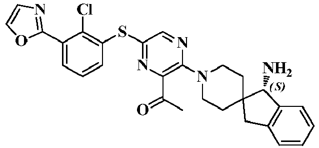 | (ESI, m/z): 532.11[M+H] ⁺ |
| 11A | Intermediate 7, Intermediate 15A | 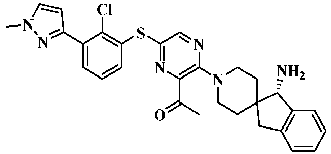 | (ESI, m/z): 545.10[M+H] ⁺ |
| 12 | Intermediate 8, Intermediate 15A | 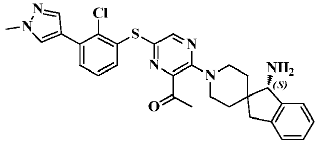 | (ESI, m/z): 545.16[M+H] ⁺ |
| 13 | Intermediate 10, Intermediate 12 | 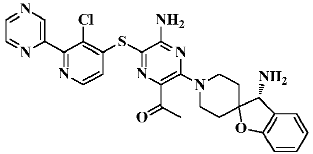 | (ESI, m/z): 561.09[M+H] ⁺ |
| 14 | Intermediate 3, Intermediate 15B | 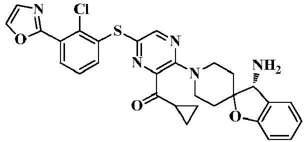 | (ESI, m/z): 560.09[M+H] ⁺ |
| 15 | Intermediate 3, Intermediate 15C | 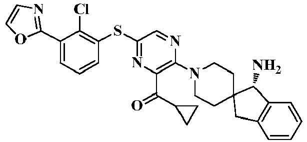 | (ESI, m/z): 558.13[M+H] ⁺ |

### Preparation Example 16: (R)-5-amino-3-(3-amino-3H-spiro[benzofuran-2,4'-piperidine]-1'-yl)-6-((2-chloro-3-(oxazole-2-yl)phenyl)sulfanyl)pyrazine-2-carboxylic acid methyl ester (Compound 16)

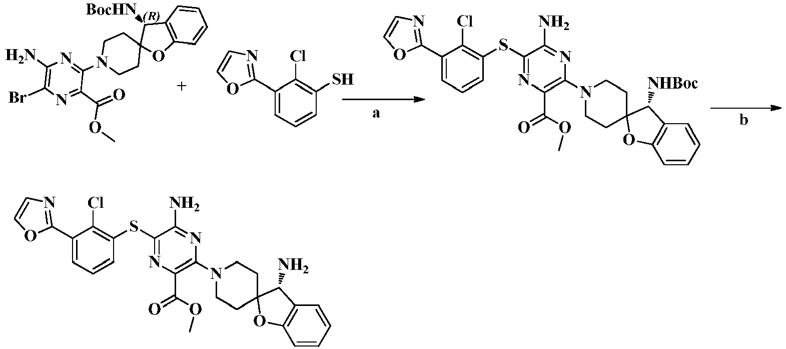

**Step a:** A solution of (R)-5-amino-6-bromo-3-(3-((tert-butyloxycarbonyl)amino)-3H-spiro[benzofuran-2,4'-piperidine]-1'-yl)pyrazine-2-carboxylic acid methyl ester (144 mg, 0.27 mmol, 1.0eqv), 2-chloro-3-(oxazole-2-yl)benzenethiol (69mg, 0.324mmol, 1.2 eqv), DIPEA (123mg, 0.954 mmol, 3.5eqv), Pd₂(dba)₃ (50 mg, 0.054 mmol, 0.2eqv) andXantphos (63mg, 0.109mmol, 0.4 eqv) in 1,4-dioxane (20ml) was reacted at 96°C for 6 hours under the protection of nitrogen gas. After cooling down to room temperature, silica gel was added to the reaction solution. The reaction system was purified by silica gel column chromatography (eluent: n-hexane:EA=20:1-1:1) to produce (R)-5-amino-3-(3-((tert-butyloxycarbonyl)amino)-3H-spiro[benzofuran-2,4'-piperidine]-1'-yl)-6-(2-chloro-3-(oxazole-2-yl)phenyl)sulfanyl)pyrazine-2-carboxylic acid methyl ester (152mg, yield: 84.75%). (ESI, m/z):665.14[M+H]⁺.

**Step b:** To a solution of (R)-5-amino-3-(3-((tert-butyloxycarbonyl)amino)-3H-spiro[benzofuran-2,4'-piperidine]-1'-yl)-6-(2-chloro-3-(oxazole-2-yl)phenyl)sulfanyl)pyrazine-2-carboxylic acid methyl ester (152mg) in dichloromethane (20ml) was added trifluoroacetic acid (5ml). The resulting mixture was stirred at room temperature for 3 hours. The reaction solution was concentrated under reduced pressure. To the residue were added EA (100m1) and water (50ml). The resulting mixture was adjusted with 25% aqueous ammonia to pH=10, and separated into two phases. The aqueous phase was extracted with EA (20ml). The organic phases were combined. The combined organic phase was washed with water (50ml) and brine (50ml), dried over anhydrous sodium sulfate and concentrated. The residue was purified by column chromatography (CH₂Cl₂:CH₃OH=50:1-15:1) to produce (R)-5-amino-3-(3-amino-3H-spiro[benzofuran-2,4'-piperidine]-1'-yl)-6-((2-chloro-3-(oxazole-2-yl)phenyl)sulfanyl)pyrazine-2-carboxylic acid methyl ester (118mg, yield: 91.4%). (ESI, *m*/*z*):565.10[M+H]⁺.

### Preparation Examples 17-20:

The preparation of Examples 17-20 was identical to the preparation of Example 16, using the corresponding intermediates as the starting materials.

| Preparation Example No. | Main Starting Materials | Compound Structure | Characterization Data |
|---|---|---|---|
| 17 | Intermediate 6, Intermediate 13 | 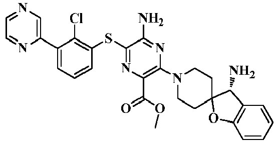 | (ESI, m/z): 576.11 [M+H] ⁺ |
| 18 | Intermediate 3, Intermediate 14 | 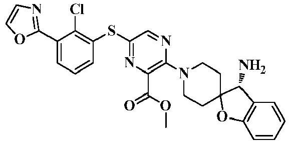 | (ESI, m/z): 576.11 [M+H] ⁺ |
| 19 | Intermediate 7, Intermediate 14A | 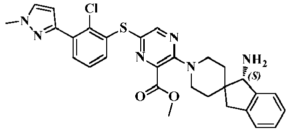 | (ESI, m/z): 561.12 [M+H] ⁺ |
| 20 | Intermediate 3, Intermediate 14A | 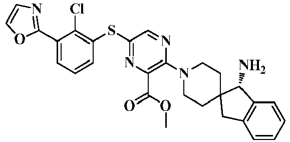 | (ESI, m/z): 548.10 [M+H] ⁺ |

### Preparation Example 21: (R)-1'-(6-amino-5-((2-chloro-3-(oxazole-2-yl)phenyl)sulfanyl)-3-(oxetane-3-yl)pyrazine-2-yl)-3H-spiro[benzofuran-2,4'-piperidine]-3-amine (Compound 21)

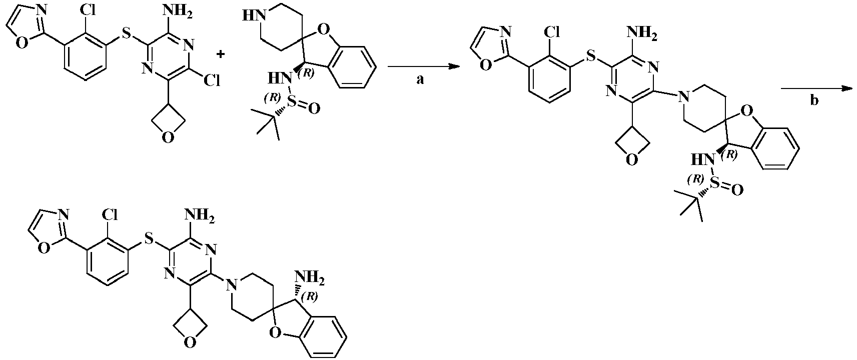

**Step a:** A solution of 6-chloro-3-((2-chloro-3-(oxazole-2-yl)phenyl)sulfanyl)-5-(oxetane-3-yl)pyrazine-2-amine (613mg, 1.556mmol, 1.0eqv), (R)-2-methyl-N-((R)-3H-spiro[benzofuran-2,4'-piperidine]-3-yl)propane-2-sulfinamide (575mg, 1.867mmol, 1.2eqv), and DIPEA (603mg, 4.665mmol, 3.0eqv) in DMSO (10ml) was reacted at 110°C for 6 hours. After cooling down to room temperature, water (100ml) was added. The resulting mixture was extracted with EA (3×50ml). The organic phases were combined. The combined organic phase was washed with H₂O (50ml) and brine (50ml), concentrated under reduced pressure, and purified by silica gel column chromatography (eluent: DCM:MeOH=100:1-25:1) to produce (R)-N-((R)-1'-(6-amino-5-((2-chloro-3-(oxazole-2-yl)phenyl)sulfanyl)-3-(oxetane-3-yl)pyrazine-2-yl)-3H-spiro[benzofuran-2,4'-piperidine]-3-yl)-2-methylpropane-2-sulfinamide (677mg, yield: 65.31%). (ESI, *m*/*z*):667.11[M+H]⁺.

**Step b:** (R)-N-((R)-1'-(6-amino-5-((2-chloro-3-(oxazole-2-yl)phenyl)sulfanyl)-3-(oxetane-3-yl)pyrazine-2-yl)-3H-spiro[benzofuran-2,4'-piperidine]-3-yl)-2-methylpropane-2-sulfinamide (677mg) was dissolved in HCl/CH₃OH solution (60mL). The reaction mixture was stirred at room temperature overnight. The reaction solution was concentrated under reduced pressure. To the residue were added EA (50ml) and water (50ml). The resulting mixture was adjusted with 25% aqueous ammonia to pH=9-10, and separated into two phases. The aqueous phase was extracted with EA (20ml). The organic phases were combined. The combined organic phase was washed with water (50ml), and brine (50ml), and concentrated. The residue was purified by column chromatography (CH₂Cl₂:CH₃OH=20:1) to produce (R)-1'-(6-amino-5-((2-chloro-3-(oxazole-2-yl)phenyl)sulfanyl)-3-(oxetane-3-yl)pyrazine-2-yl)-3H-spiro[benzofuran-2,4'-piperidine]-3-amine (487mg, yield: 85.2%). (ESI, *m*/*z*):563.10[M+H]⁺.

### Preparation Example 22:

The preparation of Example 22 was identical to the preparation of Example 21, using the corresponding intermediates as the starting materials.

| Preparation Example No. | Main Starting Materials | Compound Structure | Characterization Data |
|---|---|---|---|
| 22 | Intermediate 11, Intermediate 17 | 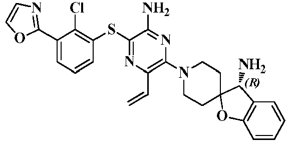 | (ESI, m/z): 533.10 [M+H] ⁺ |

### Preparation Example 23: (R)-1'-(6-amino-5-((2-chloro-3-(oxazole-2-yl)phenyl)sulfanyl)-3-fluoropyrazine-2-yl)-3H-spiro[benzofuran-2,4'-piperidine]-3-amine (Compound 23)

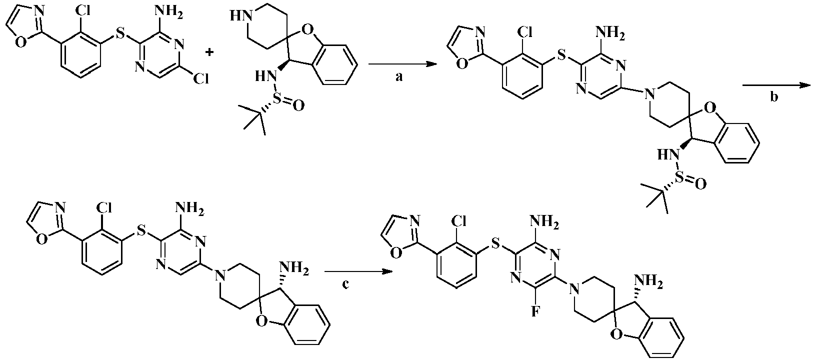

**Step a:** A solution of 6-chloro-3-((2-chloro-3-(oxazole-2-yl)phenyl)sulfanyl)pyrazine-2-amine (526mg, 1.556mmol, 1.0eqv), (R)-2-methyl-N-((R)-3H-spiro[benzofuran-2,4'-piperidine]-3-yl)propane-2-sulfinamide (575mg, 1.867mmol, 1.2eqv), and DIPEA (603mg, 4.665mmol, 3.0eqv) in DMSO (10ml) was reacted at 100°C overnight. After cooling down to room temperature, water (100ml) was added. The resulting mixture was extracted with EA (3×50ml). The organic phases were combined. The combined organic phase was washed with H₂O (50ml) and brine (50ml), concentrated under reduced pressure, and purified by silica gel column chromatography (eluent: DCM:MeOH=80:1-25:1) to produce (R)-N-((R)-1'-(6-amino-5-((2-chloro-3-(oxazole-2-yl)phenyl)sulfanyl)pyrazine-2-yl)-3H-spiro[benzofuran-2,4'-piperidine]-3-yl)-2-methylpropane-2-sulfinamide (724mg, yield: 76.26%). (ESI, *m*/*z*):611.09[M+H]⁺.

**Step b:** (R)-N-((R)-1'-(6-amino-5-((2-chloro-3-(oxazole-2-yl)phenyl)sulfanyl)pyrazine-2-yl)-3H-spiro[benzofuran-2,4'-piperidine]-3-yl)-2-methylpropane-2-sulfinamide (724mg) was dissolved in HCl/CH₃OH solution (50mL). The reaction mixture was stirred at room temperature overnight. The reaction solution was concentrated under reduced pressure. To the residue were added EA (50ml) and water (50ml). The resulting mixture was adjusted with 25% aqueous ammonia to pH=9-10, and separated into two phases. The aqueous phase was extracted with EA (20ml). The organic phases were combined. The combined organic phase was washed with water (50ml), and brine (50ml), and concentrated. The residue was purified by column chromatography (CH₂Cl₂:CH₃OH=30:1-15:1) to produce (R)-1'-(6-amino-5-((2-chloro-3-(oxazole-2-yl)phenyl)sulfanyl)pyrazine-2-yl)-3H-spiro[benzofuran-2,4'-piperidine]-3-amine (557mg, yield: 92.75%). (ESI, *m*/*z*):507.03[M+H]⁺.

**Step c:** To a solution of (R)-1'-(6-amino-5-((2-chloro-3-(oxazole-2-yl)phenyl)sulfanyl)pyrazine-2-yl)-3H-spiro[benzofuran-2,4'-piperidine]-3-amine (557mg, 1.1mmol, 1eqv) in acetonitrile (100ml) solution was added N-Fluorobenzenesulfonimide (N-fluoro-N-(phenylsulfonyl)benzenesulfonamide) (380mg, 1.21eqv, 1.1eqv) at -15°C under the protection of nitrogen gas. After the completion of the addition, the reaction system was warmed up to room temperature, and the reaction mixture was stirred overnight. The reaction system was concentrated under reduced pressure, and purified by Pre-TLC (DCM:MeOH=15:1) twice to produce (R)-1'-(6-amino-5-((2-chloro-3-(oxazole-2-yl)phenyl)sulfanyl)-3-fluoropyrazine-2-yl)-3H-spiro[benzofuran-2,4'-piperidine]-3-amine (109mg, yield: 18.9%). (ESI, *m*/*z*):525.09[M+H]⁺.

### Preparation Example 24: (R)-1-(5-amino-3-(3-amino-3H-spiro[benzofuran-2,4'-piperidine]-1'-yl)-6-((2-(oxazole-2-yl)pyrimidine-4-yl)sulfanyl)pyrazine-2-yl)ethanone (Compound 24)

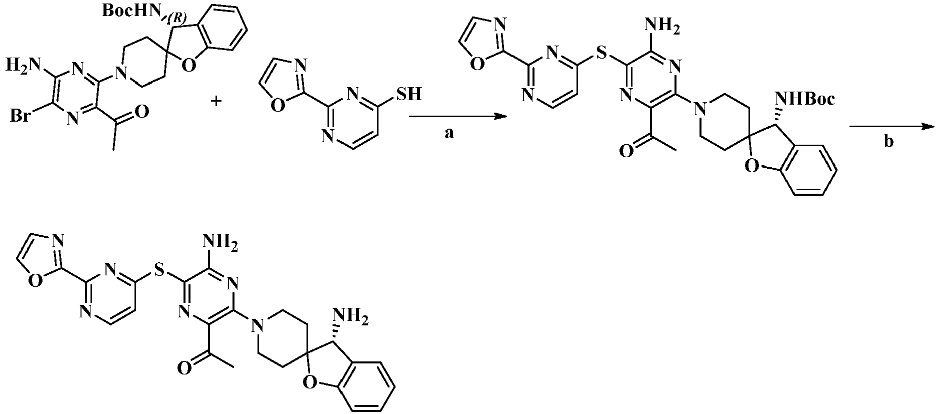

**Step a:** A solution of (R)-(1'-(3-acetyl-6-amino-5-bromopyrazine-2-yl)-3H-spiro[benzofuran-2,4'-piperidine]-3-yl)carbamic acid tert-butyl ester (140 mg, 0.27 mmol, 1.0eqv), 2-(oxazole-2-yl)pyrimidine-4-thiol (58mg, 0.325mmol, 1.2 eqv), DIPEA(123mg, 0.954 mmol, 3.5eqv), Pd₂(dba)₃ (50 mg, 0.054 mmol, 0.2eqv) and Xantphos (63mg, 0.109mmol, 0.4 eqv) in 1,4-dioxane (10mL) was reacted at 96 °C for 6 hours under the protection of nitrogen gas. After cooling down to room temperature, silica gel was added to the reaction solution. The resulting reaction system was purified by silica gel column chromatography (eluent: n-hexane:EA=10:1-1:2) to produce (R)-(1'-(3-acetyl-6-amino-5-((2-(oxazole-2-yl)pyrimidine-4-yl)sulfanyl)pyrazine-2-yl)-3H-spiro[benzofuran-2,4'-piperidine]-3-yl)carbamic acid tert-butyl ester (126mg, yield: 75.74%). (ESI, m/z):617.14[M+H]⁺.

**Step b:** To a solution of (R)-(1'-(3-acetyl-6-amino-5-((2-(oxazole-2-yl)pyrimidine-4-yl)sulfanyl)pyrazine-2-yl)-3H-spiro[benzofuran-2,4'-piperidine]-3-yl)carbamic acid tert-butyl ester (126mg) in dichloromethane (5ml) was added trifluoroacetic acid (5ml). The resulting mixture was stirred at room temperature for 3 hours. The reaction solution was concentrated under reduced pressure. To the residue were added EA (50ml) and water (50ml). The resulting mixture was adjusted with 25% aqueous ammonia to pH=10, and separated into two phases. The aqueous phase was extracted with EA (20ml). The organic phases were combined. The combined organic phase was washed with water (50ml), and brine (50ml), and concentrated under reduced pressure. The residue was purified by column chromatography (CH₂Cl₂:CH₃OH=20:1-15:1) to produce (R)-1-(5-amino-3-(3-amino-3H-spiro[benzofuran-2,4'-piperidine]-1'-yl)-6-((2-(oxazole-2-yl)pyrimidine-4-yl)sulfanyl)pyrazine-2-yl)ethanone (97mg, yield: 91.9%). (ESI, *m*/*z*):517.10[M+H]⁺.The preparation of Examples 25-55 was identical to the preparation of Examples 1-24, using 2,4-diiodopyrimidine and the corresponding Intermediates.

| Preparation Example No. | Compound Structure | Characterization Data (ESI, *m*/*z):* [M+H]⁺ | Preparation Example No. | Compound Structure | Characterization Data (ESI, *m*/*z):* [M+H]⁺ |
|---|---|---|---|---|---|
| TW-45 | 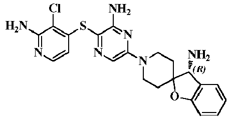 | 456.11 | 25 | 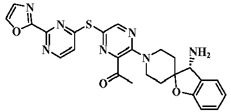 | 502.14 |
| 26 | 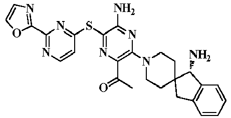 | 515.15 | 27 | 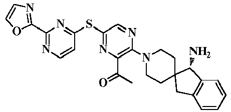 | 500.16 |
| 28 | 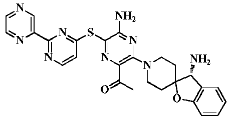 | 528.12 | 29 | 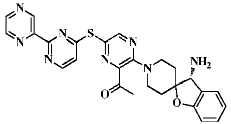 | 513.14 |
| 30 | 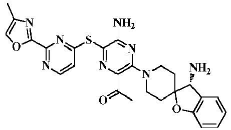 | 531.12 | 31 | 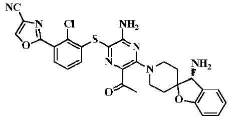 | 574.09 |
| 32 | 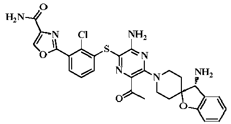 | 592.08 | 33 | 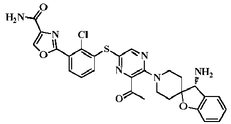 | 577.08 |
| 34 | 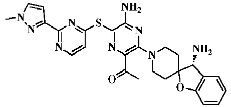 | 530.10 | 35 | 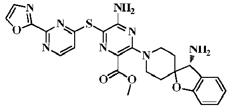 | 532.09 |
| 36 | 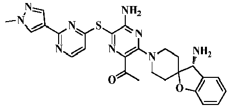 | 530.11 | 37 | 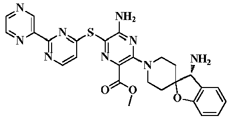 | 544.09 |
| 38 | 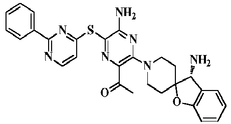 | 526.12 | 39 | 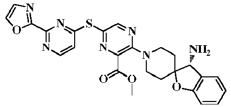 | 518.08 |
| 40 | 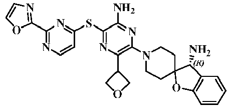 | 531.12 | 41 | 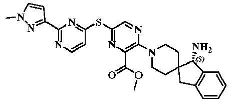 | 529.18 |
| 42 | 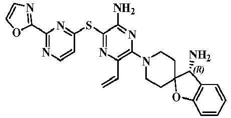 | 501.08 | 43 | 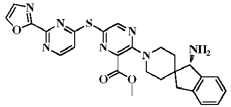 | 516.11 |
| 44 | 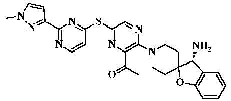 | 515.09 | 45 | 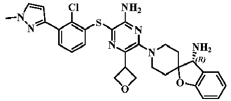 | 576.13 |
| 46 | 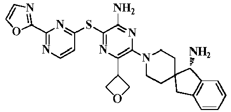 | 529.14 | 47 | 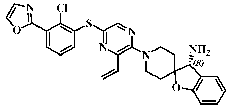 | 518.03 |
| 48 | 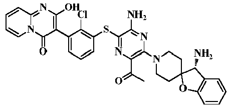 | 642.08 | 49 | 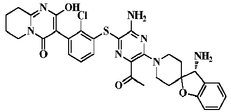 | 646.09 |
| 50 | 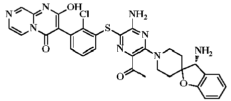 | 643.08 | 51 | 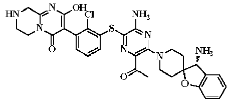 | 647.10 |
| 52 | 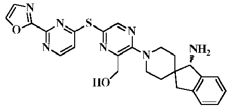 | 488.10 | 53 | 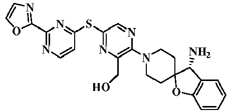 | 490.08 |
| 54 | 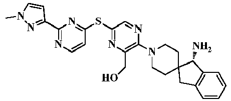 | 501.11 | 55 | 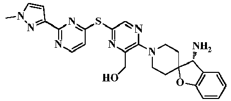 | 50309 |

### Preparation Example 56: (R)-1-(5-amino-6-((2-amino-3-chloropyridine-4-yl)sulfanyl)-3-(3-amino-3-deuterium -spiro[benzofuran-2,4'-piperidine]-1'-yl)pyrazine-2-yl)ethanone (Compound 56)

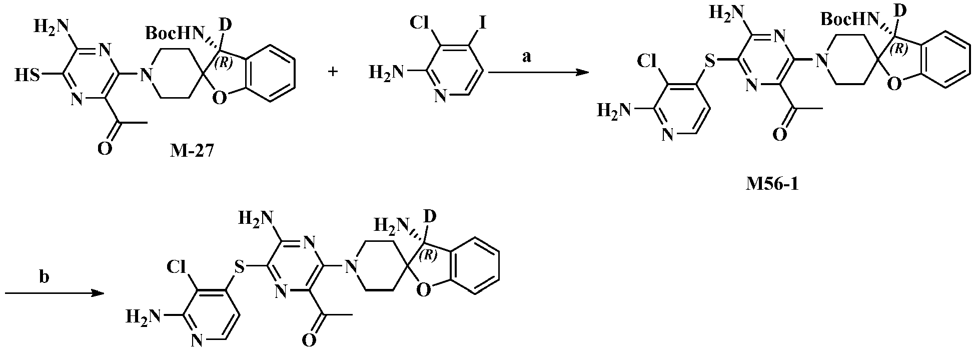

### Step a:

A solution of M-27 (256mg, 0.542mmol, 1.0eqv), 2-amino-3-chloro-4-iodopyridine (207mg, 0.813mmol, 1.5eqv), Pd₂(dba)₃ (99mg, 0.108mmol, 0.2eqv), Xantphos (125mg, 0.217mmol, 0.4eqv), and DIPEA (245mg, 1.897 mmol, 3.5eqv) in 1,4-dioxane (20ml) was reacted at 97°C overnight under the protection of nitrogen gas. The reaction solution was cooled down and concentrated to dryness. To the concentrated mixture was added water (30ml), and the reaction system was extracted with EA (20ml×2). The organic phases were combined. The combined organic phase was washed with H₂O (20ml), and brine (20ml), dried over anhydrous sodium sulfate, and filtered. The filtrate was concentrated under reduced pressure, and purified by silica gel column chromatography (eluent: n-hexane:EA=5:1-1:3) to produce M56-1 (300mg, yield: 92%), (ESI, m/z):599.17[M+H] ⁺.

**Step b:** To a solution of M56-1 (300mg, 0.501mmol, 1.0eqv) in DCM (5ml) was added CF₃COOH (5ml). The resulting mixture was reacted at room temperature for 2 hours, and then the reaction solution was rotary-dried. To the residue were added water (30ml) and EA (30ml). The resulting mixture was adjusted with 25% aqueous ammonia to pH=9-10, and separated into two phases. The aqueous phase was extracted with EA (20ml×2). The organic phases were combined. The combined organic phase was washed with H₂O (20ml), and brine (20ml), dried over anhydrous sodium sulfate, and filtered. The filtrate was concentrated under reduced pressure, and purified by silica gel column chromatography (eluent: CH₂Cl₂:CH₃OH=20:1-15:1) to produce (R)-1-(5-amino-6-((2-amino-3-chloropyridine-4-yl)sulfanyl)-3-(3-amino-3-deuterium-spiro[benzofuran-2,4'-piperidine]-1'-yl)pyrazine-2-yl)ethanone (200mg, yield: 80%) (ESI, m/z):499.10[M+H] ⁺. ¹H-NMR (600MHz, DMSO-d6) δ7.686 (d, J= 5.4Hz, 1H), 7.321 (d, J= 7.2Hz, 1H), 7.141 (t, J= 7.2 Hz, 1H), 7.081 (s, 2H), 6.862 (t, J= 7.2 Hz, 1H), 6.773 (d, J= 7.2Hz, 1H), 6.315 (s, 2H), 5.887 (d, J= 5.4Hz, 1H), 3.882-3.860 (m, 1H), 3.768-3.747 (m, 1H), 3.372-3.330 (m, 1H), 3.293-3.286 (m, 1H), 2.381 (s, 3H), 2.029-1.980 (m, 2H), 1.861-1.773 (m, 2H), 1.727-1.704 (m, 1H).

### Preparation Examples 56A and 57-59:

The preparation of Examples 56A and 57-59 was identical to the preparation of Example 56, using the corresponding intermediates as the starting materials.

| Preparation Example No. | Main Starting Materials | Compound Structure | Characterizati on Data |
|---|---|---|---|
| 56A | 2-amino-3-chloro-4-iodopyridine, Intermediate 28 (M-28) | 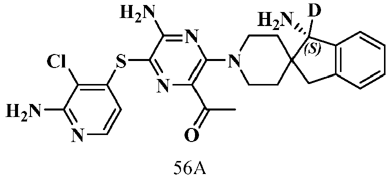 | (ESI, m/z): 497.11[M+H] ⁺ |
| 57 | 2-amino-4-bromopyrimidine, Intermediate 27 (M-27) | 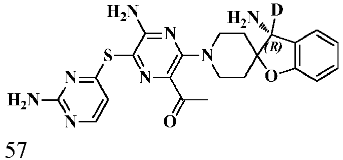 | (ESI, m/z): 466.11[M+H] ⁺ |
| 58 | 2-amino-3-chloro-4-iodopyridine, Intermediate 29 (M-29) | 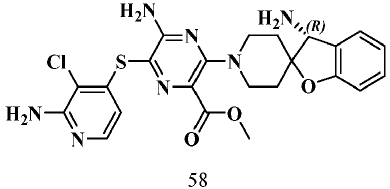 | (ESI, m/z): 514.09[M+H] ⁺ |
| 59 | 2-amino-3-chloro-4-iodopyridine, Intermediate 30 (M-30) | 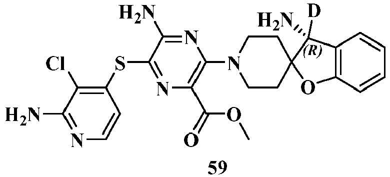 | (ESI, m/z): 515.10[M+H] ⁺ |

### Preparation Example 60: Preparation of Compound 60

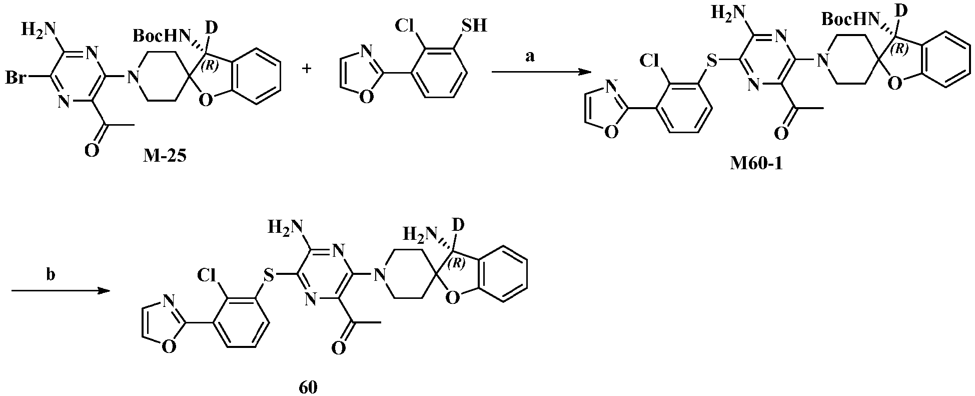

**Step a:** A solution of M-25 (840mg, 2.004mmol, 1.0eqv), 2-chloro-3-(oxazole-2-yl)benzenethiol (634mg, 3.006mmol, 1.5 eqv), DIPEA (906mg, 7.014 mmol, 3.5eqv), Pd₂(dba)₃ (367mg, 0.401mmol, 0.2eqv) and Xantphos (464mg, 0.802mmol, 0.4 eqv) in 1,4-dioxane (10mL) was reacted at 96°C overnight under the protection of nitrogen gas. After cooling down to room temperature, silica gel was added to the reaction solution. The reaction system was purified by silica gel column chromatography (eluent: n-hexane: EA=5:1-3:1) to produce M60-1 (600mg, yield: 46%). (ESI, m/z):650.12[M+H]⁺.

**Step b:** To a solution of M60-1 (600mg, 0.924mmol) in dichloromethane (15ml) was added trifluoroacetic acid (5ml). The resulting mixture was stirred at room temperature for 3 hours. The reaction solution was concentrated under reduced pressure. To the residue were added EA (50ml) and water (50ml). The resulting mixture was adjusted with 25% aqueous ammonia to pH=10, and separated into two phases. The aqueous phase was extracted with EA (20ml). The organic phases were combined. The combined organic phase was washed with water (50ml), and brine (50ml), and concentrated under reduced pressure. The residue was purified by column chromatography (CH₂Cl₂:CH₃OH=40:1-15:1) to produce Compound 60 (350mg, yield: 69%). (ESI, *m*/*z*):550.12[M+H]⁺.

### Preparation Example 61:

The preparation of Example 61 was identical to the preparation of Example 60, using the corresponding intermediates as the starting materials.

| Preparation Example No. | Main Starting Material | Compound Structure | Characterization Data |
|---|---|---|---|
| 61 | Intermediate 3, Intermediate 26 (M-26) | 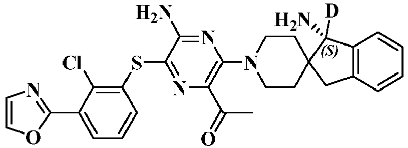 | (ESI, m/z): 548.10 [M+H]⁺, ¹H-NMR (600MHz, DMSO-d6) δ8.337 (s, 1H), 7.756 (d, J= 7.8Hz, 1H), 7.478 (s, 1H), 7.420 (t, J= 7.8 Hz, 1H), 7.375 (d, J= 7.2 Hz, 1H) 7.188 (t, J= 7.8 Hz, 1H), 7.131 (s, 1H), 7.075 (d, J= 7.8Hz, 1H), 6.895 (t, J= 7.2Hz, 1H), 6.819 (d, J= 7.8Hz, 1H), 3.896-3.874 (m, 1H), 3.787-3.764 (m, 1H), 3.413-3.337 (m, 1H), 3.299-3.280 (m, 1H), 2.317 (s, 3H), 2.071-2.022 (m, 1H), 1.876-1.820 (m, 2H), 1.755-1.717 (m, 1H), 1.393 (s, 3H). |

### Preparation Example 62:

The preparation of Example 62 was identical to the preparation of Example 60, using the corresponding intermediates as the starting materials.

| Preparation Example No. | Main Starting Material | Compound Structure | Characterization Data |
|---|---|---|---|
| 62 | 2-amino-3-chloro-4-iodopyridine, Intermediate 12 | 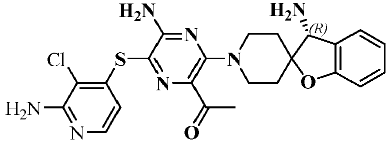 | (ESI, m/z): 498.12[M+H]^{+ 1}H-NMR (600MHz, DMSO-d6) δ7.685 (d, J= 5.4Hz, 1H), 7.339 (d, J= 7.2Hz, 1H), 7.157 (t, J= 7.2 Hz, 1H), 7.081 (s, 2H), 6.874 (t, J= 7.2 Hz, 1H), 6.789 (d, J= 7.2Hz, 1H), 6.314 (s, 2H), 5.886 (d, J=5.4Hz, 1H), 4.151 (m, 1H), 3.890-3.868 (m, 1H), 3.779-3.757 (m, 1H), 2.382 (s, 3H), 2.041-1.992 (m, 1H), 1.869-1.710 (m, 4H), 1.234 (s, 2H), 0.863-0.841 (m, 1H). |

| | | | |
|---|---|---|---|
| Note: Preparation of positive drug: Compound TW-45 (having the following structure) was prepared according to the preparation method of compound 45 in TW201840553A. | | | |

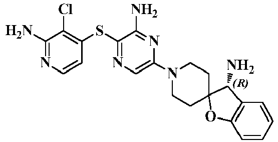

Biological assay of the compounds of the present invention is described below.

### Assay Example 1: Assay for the inhibitory effect on the SHP2 enzyme

### 1. Working buffer preparation and compound preparation

1x Assay buffer was prepared. A compound to be tested was diluted with DMSO solvent to a stock concentration of 10 mM. The compound to be tested was first diluted to an intermediate concentration of 1000 µM and then further diluted in a 3-fold gradient using DMSO. Therefore, the intermediate gradient dilution concentration of the compound to be tested was: 1000 µM, 3-fold dilution, 11 gradients. 100 nL of each concentration of the compound in the 384-well LDV echo plate was transferred to a new 384-well ELISA plate (assay plate) (coming, Cat # 4514) by the Echo550 instrument. The final concentration of the compound to be tested was 10 µM, 3-fold dilution, 11 gradients in duplicate after 10 µL of the reaction reagent was added. The final DMSO concentration was 1%. For both negative and positive control wells, 100 nL DMSO would be transferred.

### 2. SHP2 enzymatic reaction steps

A mixed liquor of 2X working SHP2 reagent (0.4 nM) and 0.5 µM SHP-2 activated peptide (BPS bioscience #79319-2) was prepared with 1X enzyme buffer. After the above-mixed liquor was incubated at 25°C for 60 minutes, 5 µL of activated SHP2 kinase solution was transferred with a pipettor to the compound wells and the Max control wells (Corning, #4514) of the plate. Then 5 µL of 1x kinase buffer was transferred to the Min control wells. The plate was centrifuged at 1000 rpm for 30 seconds, sealed, and incubated in a 25°C constant-temperature incubator for 30 minutes. A substrate solution of DiFMUP ( # D6567, Invitrogen^{™}) was prepared with 1x kinase buffer, and its concentration should be 2 times as the final concentration of the assay (DiFMUP final concentration: 10 µM). 5 µL of the prepared substrate solution was transferred with an electric pipettor into each well of the ELISA plate to start the reaction. The plate was centrifuged at 1000 rpm for 30 seconds, sealed, and incubated in a 25°C constant-temperature incubator for 60 minutes. Read the plate at the excitation/emission wavelength of 358/455 nm after the ELISA plate was placed on the Spark machine.

### 3. Calculation methods and assay results:

The RLU values were collected from Spark and the inhibition rate was calculated with the RLU values. Inhibition rate=(maximum value-sample RLU)/(maximum value-minimum value)* 100. "Minimum value" was referred to the RLU of the enzyme-free control, and "maximum value" was referred to the RLU of the DMSO control. The data were fit by using the XLFit Excel module (version 5.4.0.8) to obtain IC₅₀ values, and the results are shown in Table 1.

**Table 1: Enzymatic effect assay of the compounds of the present invention on SHP2**

| Preparation Example No. | Enzymatic Effect | Preparation Example No. | Enzymatic Effect | Preparation Example No. | Enzymatic Effect |
|---|---|---|---|---|---|
| 1 | A | 22 | A | 58 | A |
| 8 | A | 23 | A | 59 | A |
| 16 | A | 24 | A | 60 | A |
| 19 | A | 56 | A | 61 | A |
| 21 | A | 57 | A | 62 | A |

| | | | | | |
|---|---|---|---|---|---|
| Note: Compounds designated as "A" had IC₅₀ values of less than or equal to 20 nM. | | | | | |

The above data show that the compounds of the present application had a strong inhibitory effect on SHP2 kinase.

### Assay Example 2: In vitro inhibitory effect on the proliferation of human acute myeloid leukemia MV-4-11 cells

1. Assay material:
   The human acute myeloid leukemia MV-4-11 cells used in the assay were purchased from Shanghai Institutes for Biological Sciences, Chinese Academy of Sciences. The complete culture medium required for cell culture was IMDM (GIBCO Company), which was supplemented with 10% fetal bovine serum (GIBCO Company). Cells were cultured in a 37°C, 5% CO₂ incubator. The reagents used in the assay included dimethyl sulfoxide (purchased from Tianjin Kemiou Chemical Reagent Co., Ltd.), and MTT (Shanghai Topbiochem Co., Ltd., CAS. NO. 298-93-1). The test substance was sealed and stored at 4°C.
2. Assay method and result:
   Dimethyl sulfoxide was used as the solvent to fully dissolve the test substance and prepare a stock solution with a concentration of 5×10⁻² mol/L. The stock solution was stored at -20°C. A complete culture medium was used as the diluent, and the test substance was diluted to different concentrations in a gradient for later use. 100 µL/well (2×10⁴ cells/well) of a suspension of MV-4-11 cells in the complete culture medium was added to a 96-well culture plate. 100 µL/well of the corresponding test substances at different concentrations were added respectively. 8 concentrations were set for each test substance, and 3 duplicate wells were set for each concentration. The plate was cultured in a 37°C, 5% CO₂ incubator. 20 µL/well of MTT was added after 72 hours of exposure to the test substances. The plate was cultured for 4 hours in a 37°C, 5% CO₂ incubator. The supernatant was discarded, and 150 µL/well of dimethyl sulfoxide was added. The plate contents were oscillated and mixed evenly. The microplate reader was used to detect the OD value at a wavelength of 550 nm. The well only containing the cell suspension without test substance was a control well, and the well only containing the complete culture medium was a blank well. The inhibition rate of the cell growth was calculated with the following formula:
   Inhibition rate=(OD_{control well}-OD_{test well})/(OD_{control well}-OD_{blank well})* 100%. According to the inhibition rates at each concentration, SPSS software was used to calculate the half inhibitory concentration IC₅₀ value. The results are shown in Table 2.

**Table 2: Assay on the cell proliferation inhibitory effect of the compounds of the present invention**

| Preparation Example No. | human acute myeloid leukemia MV-4-11 cells (IC₅₀ nM) | Preparation Example No. | human acute myeloid leukemia MV-4-11 cells (IC₅₀ nM) |
|---|---|---|---|
| 1 | A | 56 | A |
| 8 | A | 57 | A |
| 16 | A | 59 | A |
| 19 | A | | |

| | | | |
|---|---|---|---|
| Note: Compounds designated as "A" had IC₅₀ values of less than or equal to 20 nM. | | | |

The above data showed that the compounds of the present invention showed good inhibitory activity in the assay on the inhibition of the human acute myeloid leukemia cell proliferation.

### Assay Example 3: In vitro inhibitory effect on the proliferation of human lung adenocarcinoma NCI-H441 cells

### 1. Assay material:

The human lung adenocarcinoma cell NCI-H441 used in the assay was purchased from ATCC. The complete culture medium required for cell culture was RPMI 1640 Medium (GIBCO Company), which was supplemented with 10% fetal bovine serum (GIBCO Company). Cells were cultured in a 37°C, 5% CO₂ incubator. The reagents used in the assay included dimethyl sulfoxide (purchased from Sigma), a 3D Cell Viability assay kit (purchased from Promega), and the control substance TW-45 used in the assay was obtained homemade. The test substance was sealed and stored at 4°C.

### 2. Assay method and result:

Dimethyl sulfoxide was used as the solvent to fully dissolve the test substance and prepare a stock solution with a concentration of 1×10⁻² mol/L. The stock solution was stored at -20°C. A complete culture medium was used as the diluent, and the test substance was diluted to different concentrations in gradient for later use (the final test concentration was 10000, 3333, 1111, 370, 123, 41, 13.7, 4.5, 1.5, and 0.5 nM). Cells were cultured in a 37°C, 5% CO₂ incubator for 5 days. Detection was made on the fifth day. 30 µL of reagent (Celltiter Glo Assay Kit-3D) was added to each well and the plate was shaken on a plate oscillator (protected from light) for 30 minutes. The plate was incubated at room temperature (protected from light) for 120 minutes. Chemiluminescence values were recorded with a Multiplate reader.

Data analysis: The stability inspection was performed using VC and PC data. Inhibition percentage=100-(Signal_{cmpd}-Signal_{Ave-PC})/(Signal_{Ave-VC}-Signal_{Ave-PC})×100. LUM: chemiluminescence signal of each well, VC: average chemiluminescence signal of high-quality control (the well containing 0.1% DMSO), PC:
average chemiluminescence signal of low-quality control (blank culture medium only).

GraphPad Prism 8 software was used to calculate the IC₅₀ of the compound and plot the effect-dose curve: Y=Bottom + (Top-Bottom) / (1+10^((LogIC₅₀-X)*HillSlope)). X: logarithm of cpd concentration; Y: inhibition percentage.

The results are shown in Table 3.

**Table 3: Assay on the NCI-H441 cell proliferation inhibitory effect of the compounds of the present invention**

| Preparation Example No. | Human lung adenocarcinoma cell NCI-H441(IC₅₀ nM) |
|---|---|
| 56 | A |
| 62 | B |

| | |
|---|---|
| Note: Compounds designated as "A" had IC₅₀ values of less than or equal to 20 nM, and Compounds designated as "B" had IC₅₀ values of greater than 20 nM and less than 50 nM. | |

The above data showed that the compounds of the present invention showed good inhibitory activity in the assay on the inhibition of the human lung adenocarcinoma cell proliferation.

### Assay Example 4: Mouse pharmacokinetic assay

### 1. Assay method:

ICR mice were intragastrically administered with the compound at 10 mg/kg or 20 mg/kg. At different time points (0.25 h, 0.5 h, 1 h, 2 h, 4 h, 8 h, 24 h) after administration, the mice blood was collected from the orbit. The collected whole blood was anticoagulated with heparin sodium and centrifuged at 3000 g to obtain mouse plasma samples. Methanol was added to precipitate proteins. The drug concentration in the mouse plasma after administration was determined by the HPLC-MS/MS method. A drug concentration-time curve was plotted and the pharmacokinetic parameters were calculated. The pharmacokinetic behavior in mice after the compound administration was described by means of non-compartment model statistical moment parameters.

### 2. Assay results:

The above-mentioned pharmacokinetic assay was conducted on the compound of Preparation Example 1 and the compound of Preparation Example 56 of the present disclosure. The assay results showed that the compounds of the present invention had relatively high exposure in mice and both were well absorbed in vivo.

**Table 4: Mouse pharmacokinetic assay results of the compounds of the present invention**

| Preparation Example No. | Dose (mg/kg) | Tₘₐₓ (h) | Cₘₐₓ (ng/mL) | AUC₀₋₂₄ₕ (h*ng/mL) |
|---|---|---|---|---|
| 1 | 20 | 4 | 2493 | 16928 |
| 56 | 10 | 0.5 | 3573 | 18258 |

### Assay Example 5: hERG assay

### 1. Assay method:

This assay included the following aspects:
A manual patch clamp technique was used to record the hERG current in the CHO-K1 cell line stably expressing the hERG channel;
The inhibition rate at each concentration was calculated based on the hERG tail current;
Each compound was tested at 5 concentrations to calculate the IC₅₀ value;
Three cells were tested for each concentration;
One positive control drug was set.

HERG currents were recorded using the whole-cell patch-clamp technique. The cell suspension was added to the cell tank and placed on the upright microscope stage. After the cell adherence, the cells were perfused with extracellular fluid at a flow rate of 1-2 mL/min. The glass microelectrode was drawn in two steps by a micropipette puller, and its resistance value in water was 2-5 MΩ. After establishing the whole-cell recording, the clamp potential was maintained at -80 mV. The hERG tail current was elicited by applying a depolarizing voltage to +60 mV upon the voltage stimulation and then repolarizing to -50 mV. All recordings were made after the current stabilization. Extracellular perfusion administration started from a low concentration, and each concentration lasted for 5-10 minutes until the current was stable, and then proceeded to the next concentration. The half inhibitory concentration (IC₅₀) of the test compound was derived from the best fit of the Logistic equation.

### 2. Assay results:

**Table 5: hERG IC₅₀ values of compounds recorded in the CHO-K1 stable cell line**

| Preparation Example No. | IC₅₀ (µM) |
|---|---|
| 1 | A |
| 56 | A |

| | |
|---|---|
| Note: Compounds designated as "A" had IC₅₀ values of greater than 10 µM. | |

The assay results showed that Compound No. 1 and Compound No. 56 of the present disclosure had no obvious inhibitory effect on the hERG channel within the detection concentration range of this assay, which could reflect to a certain extent that the compounds of the present invention had low cardiotoxicity, which has positive significance on drug safety assessment.

### Assay Example 6: Mouse tolerance assay

### Assay method:

Assay animals: ICR mice, female, 5 weeks old
Solvent: 2% DMSO + 98% (0.5% MC), treated by sufficient vortex and sonicated to obtain a uniform suspension
Assay method: Female ICR mice were divided into 4 groups according to weight balance, with 5 mice in each group. The administration doses were 50 mg/kg and 200 mg/kg respectively. The compound was administrated once daily for 7 consecutive days. The administration dosage and results are shown in the table below.
Assay results:

**Table 6: Mouse tolerance assay results of the compound of the present invention (7 days)**

| Preparation Example No. | Dose | Number of animals (number of deaths/total number) |
|---|---|---|
| 56 | 50 mg/kg | 0/5F |
| | 200 mg/kg | 0/5F |
| Positive control compound TW-45 | 50 mg/kg | 0/5F |
| | 200 mg/kg | 5F/5F |

The assay results showed that no death appeared after 7 days of administration of the compound No. 56 of the present invention at 50 mg/kg and 200 mg/kg; while death appeared after 4 days of administration of the positive control compound TW-45 at 200 mg/kg, and all animals of the positive control had died by the 7th day. The mouse maximum tolerated dose MTD was >200 mg/kg for No. 56, and <200 mg/kg for TW-45. It could be seen that the tolerance of compound No. 56 of the present invention in mice was significantly better than that of the positive control compound TW-45.

## Claims

1. A compound represented by formula (I) or a prodrug, tautomer, stereoisomer, solvate, isotope derivative, or pharmaceutically acceptable salt thereof, which compound has the following structure: 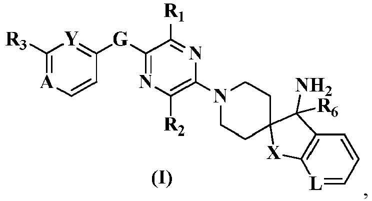 wherein,
X is selected from O, CH₂, NH, and S;
L is N or CH;
G is a bond or S;
A is CH or N;
R₃ is independently selected from optionally substituted 8-12 membered heterocyclyl, optionally substituted C₆₋₁₀ aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, - COOCH₃, -CONH₂, C₁₋₆ alkyl, C₁₋₆ alkoxyl, C₃₋₁₀ cycloalkyl, 3-10 membered heterocycloalkyl, C₆₋₁₀ aryl, and 5-12 membered heteroaryl;
R₁ is independently selected from hydrogen, deuterium, and amino;
R₂ is independently selected from hydrogen, halogen, cyano, -CONH₂, -CORs, -COORs, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl; said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two hydrogens on the same substitution site are replaced by the same oxygen to form a double bond;
the heteroatoms in the heterocycloalkyl, heteroaryl, and heterocyclyl are independently selected from O, N, and S, and the number of heteroatoms is 1, 2, 3, or 4;
wherein the compound of formula (I) does not include the following compounds: 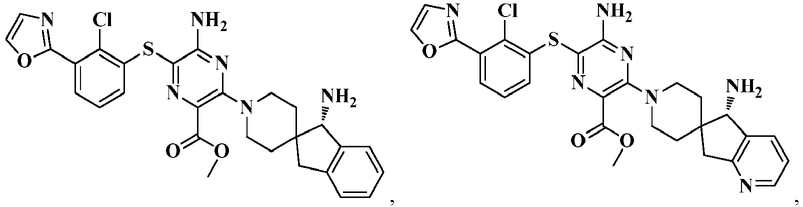 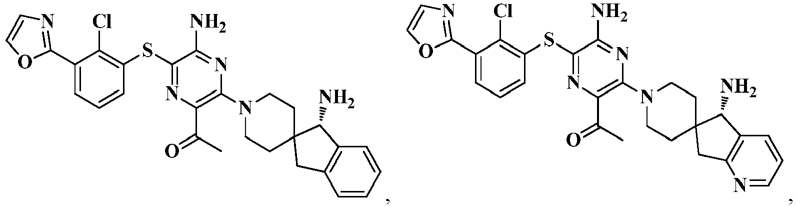 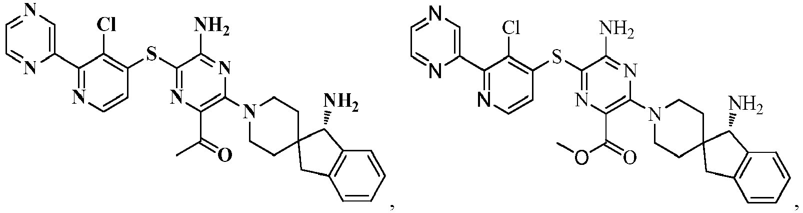 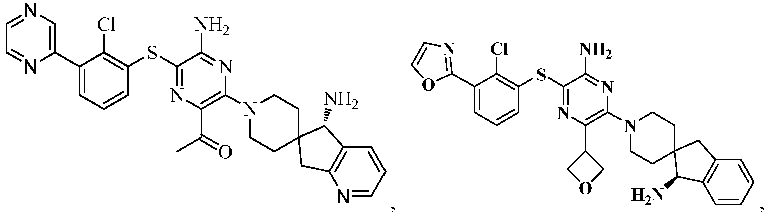 and

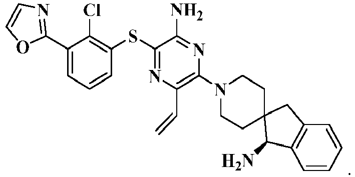

2. A compound represented by formula (I) or a prodrug, tautomer, stereoisomer, solvate, isotope derivative, or pharmaceutically acceptable salt thereof, which compound has the following structure: 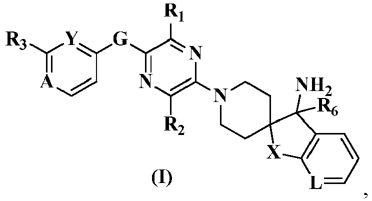 wherein,
X is selected from O, CH₂, NH, and S;
L is N or CH;
G is a bond or S;
A is CH or N;
R₃ is amino;
R₁ is independently selected from hydrogen, amino, and deuterium;
R₂ is independently selected from halogen, cyano, -CONH₂, -COR₅, -COOR₅, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two H's on the same substitution site are replaced by the same O to form a double bond;
the heteroatoms in the heterocycloalkyl, heteroaryl, and heterocyclyl are independently selected from O, N, and S, and the number of heteroatoms is 1, 2, 3, or 4;
wherein the compound of formula (I) does not include the following compounds:

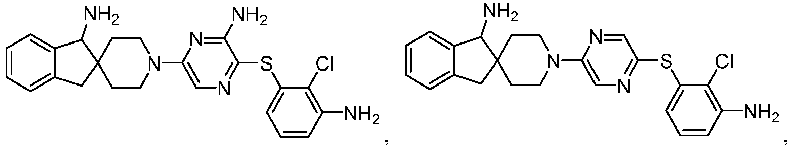

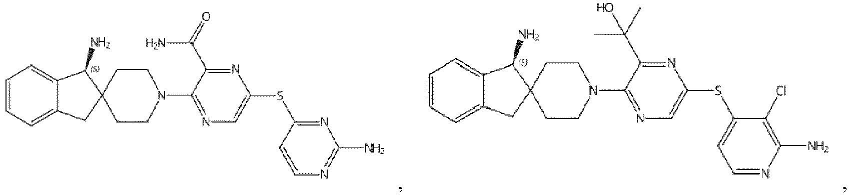

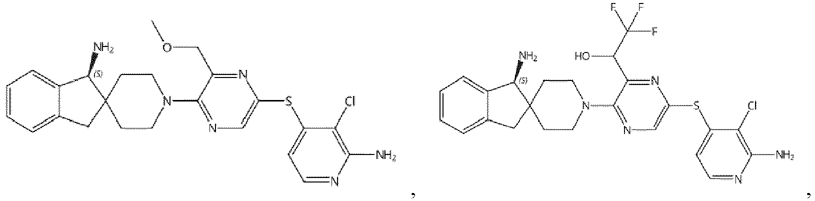

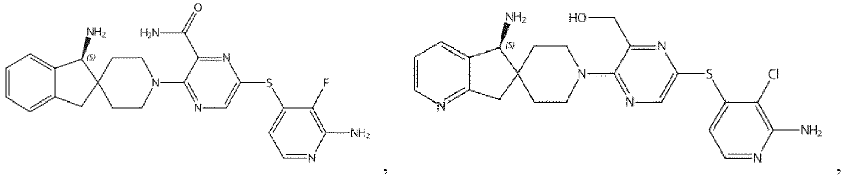

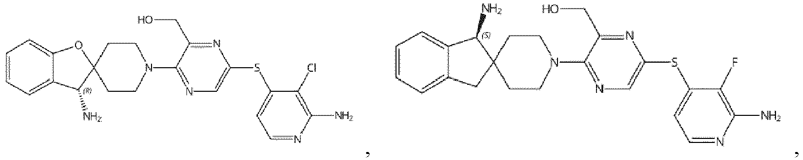

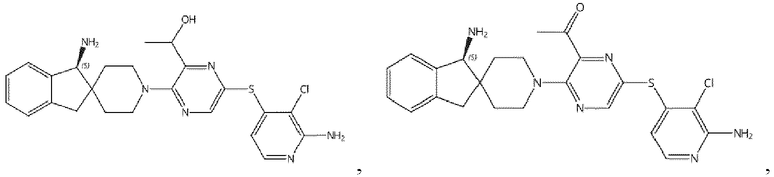

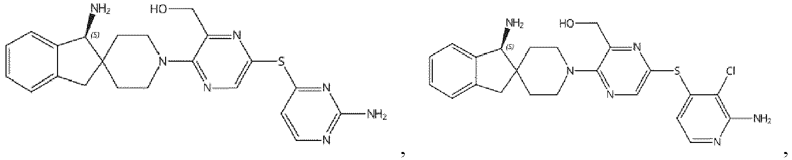

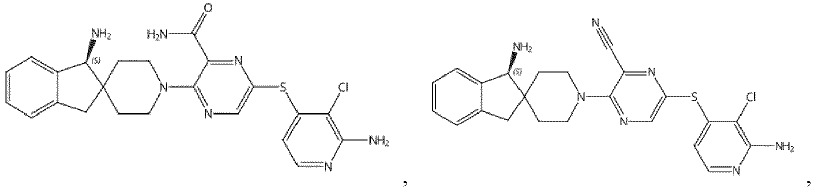

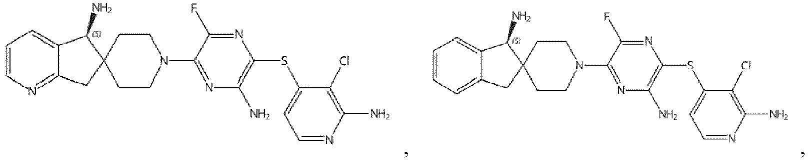

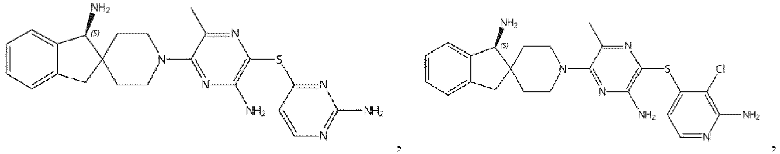

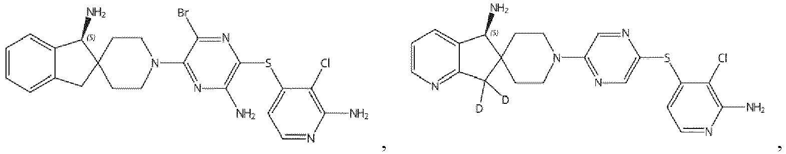

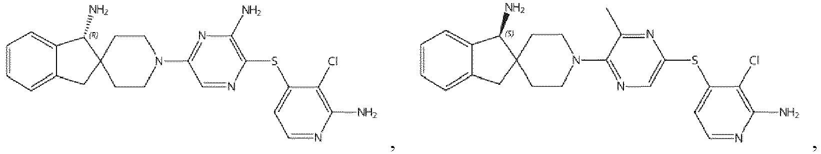

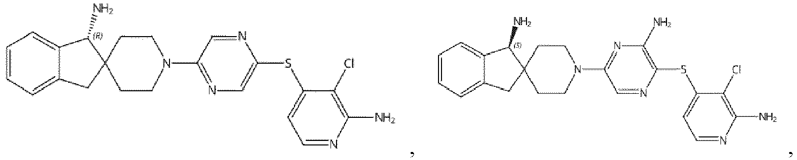

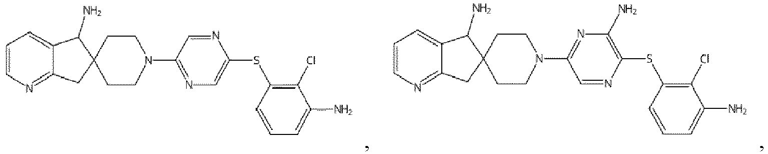

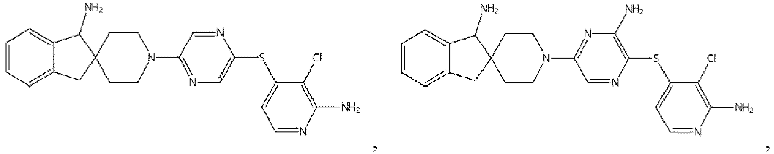

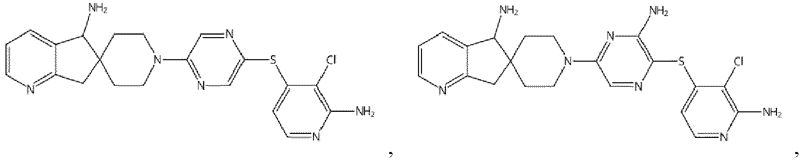

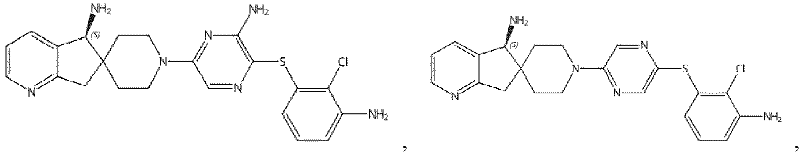

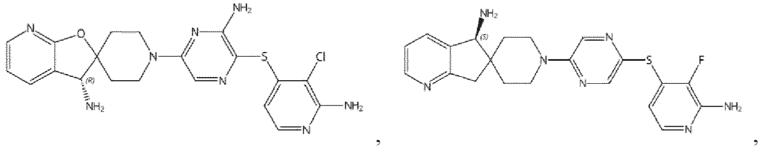

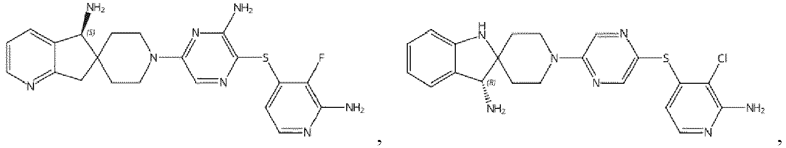

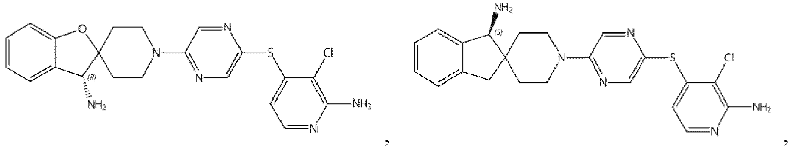

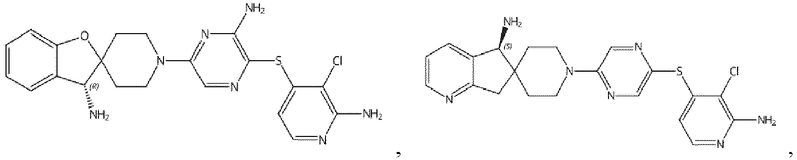

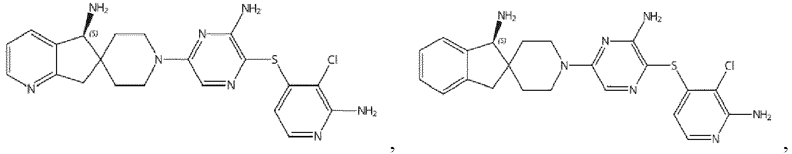

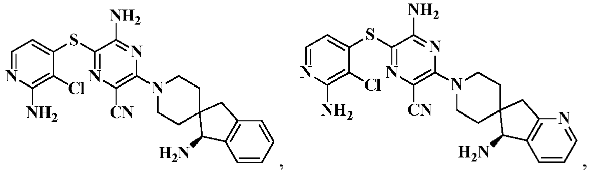

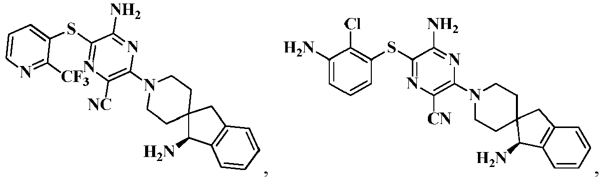

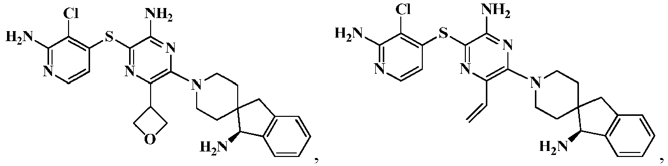

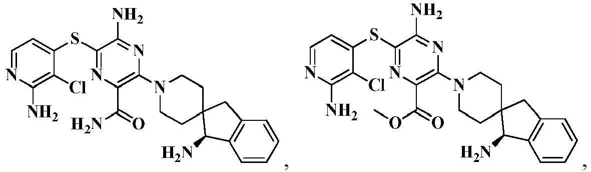

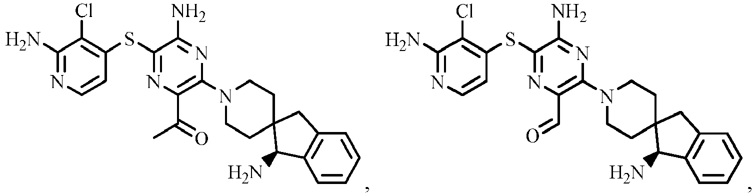

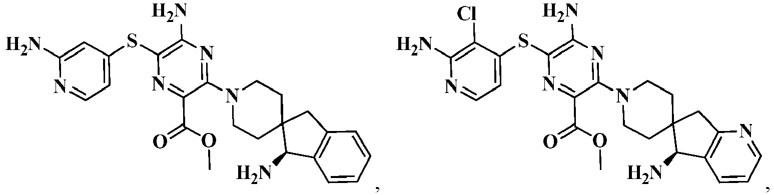

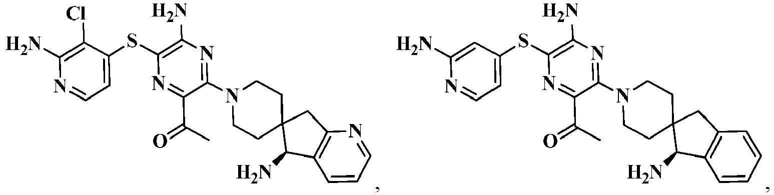

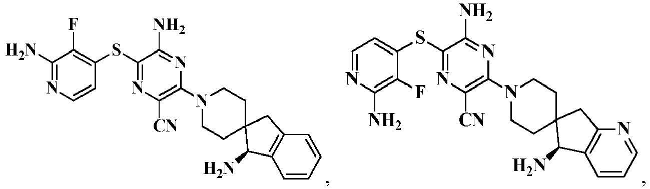

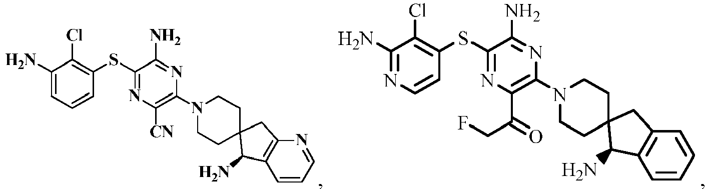

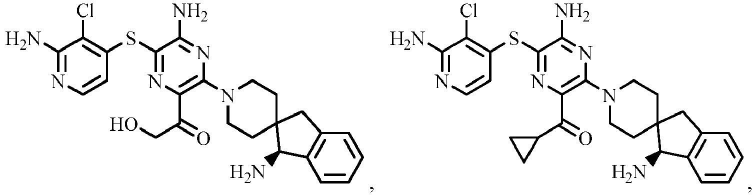

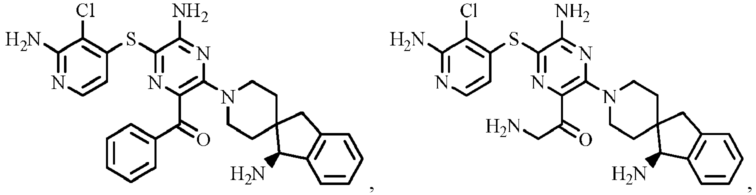

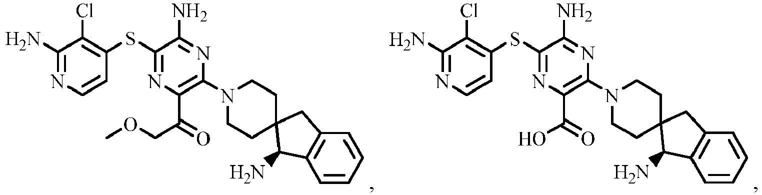

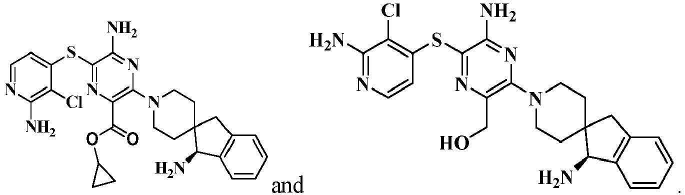

3. A compound represented by formula (I), or a prodrug, tautomer, stereoisomer, solvate, isotope derivative, or pharmaceutically acceptable salt thereof, which compound has the following structure: 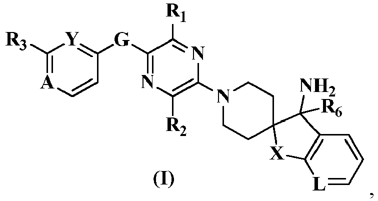 wherein,
X is selected from O, CH₂, NH, and S;
L is N or CH;
G is a bond or S;
A is CH or N;
R₃ is independently selected from amino, optionally substituted 8-12 membered heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, -COOH, -COCH₃, - COOCH₃, -CONH₂, C₁₋₆alkyl, C₁₋₆alkoxyl, C₃₋₁₀cycloalkyl, 3-10 membered heterocycloalkyl, C₆₋₁₀aryl, and 5-12 membered heteroaryl;
R₁ is independently selected from hydrogen, deuterium, and amino;
R₂ is independently selected from hydrogen, halogen, cyano, -CONH₂, -COR₅, -COOR₅, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
Y is independently selected from N and CR₄;
R₄ is independently selected from hydrogen, halogen, hydroxy, amino, cyano, optionally substituted C₁₋₆alkyl, and optionally substituted C₁₋₆alkoxyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, C₁₋₆alkyl, and C₁₋₆alkoxyl;
R₆ is independently selected from hydrogen and deuterium;
said "oxo" group means that two H's on the same substitution site are replaced by the same O to form a double bond;
the heteroatoms in the heterocycloalkyl, heteroaryl, and heterocyclyl are independently selected from O, N, and S, and the number of heteroatoms is 1, 2, 3, or 4;
wherein the compound represented by formula (I) does not include the following compounds:

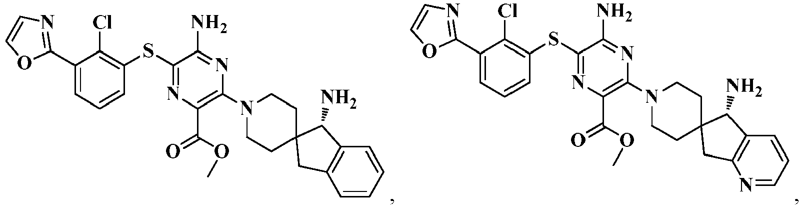

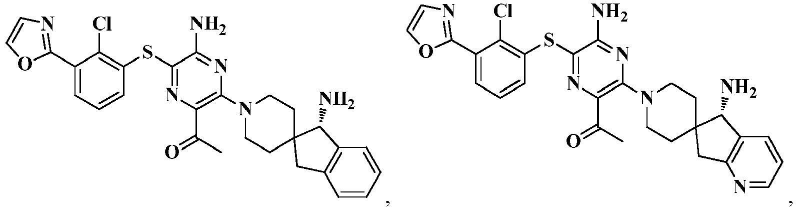

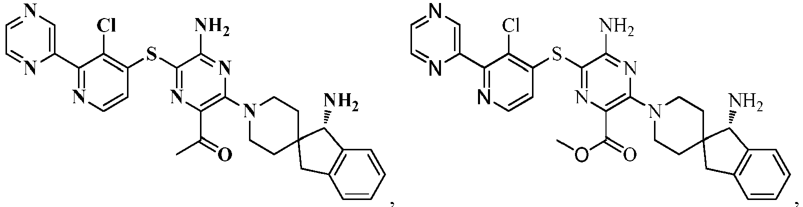

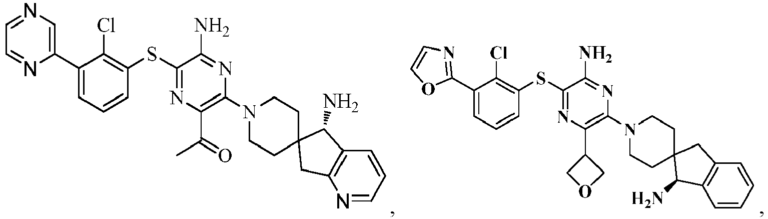

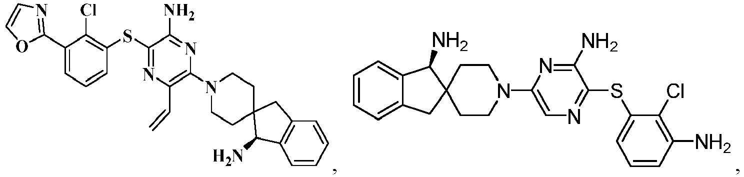

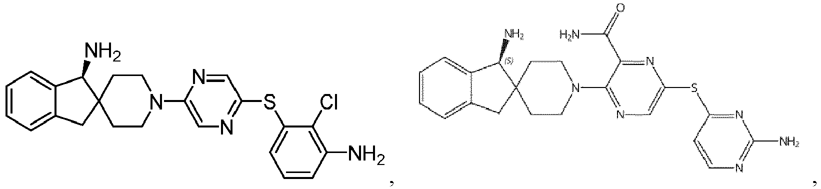

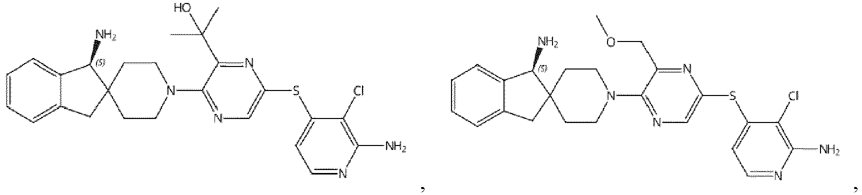

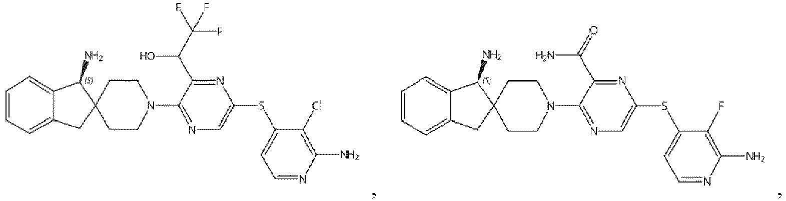

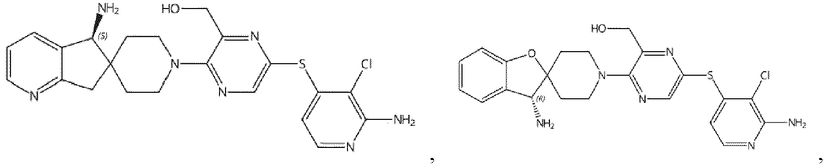

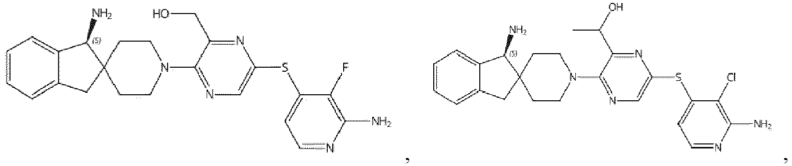

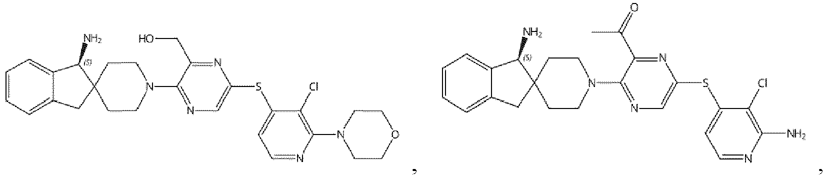

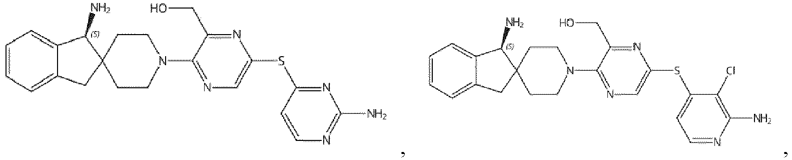

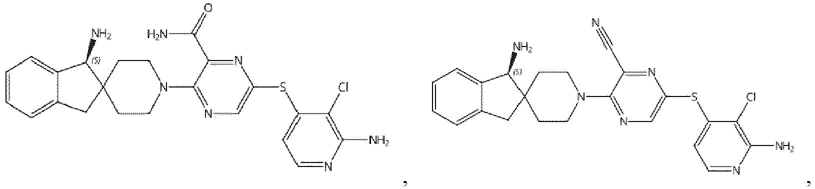

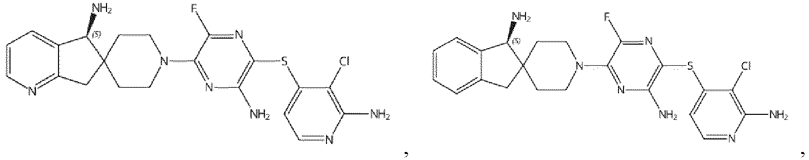

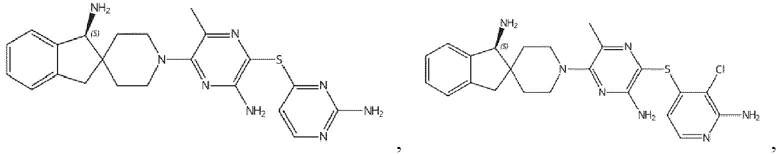

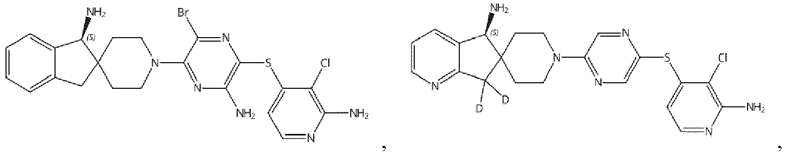

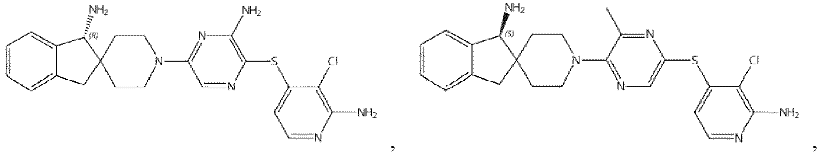

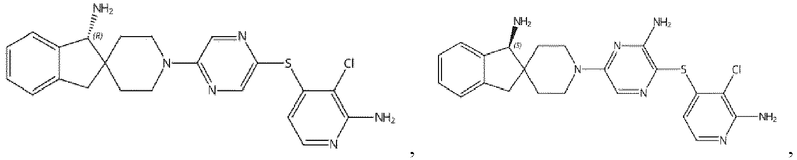

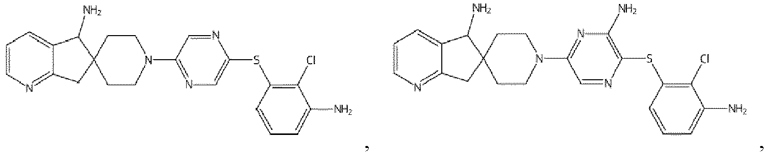

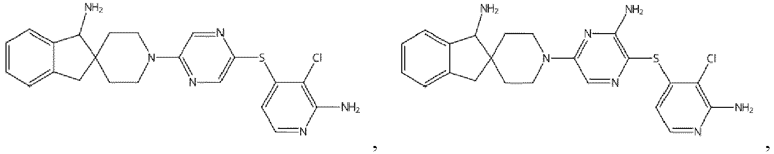

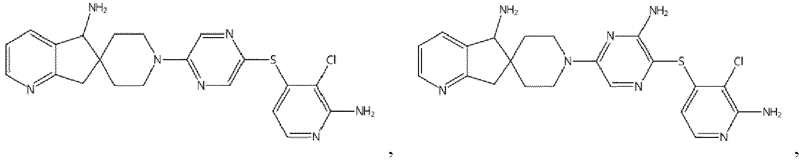

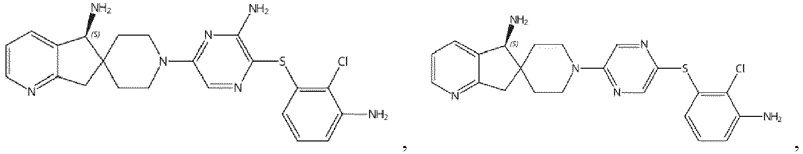

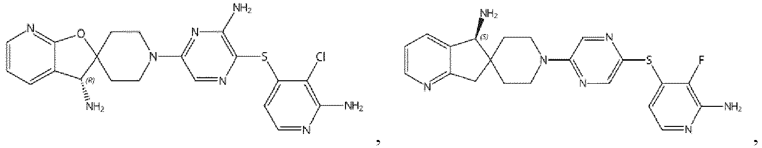

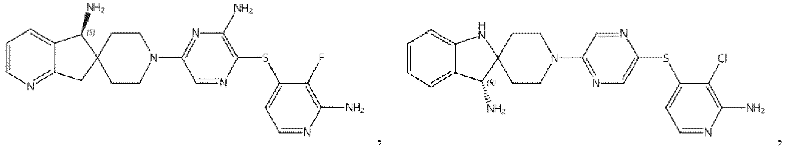

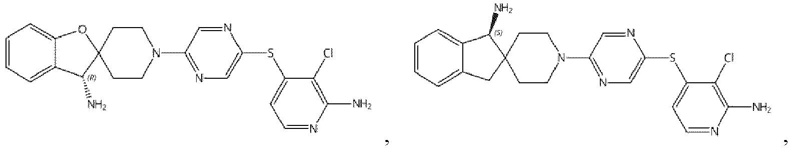

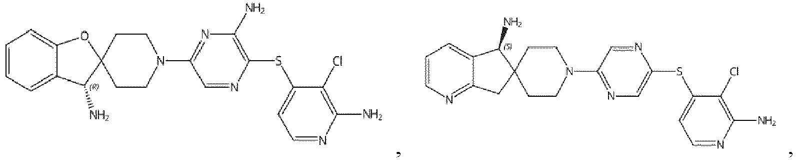

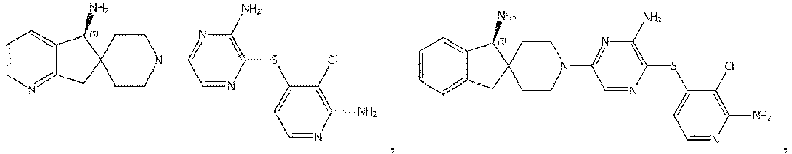

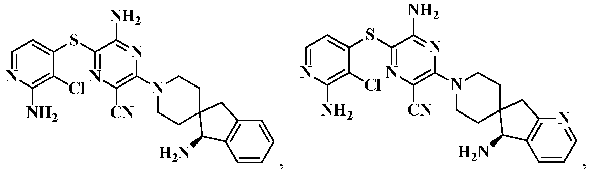

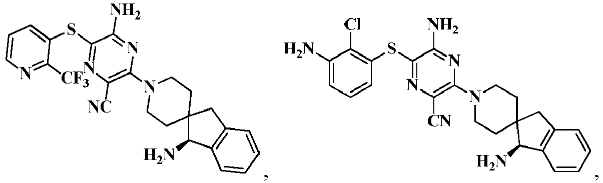

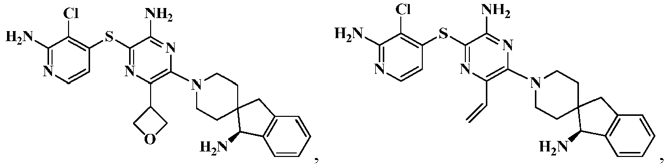

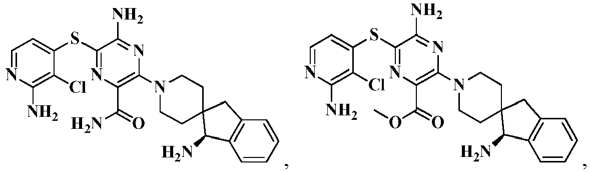

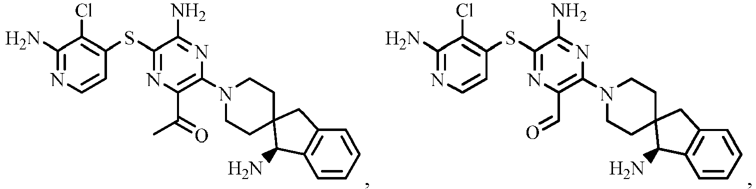

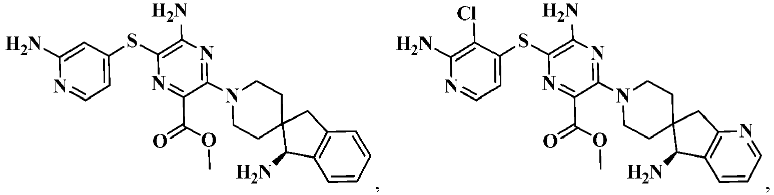

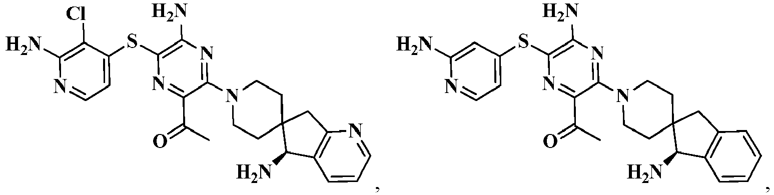

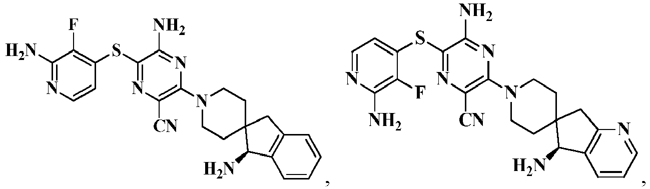

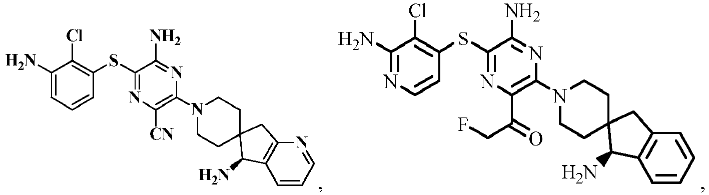

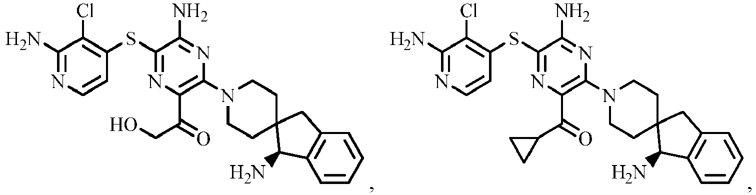

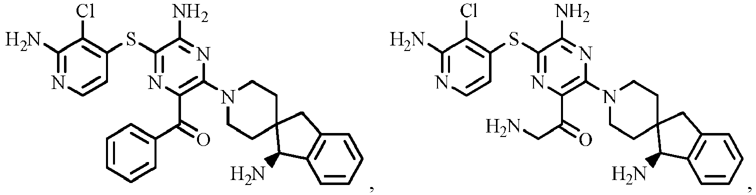

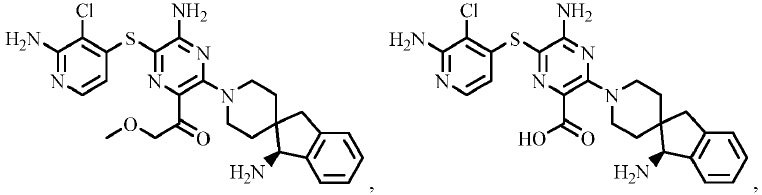

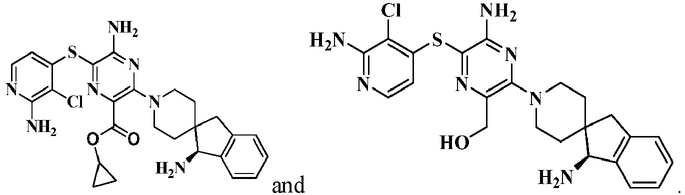

4. The compound according to claim 1 or 3 or a prodrug, tautomer, stereoisomer, solvate, isotope derivative, or pharmaceutically acceptable salt thereof, wherein the compound has a structure as represented by formula (I-1): 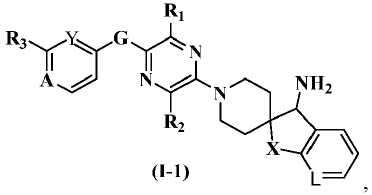 wherein,
X, L, G, A, R₃, R₁, R₂, Y, and R₄ are as defined in claim 1 or 3.

5. The compound according to claim 1 or 3 or a prodrug, tautomer, stereoisomer, solvate, isotope derivative, or pharmaceutically acceptable salt thereof, wherein R₆ is deuterium.

6. The compound according to any one of claims 1 and 3-5 or a prodrug, tautomer, stereoisomer, solvate, isotope derivative, or pharmaceutically acceptable salt thereof, wherein R₃ is independently selected from optionally substituted 8-12membered bicyclic heterocyclyl, optionally substituted C₆₋₁₀aryl, and optionally substituted 5-14 membered heteroaryl; or
R₃ is independently selected from optionally substituted 8-10membered bicyclic heterocyclyl, optionally substituted C₆₋₈aryl, and optionally substituted 5-10 membered heteroaryl; or
R₃ is independently selected from optionally substituted 8-10membered bicyclic heterocyclyl, optionally substituted C₆₋₈aryl, and optionally substituted 5-10 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, oxo, cyano, -CONH₂, and C₁₋₆alkyl; or
R₃ is independently selected from optionally substituted C₆₋₈aryl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, oxo, cyano, -CONH₂, and C₁₋₆alkyl; or
R₃ is independently selected from optionally substituted C₆aryl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, - CONH₂, and C₁₋₆alkyl (e.g., methyl); or
R₃ is independently selected from optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, cyano, -CONH₂, and C₁₋₃alkyl (e.g., methyl), wherein the heteroatoms in the heteroaryl are independently selected from O, N, and S, and the number of heteroatoms is 1 or 2.

7. The compound according to any one of claims 1 and 3-6 or a prodrug, tautomer, stereoisomer, solvate, isotope derivative, or pharmaceutically acceptable salt thereof, wherein R₂ is independently selected from hydrogen, halogen, cyano, -CONH₂, -COR₅, -COOR₅, optionally substituted C₁₋₆alkyl, optionally substituted C₂₋₆alkenyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from halogen, cyano, -CONH₂, -CORs, -COORs, hydroxy-substituted C₁₋₄alkyl, C₂₋₆alkenyl, C₃₋₆cycloalkyl, and 3-6 membered heterocycloalkyl; or
R₂ is independently selected from halogen, cyano, -CONH₂, -CORs, -COORs, C₁₋₄alkyl that is substituted by one or more substituents selected from hydroxy, C₁₋₆alkoxyl and halogen, C₂₋₆alkenyl, C₃₋₆cycloalkyl, and 3-6 membered heterocycloalkyl; or
R₂ is independently selected from halogen, -CORs, -COORs, hydroxy-substituted C₁₋₄alkyl, C₂₋₆alkenyl and 3-6 membered heterocycloalkyl; or
R₂ is independently selected from -CORs and -COORs; or
R₂ is independently selected from -COR₅.

8. The compound according to any one of claims 1-7 or a prodrug, tautomer, stereoisomer, solvate, isotope derivative, or pharmaceutically acceptable salt thereof, wherein R₁ is independently selected from hydrogen and amino; preferably, R₁ is amino.

9. The compound according to claim 2 or 3 or a prodrug, tautomer, stereoisomer, solvate, isotope derivative, or pharmaceutically acceptable salt thereof, wherein
when R₁ is amino,
R₂ is independently selected from cyano, -CONH₂, -COR₅, -COOR₅, unsubstituted C₂₋₆alkyl, substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from cyano, -CONH₂, -CORs, -COORs, substituted C₁₋₆alkyl, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from cyano, -CONH₂, -CORs, -COORs, C₁₋₆alkyl that is substituted by one or more substituents selected from hydroxy, halogen, and C₁₋₃alkoxyl, C₂₋₆alkenyl, C₃₋₆cycloalkyl, and 3-6 membered heterocycloalkyl; or
R₂ is independently selected from -CORs; and
when R₁ is deuterium,
R₂ is independently selected from halogen, cyano, -CONH₂, -CORs, -COORs, unsubstituted C₂₋₆alkyl, substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from halogen, cyano, -CONH₂, -CORs, -COORs, substituted C₁₋₆alkyl, C₂₋₆alkenyl, C₃₋₆cycloalkyl, and 3-6 membered heterocycloalkyl; or
R₂ is selected from -CO-(C₃₋₆cycloalkyl), -COOR₅, optionally substituted C₂₋₆alkenyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; and
when R₁ is hydrogen,
R₂ is independently selected from halogen, -CO-(C₃-C₆cycloalkyl), -COORs, optionally substituted C₂₋₆alkenyl, optionally substituted C₂₋₆alkynyl, optionally substituted C₃₋₁₀cycloalkyl, and optionally substituted 3-10 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₂ is independently selected from halogen, -CO-(C₃-C₆cycloalkyl), -COORs, C₂₋₆alkenyl, C₃₋₆cycloalkyl, and 3-6 membered heterocycloalkyl;
preferably, R₆ is deuterium.

10. The compound according to claim 2 or a prodrug, tautomer, stereoisomer, solvate, isotope derivative, or pharmaceutically acceptable salt thereof, wherein the compound has a structure as represented by formula (I-2): 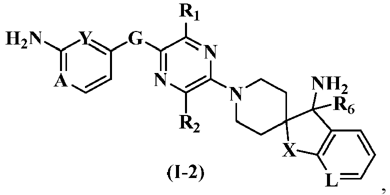
wherein R₁ is independently selected from deuterium and amino, preferably amino;
R₂ is independently selected from cyano, -CONH₂, -COR₅, -COOR₅, optionally substituted C₁₋₆alkyl, optionally substituted C₂₋₆alkenyl, optionally substituted C₂₋₆alkynyl, optionally substituted C₃₋₁₀cycloalkyl, and optionally substituted 3-10 membered heterocycloalkyl, wherein R₅ is independently selected from hydrogen, optionally substituted C₁₋₆alkyl, optionally substituted C₃₋₆cycloalkyl, optionally substituted 3-6 membered heterocycloalkyl, optionally substituted C₆₋₁₀aryl, and optionally substituted 5-10 membered heteroaryl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl;
R₆ is hydrogen or deuterium, preferably deuterium;
X is selected from O, CH₂, NH and S, preferably O;
L, G, A, and Y are as defined in claim 2.

11. The compound according to any of claims 1-10 or a prodrug, tautomer, stereoisomer, solvate, isotope derivative, or pharmaceutically acceptable salt thereof, wherein R₅ is independently selected from hydrogen, optionally substituted C₁₋₆alkyl, optionally substituted C₃₋₆cycloalkyl, and optionally substituted 3-6 membered heterocycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, amino, nitro, oxo, cyano, C₁₋₆alkyl, and C₁₋₆alkoxyl; or
R₅ is independently selected from hydrogen, C₁₋₆alkyl, C₃₋₆cycloalkyl, and 3-6 membered heterocycloalkyl; or
R₅ is independently selected from C₁₋₃alkyl and C₃₋₆cycloalkyl; or
R₅ is C₁₋₃alkyl; or
R₅ is methyl.

12. The compound according to any of claims 1-11 or a prodrug, tautomer, stereoisomer, solvate, isotope derivative, or pharmaceutically acceptable salt thereof, wherein X is O or CH₂; or X is O.

13. The compound according to any of claims 1-12 or a prodrug, tautomer, stereoisomer, solvate, isotope derivative, or pharmaceutically acceptable salt thereof, wherein the compound has a structure as represented by formula (I-A), (I-B), (I-1-A), (I-1-B), (I-2-A) or (I-2-B): 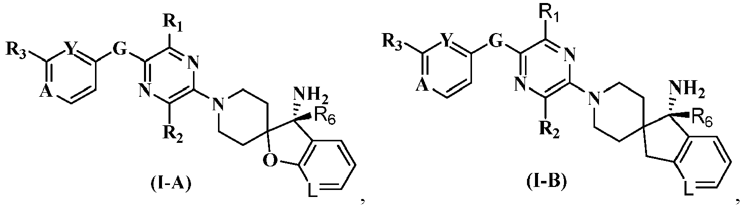 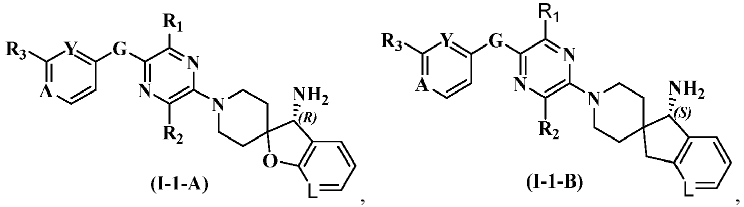 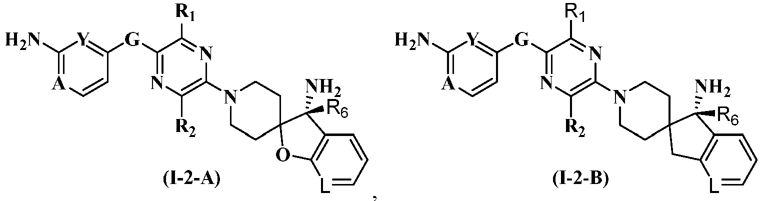 wherein L, G, A, Y, R₁, R₂, R₃, and R₆ are as defined in any of claims 1-9.

14. The compound according to any of claims 1-13 or a prodrug, tautomer, stereoisomer, solvate, isotope derivative, or pharmaceutically acceptable salt thereof, wherein L is CH.

15. The compound according to any of claims 1-14 or a prodrug, tautomer, stereoisomer, solvate, isotope derivative, or pharmaceutically acceptable salt thereof, wherein G is S.

16. The compound according to any of claims 1-15 or a prodrug, tautomer, stereoisomer, solvate, isotope derivative, or pharmaceutically acceptable salt thereof, wherein A is CH; or A is N and Y is N, or A is CH or N and Y is CR₄.

17. The compound according to any of claims 1-16 or a prodrug, tautomer, stereoisomer, solvate, isotope derivative, or pharmaceutically acceptable salt thereof, wherein R₄ is independently selected from hydrogen, halogen, hydroxy, amino, cyano, optionally substituted C₁₋₆alkyl, and optionally substituted C₁₋₆alkoxyl; said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and amino; or
R₄ is independently selected from hydrogen, halogen, amino, cyano, C₁₋₆alkyl, C₁₋₆alkoxyl, and C₁₋₆alkylamino; or
R₄ is independently selected from hydrogen, fluoro, chloro, amino, cyano, methyl, and methoxy; or
R₄ is chloro.

18. The compound according to claim 1 or 3 or a prodrug, tautomer, stereoisomer, solvate, isotope derivative, or pharmaceutically acceptable salt thereof, wherein X is O or CH₂; L is CH; G is S; A is CH or N; R₃ is independently selected from optionally substituted 8-12 membered heterocyclyl, optionally substituted C₆₋₁₀aryl, and optionally substituted 5-14 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, -CONH₂ and C₁₋₆alkyl; R₁ is independently selected from hydrogen, deuterium, and amino; R₂ is independently selected from halogen, -CORs, -COOR₅, optionally substituted C₁₋₆alkyl, optionally substituted C₂₋₆alkenyl, and optionally substituted 3-10 membered heterocycloalkyl, wherein R₅ is independently selected from unsubstituted C₁₋₆alkyl and unsubstituted C₃₋₆cycloalkyl, wherein said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from N and CR₄; R₄ is halogen; R₆ is independently selected from hydrogen, and deuterium; or
X is O or CH₂; L is CH; G is S; A is CH or N; R₃ is independently selected from optionally substituted 8-10membered bicyclic heterocyclyl, optionally substituted C₆ or C₁₀aryl, and optionally substituted 5-6 membered heteroaryl, wherein said "optionally substituted" means that the hydrogen on the group to be substituted is unsubstituted or the hydrogen(s) on one or more substitution sites of the group to be substituted is/are independently substituted by a substituent selected from a group consisting of hydroxy, oxo, cyano, -CONH₂ and C₁₋₆alkyl; R₁ is independently selected from hydrogen, deuterium, and amino; R₂ is independently selected from halogen, -COR₅, -COOR₅, optionally substituted C₁₋₆alkyl, optionally substituted C₂₋₆alkenyl, and optionally substituted 3-6 membered heterocycloalkyl, wherein R₅ is independently selected from unsubstituted C₁₋₆alkyl and unsubstituted C₃₋₆cycloalkyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C ₁₋₆alkoxyl; Y is independently selected from N and CR₄; R₄ is halogen; R₆ is independently selected from hydrogen, and deuterium; or
X is O or CH₂; L is CH; G is S; A is CH or N; R₃ is independently selected from 5-6 membered heteroaryl that is optionally substituted by C₁₋₆alkyl; R₁ is independently selected from hydrogen, deuterium, and amino; R₂ is independently selected from halogen, -CORs, -COORs, optionally substituted C₂₋₆alkenyl, and optionally substituted 3-6 membered heterocycloalkyl, wherein R₅ is independently selected from unsubstituted C₁₋₆alkyl and unsubstituted C₃₋₆cycloalkyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from N and CR₄; R₄ is halogen; R₆ is independently selected from hydrogen and deuterium; or
X is O or CH₂; L is CH; G is S; A is CH or N; R₃ is independently selected from 5-6 membered heteroaryl that is optionally substituted by C₁₋₆alkyl; R₁ is independently selected from hydrogen, deuterium, and amino; R₂ is independently selected from halogen, -CORs, -COORs, unsubstituted C₂₋₆alkenyl, and unsubstituted 3-6 membered heterocycloalkyl, wherein R₅ is independently selected from unsubstituted C₁₋₆alkyl and unsubstituted C₃₋₆cycloalkyl; Y is independently selected from N and CR₄; R₄ is halogen; R₆ is independently selected from hydrogen, and deuterium; or
X is O or CH₂; L is CH; G is S; A is CH or N; R₃ is independently selected from 5-membered heteroaryl that is optionally substituted by C₁₋₄alkyl; R₁ is independently selected from hydrogen, deuterium, and amino; R₂ is independently selected from -CORs and -COORs, wherein R₅ is independently selected from unsubstituted C₁₋₄alkyl and unsubstituted C₃₋₆cycloalkyl; Y is independently selected from N and CR₄; R₄ is halogen, for example, Cl or F, preferably Cl; R₆ is independently selected from hydrogen and deuterium; or
X is O or CH₂; L is CH; G is S; when A is CH: Y is CR₄; R₄ is halogen, for example, Cl or F, preferably Cl; or when A is N: Y is independently selected from and CR₄; R₄ is halogen, for example, Cl or F, preferably Cl; R₃ is independently selected from 5 membered heteroaryl that is optionally substituted by C ₁₋₂alkyl (e.g., methyl); R₁ is independently selected from hydrogen, deuterium, and amino; R₂ is independently selected from -COR₅ and -COOR₅, wherein R₅ is independently selected from unsubstituted C₁₋₂alkyl (e.g., methyl) or unsubstituted C₃₋₄cycloalkyl (e.g., cyclopropyl); R₆ is independently selected from hydrogen and deuterium.

19. The compound according to claim 2 or a prodrug, tautomer, stereoisomer, solvate, isotope derivative, or pharmaceutically acceptable salt thereof, wherein X is O or CH₂; L is CH; G is S; A is CH or N; R₃ is amino; R₂ is independently selected from halogen, -CORs, -COORs, optionally substituted C₁₋₆alkyl, optionally substituted C₂₋₆alkenyl, and optionally substituted 3-10 membered heterocycloalkyl, wherein R₅ is independently selected from unsubstituted C₁₋₆alkyl and unsubstituted C₃₋₆cycloalkyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from N and CR₄; R₄ is halogen; R₆ is independently selected from hydrogen and deuterium; or
X is O or CH₂; L is CH; G is S; A is CH or N; R₃ is amino; R₁ is independently selected from hydrogen, deuterium, and amino; R₂ is independently selected from halogen, -CORs, -COORs, optionally substituted C₁₋₆alkyl, optionally substituted C₂₋₆alkenyl, and optionally substituted 3-6 membered heterocycloalkyl, wherein R₅ is independently selected from unsubstituted C₁₋₆alkyl and unsubstituted C₃₋₆cycloalkyl, said "optionally substituted" means that the hydrogen on the group to be substituted is unsubstituted or the hydrogen(s) on one or more substitution sites of the group to be substituted is/are independently substituted by a substituent selected from a group consisting of halogen, hydroxy, and C₁₋₆alkoxyl; Y is independently selected from N and CR₄; R₄ is halogen; R₆ is independently selected from hydrogen and deuterium; or X is O; L is CH; G is S; A is N; R₃ is amino; R₁ is amino; R₂ is independently selected from -CORs and - COOR₅, wherein R₅ is independently selected from unsubstituted C₁₋₆alkyl and unsubstituted C₃₋₆cycloalkyl; Y is independently selected from N and CR₄; R₄ is halogen; R₆ is independently selected from hydrogen and deuterium; or
X is O; L is CH; G is S; A is N; R₃ is amino; R₁ is amino; R₂ is independently selected from -CORs and - COOR₅, wherein R₅ is independently selected from unsubstituted C₁₋₂alkyl (e.g., methyl) and unsubstituted C₃₋₄cycloalkyl (e.g., cyclopropyl); Y is independently selected from N and CR₄; R₄ is halogen (e.g. Cl); R₆ is independently selected from hydrogen and deuterium.

20. The compound according to claim 3 or a prodrug, tautomer, stereoisomer, solvate, isotope derivative, or pharmaceutically acceptable salt thereof, wherein said compound is selected from:
| Comp. No. | Structure | Comp. No. | Structure |
|---|---|---|---|
| 1 | 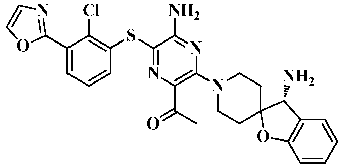 | 2 | 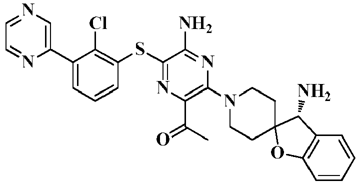 |
| 3 | 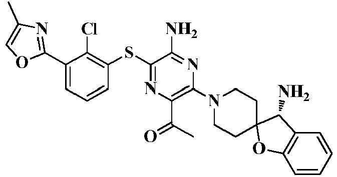 | 4 | 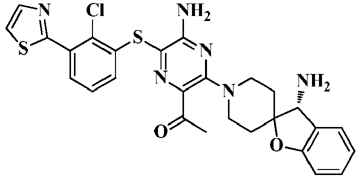 |
| 5 | 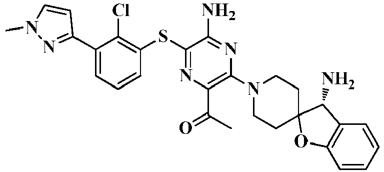 | 6 | 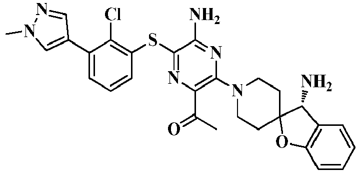 |
| 7 | 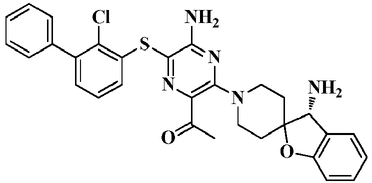 | 8 | 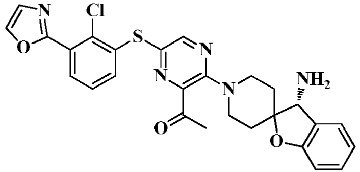 |
| 9 | 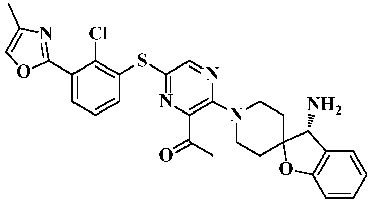 | 10 | 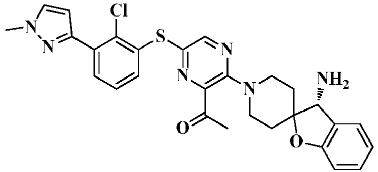 |
| 11 | 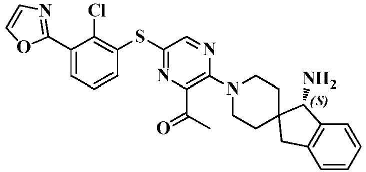 | 11A | 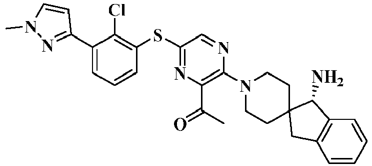 |
| 12 | 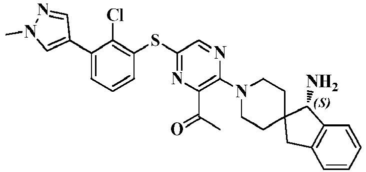 | 13 | 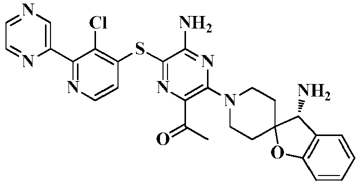 |
| 14 | 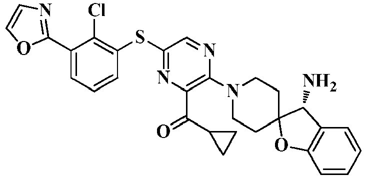 | 15 | 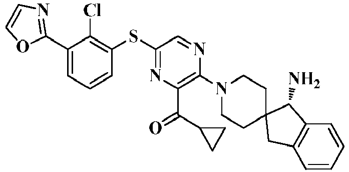 |
| 16 | 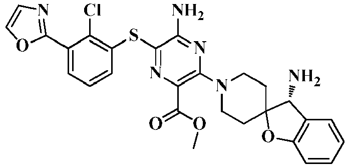 | 17 | 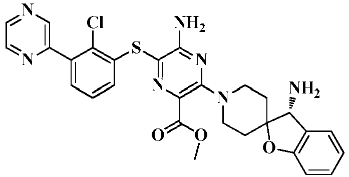 |
| 18 | 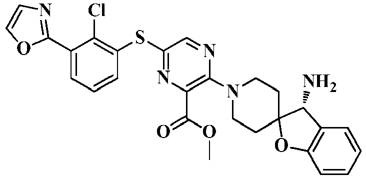 | 19 | 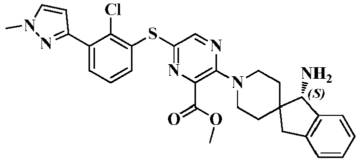 |
| 20 | 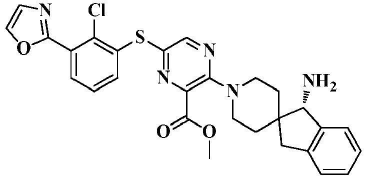 | 21 | 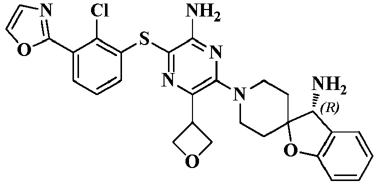 |
| 22 | 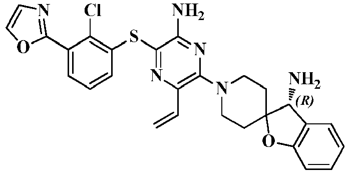 | 23 | 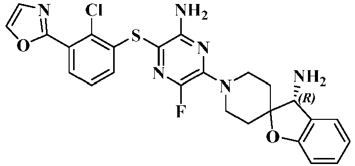 |
| 24 | 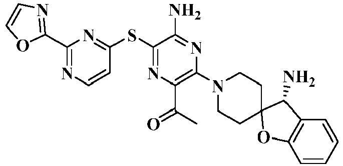 | 25 | 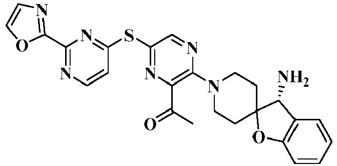 |
| 26 | 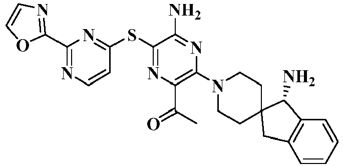 | 27 | 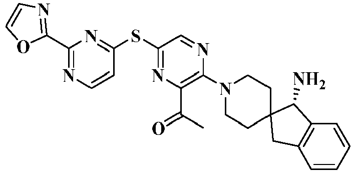 |
| 28 | 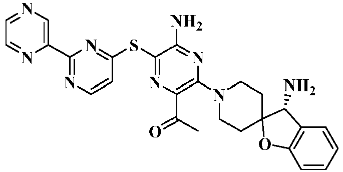 | 29 | 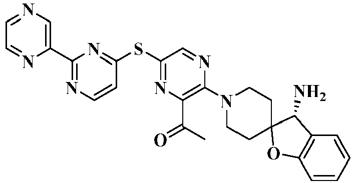 |
| 30 | 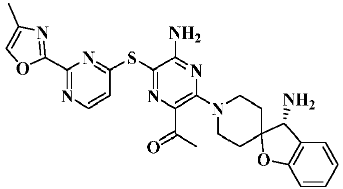 | 31 | 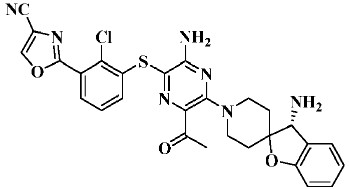 |
| 32 | 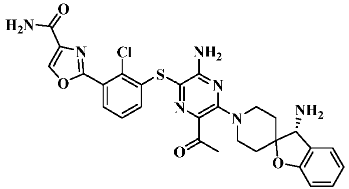 | 33 | 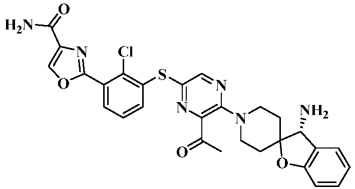 |
| 34 | 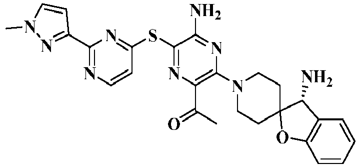 | 35 | 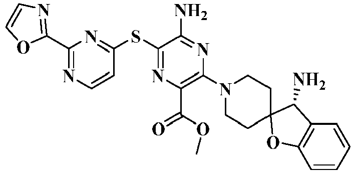 |
| 36 | 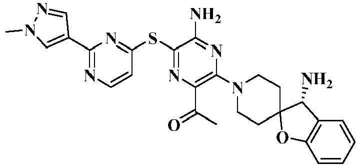 | 37 | 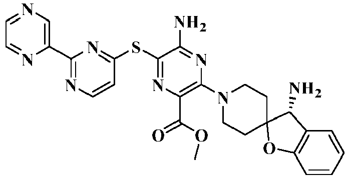 |
| 38 | 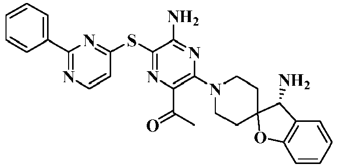 | 39 | 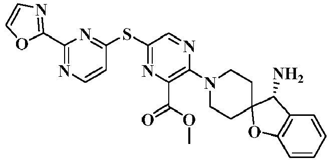 |
| 40 | 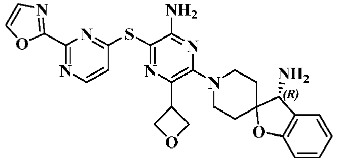 | 41 | 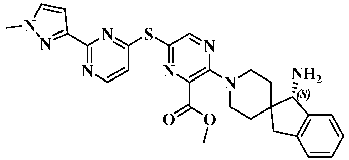 |
| 42 | 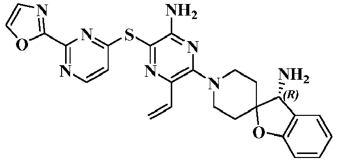 | 43 | 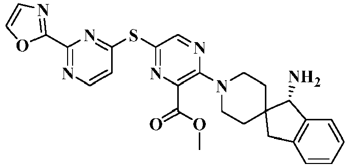 |
| 44 | 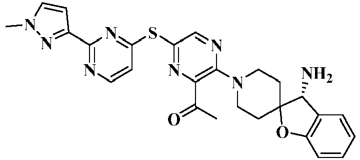 | 45 | 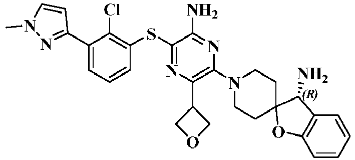 |
| 46 | 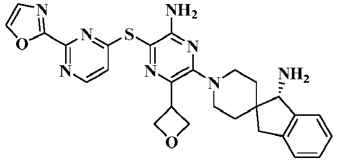 | 47 | 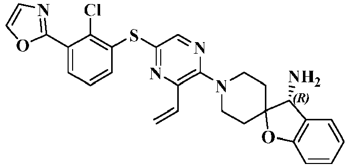 |
| 48 | 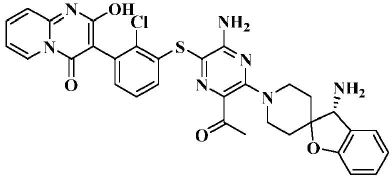 | 49 | 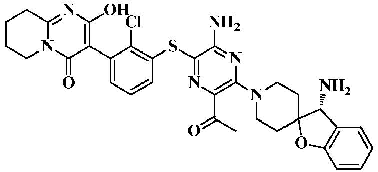 |
| 50 | 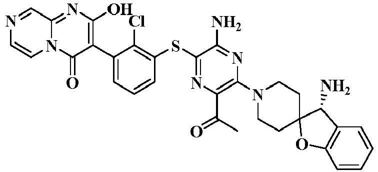 | 51 | 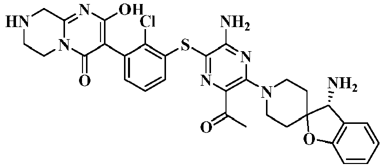 |
| 52 | 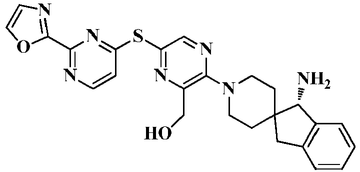 | 53 | 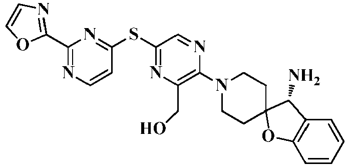 |
| 54 | 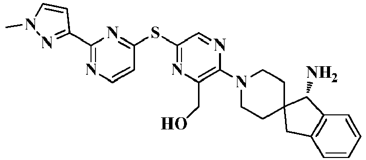 | 55 | 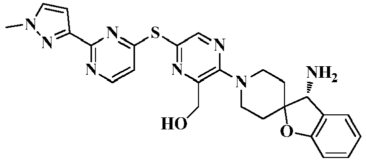 |
| 56 | 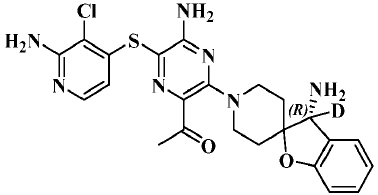 | 57 | 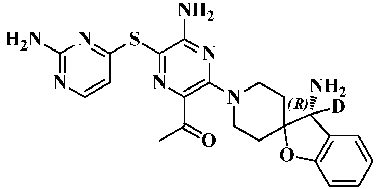 |
| 58 | 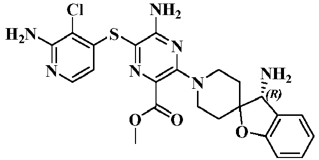 | 59 | 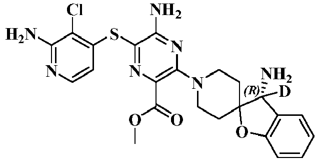 |
| 60 | 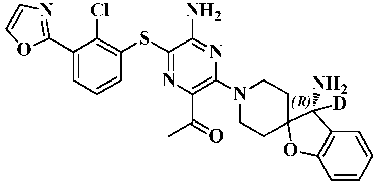 | 61 | 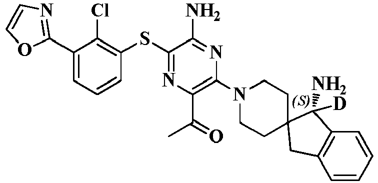 |
| 56A | 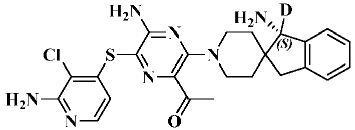 | 62 | 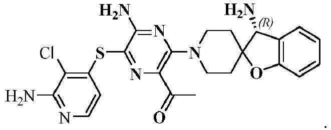 |

21. The compound according to claim 3 or a prodrug, tautomer, stereoisomer, solvate, isotope derivative, or pharmaceutically acceptable salt thereof, wherein said compound is selected from:
| Comp. No. | Structure | Comp. No. | Structure |
|---|---|---|---|
| 1 | 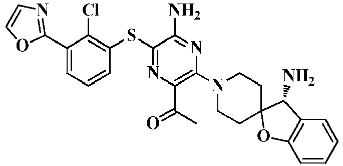 | 8 | 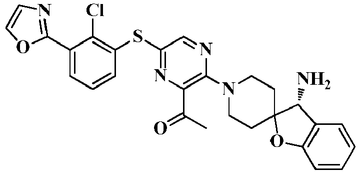 |
| 16 | 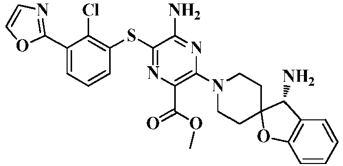 | 19 | 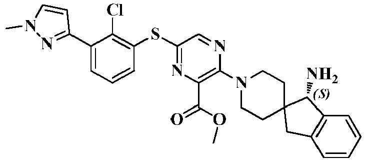 |
| 22 | 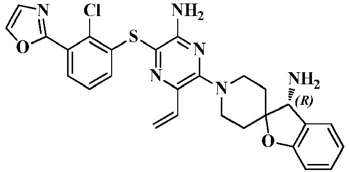 | 21 | 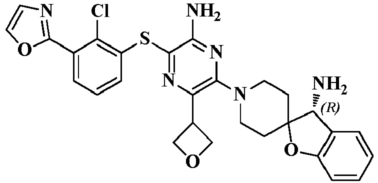 |
| 24 | 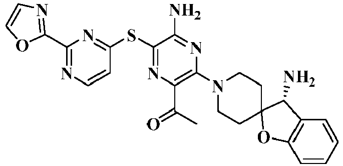 | 23 | 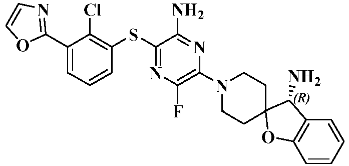 |
| 56 | 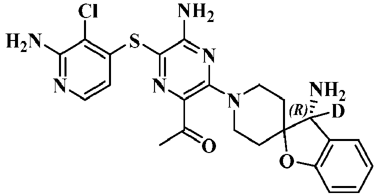 | 57 | 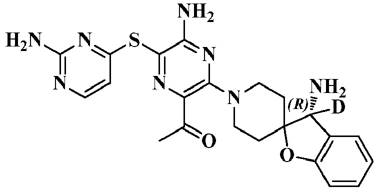 |
| 58 | 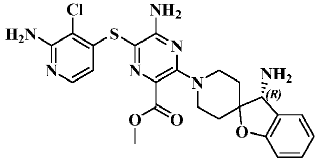 | 59 | 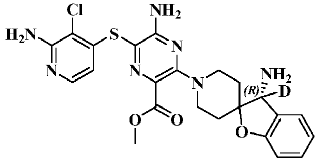 |
| 60 | 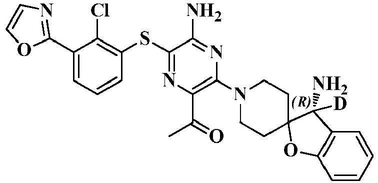 | 61 | 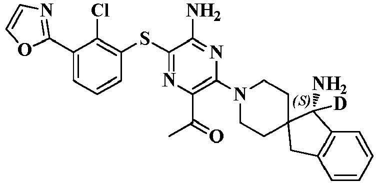 |
| 62 | 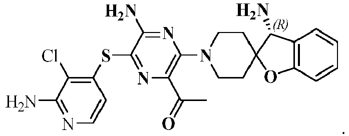 | | |

22. The compound according to claim 3 or a prodrug, tautomer, stereoisomer, solvate, isotope derivative, or pharmaceutically acceptable salt thereof, wherein said compound is selected from:
| Comp. No. | Structure | Comp. No. | Structure |
|---|---|---|---|
| 1 | 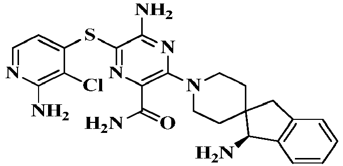 | 8 | 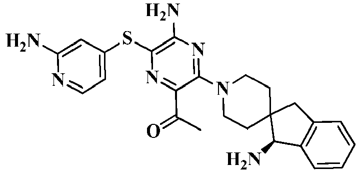 |
| 16 | 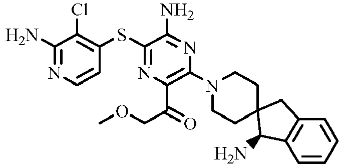 | 19 | 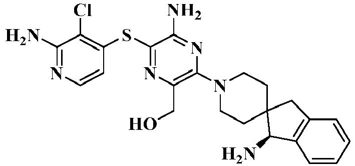 |
| 56 | 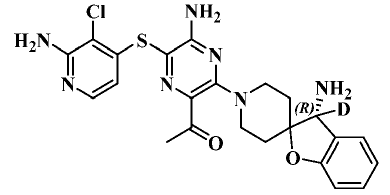 | 57 | 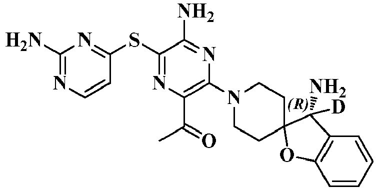 |
| 59 | 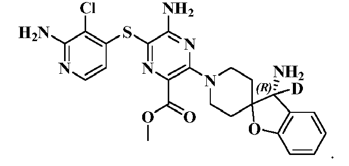 | | |

23. The compound according to claim 3 or a prodrug, tautomer, stereoisomer, solvate, isotope derivative, or pharmaceutically acceptable salt thereof, wherein said compound is selected from:
| Comp. No. | Structure | Comp. No. | Structure |
|---|---|---|---|
| 1 | 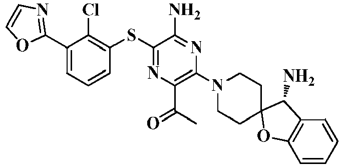 | 56 | 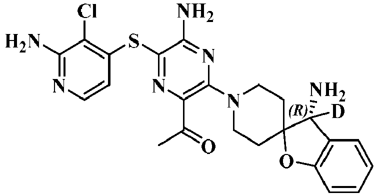 |

24. A pharmaceutical composition, which contains a compound according to any one of claims 1-23 or prodrug, tautomer, stereoisomer, solvate, isotope derivative, or pharmaceutically acceptable salt thereof.

25. Use of the compound according to any of claims 1-23 or prodrug, tautomer, stereoisomer, solvate, isotope derivative, or pharmaceutically acceptable salt thereof or the pharmaceutical composition according to claim 24 in the manufacture of a medicament for preventing and/or treating diseases, disorders, and conditions that are mediated by SHP2 activity.

26. Use according to claim 25, wherein the diseases, disorders, and conditions are tumors, cancer metastases, cardiovascular diseases, immune disorders, or visual disorders; preferably, the tumor includes solid tumor and hematological malignancy; further preferably, the solid tumor includes lung cancer, the hematological malignancy includes leukemia, preferably acute myeloid leukemia.

27. Use according to claim 25 or 26, wherein the compound according to any one of claims 1-23 or prodrug, tautomer, stereoisomer, solvate, isotope derivative, or pharmaceutically acceptable salt thereof or the pharmaceutical composition according to claim 24 is used in combination with one, two or more other compounds having tumor inhibitory activity.
